# EUROPEAN PATENT APPLICATION

(11) **EP 4 650 354 A1**
(43) Date of publication of application: **19.11.2025**
(21) Application number: 24741336.2
(22) Date of filing: 11.01.2024
(51) Int. Cl.: C07D 405/14, C07D 403/06, A61P 19/00, A61P 35/00, A61K 31/513

(54) **INDOLONE DERIVATIVE HAVING IRAK4 INHIBITORY ACTIVITY, AND PREPARATION METHOD THEREFOR AND USE THEREOF**

(30) Priority: 12.01.2023 CN 202310071055
(71) Applicant: Shenzhen Zhongge Biological Technology Co., Ltd., Futian District Shenzhen, Guangdong 518017 (CN)
(72) Inventor: DU, Xinming, Shenzhen, Guangdong 518017 (CN); DU, Lifei, Shenzhen, Guangdong 518017 (CN); CHEN, Zhaoqiang, Shenzhen, Guangdong 518017 (CN); MA, Junjun, Shenzhen, Guangdong 518017 (CN); WANG, Shaohui, Shenzhen, Guangdong 518017 (CN); CHEN, Bin, Shenzhen, Guangdong 518017 (CN); ZHANG, Peiyu, Shenzhen, Guangdong 518017 (CN)
(74) Representative: Keil & Schaafhausen Patentanwälte PartGmbB
(86) International application number: PCT/CN2024/071880
(87) International publication number: WO 2024/149340

(57) **Abstract**

The present application relates to an indolone derivative having an IRAK4 inhibitory activity as represented by formula (I), a preparation method therefor and the use thereof, and further relates to a compound capable of inhibiting and degrading the IRAK4 protein and a pharmaceutical composition comprising the indolone derivative or the compound capable of inhibiting and degrading the IRAK4 protein.

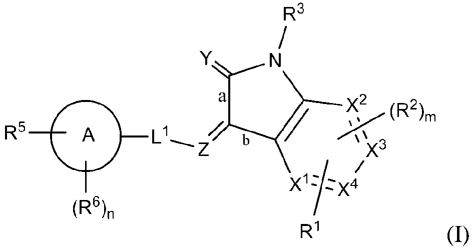

## Description

### Cross-Reference to Related Applications

The present application claims the priority to Chinese patent application No. CN202310071055.0 filled on January 12, 2023, the whole content of which is incorporated herein by reference in its entirety.

### Technical Field

The present application relates to an indolone derivative having IRAK4 inhibitory activity, a preparation method therefor, and the use thereof, and also relates to a compound capable of inhibiting and degrading IRAK4 protein and a pharmaceutical composition comprising the indolone derivative or the compound capable of inhibiting and degrading IRAK4 protein.

### Background Art

IRAK4 is a serine/threonine protein kinase, belongs to the interleukin-1 receptor-associated kinase family, which includes four subtypes: IRAK1, IRAK2, IRAK3 (or referred to as 'IRAKM'), and IRAK4. IRAK1, IRAK2, and IRAK4 promote the release of inflammatory factors, while IRAK3 is involved in the antiinflammatory process. Among the four subtypes, the biological function of IRAK4 has been clearly elucidated. After TLR or IL-1R senses external signal stimulation, a Myddosome complex formed by IRAK4 activates MAPK and NF-κB pathways, thereby leading to release of a variety of inflammatory factors.

Studies have confirmed that IRAK4 is highly expressed in various tumor cells and inflammatory models, and the research and development of IRAK4-targeted inhibitor has increasingly become an important direction of autoimmune diseases and tumor therapies. IRAK4 has two functions: kinase activity and scaffold activity, and the two functions both play an important role in downstream signal regulation. Traditional small molecule IRAK4 kinase inhibitors cannot achieve ideal therapeutic effect only by inhibiting the kinase activity thereof, but also have subsequent problems such as target protein mutation-induced resistance.

Proteolysis Targeting Chimeria (PROTAC) technology is a new technology that has emerged in recent years. The technology has gained significant attention since it came out in 2001. At present, many drugs based on this technology have entered the clinical research phase, and some of them have entered clinical phase 2. As a heterogeneous bifunctional molecule, PROTAC consists of three parts, i.e., a small molecule inhibitor at one end that can recognize the target protein, a linker group, and a ligand at the other end that can recognize E3 ubiquitin ligase. This bifunctional molecule recognizes the target protein in vivo and brings the target protein and the E3 ubiquitin ligase into close proximity to form a ternary complex, followed by ubiquitination labeling on the target protein, thus initiating a ubiquitin-proteasome dependent degradation pathway. Compared with the traditional small molecular inhibitors, PROTAC technology can inhibit the two functions of IRAK4 by degrading IRAK4 protein, so that the problem of insufficient activity of small molecular inhibitors or target protein mutation can be effectively solved. At present, no PROTAC drug targeting IRAK4 has entered the clinical research phase in China.

It is necessary to develop novel small molecule inhibitor of IRAK4 and IRAK4-targeted PROTAC molecules for the treatment of a disease, disorder or condition associated with IRAK4 protein kinase.

### Summary of the Invention

The present invention provides a small molecule indolone derivative having IRAK4 inhibitory activity and an IRAK4-targeted PROTAC molecule, which are used for the treatment of a disease, disorder or condition associated with IRAK4 protein kinase. The compounds of the present invention have high selectivity to IRAK4, and can inhibit or inhibit and degrade IRAK4 protein kinase. In addition, the compounds of the present invention have more excellent properties, such as better physical and chemical properties (e.g., solubility, physical and/or chemical stability), improved pharmacokinetic properties (e.g., improved bioavailability, improved metabolic stability, appropriate half-life and duration of action), improved safety (lower toxicity (e.g., reduced cardiotoxicity) and/or fewer side effects), and less susceptibility to drug resistance.

In one aspect, the present invention provides a compound of formula (I) as defined below: 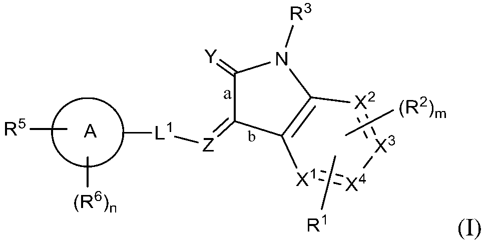 or a stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof.

In another aspect, the present invention provides the use of the compound of formula (I) or the stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof in the preparation of a proteolysis targeting chimera (PROTAC).

In another aspect, the present invention provides a proteolysis targeting chimera (PROTAC), comprising a moiety with IRAK4 protein kinase inhibitory activity, wherein the moiety is derived from the compound of formula (I) or the stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof according to the present invention.

In another aspect, the present invention provides a pharmaceutical composition comprising the compound of formula (I) or the stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof according to the present invention, or the PROTAC molecule of the present invention, and a pharmaceutically acceptable excipient, carrier or diluent. The pharmaceutical composition is preferably a solid formulation, a liquid formulation, or a transdermal formulation.

In another aspect, the present invention provides the use of the compound of formula (I) or the stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof according to the present invention, or the PROTAC molecule of the present invention, or the pharmaceutical composition of the present invention in the preparation of a medicament for treating a disease, disorder or condition associated with IRAK4 protein kinase.

In another aspect, the present invention provides a method for treating a disease, disorder or condition associated with IRAK4 protein kinase, comprising administering to an individual in need thereof a therapeutically effective amount of the compound of formula (I) or the stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof according to the present invention, or the PROTAC molecule of the present invention, or the pharmaceutical composition of the present invention.

In another aspect, the present invention provides a method for preparing the compound of formula (I) of the present invention and the PROTAC molecule of the present invention.

### Detailed Description of Embodiments

### Definitions

Unless otherwise defined below, all technical and scientific terms as used herein are intended to have the same meaning as commonly understood by those skilled in the art. Reference to technologies used herein is intended to refer to technologies as commonly understood in the art, including those technology variations or equivalent technology substitutions that are obvious to those skilled in the art. Although it is believed that the following terms are well understood by those skilled in the art, the following definitions are set forth to better explain the present invention.

The terms "including", "comprising", "having", "containing" or "involving" and other variants thereof herein are inclusive or open-ended, and other unlisted elements or method steps are not excluded (that is, these terms also cover the terms "consisting essentially of" and "consisting of").

As used herein, the term "alkane" means a linear or branched saturated aliphatic hydrocarbon.

As used herein, the term "alkyl" means a linear or branched monovalent saturated aliphatic hydrocarbyl group, which can be regarded as a group derived by the loss of one hydrogen atom from an alkane. In some embodiments, the alkyl has 1 to 12, such as 1 to 6 (such as 1, 2, 3, 4, 5, or 6) carbon atoms. For example, as used herein, the term "C₁₋₆ alkyl" refers to a linear or branched group having 1 to 6 carbon atoms, including "C₂₋₆ alkyl", "C₂₋₅ alkyl" and "C₁₋₄ alkyl". Examples of "C₁₋₆ alkyl" include methyl, ethyl, n-propyl, isopropyl, n-butyl, isobutyl, sec-butyl, tert-butyl, n-pentyl, and n-hexyl. The alkyl is optionally substituted with one or more (such as 1 to 3) suitable substituents such as halogen (in this case, the group is referred to as "haloalkyl", such as CF₃, C₂F₅, CHF₂, CH₂F, CH₂CF₃, CH₂Cl, or -CH₂CH₂CF₃). The term "C₁₋₄ alkyl" refers to alkyl having 1 to 4 carbon atoms (i.e., methyl, ethyl, n-propyl, isopropyl, n-butyl, isobutyl, sec-butyl, or tert-butyl).

As used herein, the term "alkylene" means a linear or branched divalent saturated aliphatic hydrocarbyl group. In some embodiments, the alkylene has 1 to 12 carbon atoms, preferably 1, 2, 3, 4, 5, or 6 carbon atoms, such as methylene, ethylene, propylene, or butylene.

As used herein, the term "alkenyl" means a linear or branched monovalent aliphatic hydrocarbyl group that contains one or more double bonds. In some embodiments, the alkenyl has 2 to 6 carbon atoms ("C₂₋₆ alkenyl"). The alkenyl is, for example, -CH=CH₂, -CH₂CH=CH₂, -C(CH₃)=CH₂, -CH₂-CH=CH-CH₃, 2-pentenyl, 3-pentenyl, 4-pentenyl, 2-hexenyl, 3-hexenyl, 4-hexenyl, 5-hexenyl, 2-methyl-2-propenyl, and 4-methyl-3-pentenyl. When the compound of the present invention contains alkenyl, the compound may exist in a pure E (entgegen) form, a pure Z (zusammen) form, or as any mixture thereof. The term "alkenylene" is a corresponding divalent group, including, for example, "C₂₋₆ alkenylene" and "C₂₋₄ alkenylene", and specific examples thereof include but are not limited to: -CH=CH-, -CH₂CH=CH-, -C(CH₃)=CH-, butenylene, pentenylene, hexenylene, cyclopentenylene, cyclohexenylene, etc.

As used herein, the term "fused" means that two or more cyclic structures share two adjacent atoms with each other.

As used herein, the terms "cyclohydrocarbyl", "hydrocarbon ring", and "cyclohydrocarbylene"refer to saturated (i.e., "cycloalkyl" and "cycloalkylene") or partially unsaturated (i.e., having one or more double bonds (i.e., "cycloalkenyl" and "cycloalkenylene") and/or triple bonds within the ring) monocyclic or polycyclic fused hydrocarbon rings having, for example, 3-10 (suitably having 3-8, more suitably having 3-7, 3-6, 4-6, or 5-6) ring carbon atoms, which include but are not limited to cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclobutenyl(ene) (ring), cyclopentenyl(ene) (ring), cyclohexenyl(ene) (ring), cycloheptenyl(ene) (ring), cyclooctenyl(ene) (ring), cyclononenyl(ene) (ring), etc. In some embodiments, the cyclohydrocarbyl includes aryl-fused cyclohydrocarbyl, as long as the entire ring system is non-aromatic.

As used herein, the term "cycloalkyl" refers to a saturated monocyclic or polycyclic (such as bicyclic) hydrocarbon ring (e.g., monocyclic, such as cyclopropyl, cyclobutyl, cyclopentyl, cyclohexyl, cycloheptyl, cyclooctyl, and cyclononyl, or bicyclic, including spiro, fused or bridged systems (such as bicyclo[1.1.1]pentyl, bicyclo[2.2.1]heptyl, bicyclo[3.2.1]octyl, bicyclo[5.2.0]nonyl, and decahydronaphthyl), which are optionally substituted with one or more (such as 1 to 3) suitable substituents. The cycloalkyl group has 3 to 15 carbon atoms, suitably 3 to 10 carbon atoms, more suitably 3-8 carbon atoms, for example 3-7, 3-6, 4-6, or 5-6 carbon atoms. For example, the term "C₃₋₆cycloalkyl" refers to a saturated monocyclic or polycyclic (such as bicyclic) hydrocarbon ring having 3 to 6 ring-forming carbon atoms (e.g., cyclopropyl, cyclobutyl, cyclopentyl, or cyclohexyl). The cycloalkyl is optionally substituted with one or more (such as 1 to 3) suitable substituents, e.g., methyl-substituted cyclopropyl.

As used herein, the terms "cycloalkenyl" and "cycloalkenylene" refer to monocyclic or polycyclic (such as bicyclic) fused hydrocarbon rings having one or more double bonds in the ring (e.g., monocyclic, such as cyclopropenyl, cyclobutenyl, cyclopentenyl, cyclopentadienyl, cyclohexenyl, cyclohexadienyl, cycloheptenyl, cyclooctenyl, and cyclononenyl, or dicyclic). The cycloalkenyl and "cycloalkenylene" have 3 to 10 carbon atoms, suitably 3-8, for example 3-7, 3-6, 4-6, or 5-6. The cycloalkenyl and cycloalkenylene are optionally substituted with one or more (such as 1 to 3) suitable substituents, e.g., methyl-substituted cyclopentenyl.

As used herein, the terms "heterocyclyl", "heterocyclic ring" and "heterocyclylene" refer to saturated (i.e., "heterocycloalkyl" and "heterocycloalkylene") or partially unsaturated (e.g., having one or more double bonds in the ring (i.e., "heterocycloalkenyl" and "heterocycloalkenylene")) monovalent monocyclic or bicyclic fused cyclic structures, which have 2, 3, 4, 5, 6, 7, 8, or 9 carbon atoms and one or more (e.g., 1, 2, 3, or 4) heteroatom-containing groups selected from O, S, S(=O), S(=O)₂, and NR' in the ring, wherein R' is as defined above. The heterocyclyl may be connected to the rest of the molecule via any of the carbon atoms or the nitrogen atom (if present). Particularly, 3- to 10-membered heterocyclyl is a group containing 3-10 (for example, 3-8, 3-7, 3-6, 4-6, or 5-6) ring atoms, including carbon atoms and heteroatoms. Heterocyclyl includes nitrogen-containing heterocyclyl, oxygen-containing bridged cyclic groups, and sulfur-containing heterocyclyl. Examples that can be listed include, but are not limited to, oxiranyl, aziridinyl, azetidinyl, oxetanyl, tetrahydrofuryl, tetrahydrothienyl, dioxolinyl, pyrrolidinyl, pyrrolidonyl, oxazolidine, thiazolidinyl, pyrazolidinyl, imidazolidinyl, pyrazolidinyl, pyrrolinyl, tetrahydropyranyl, piperidinyl, hexahydropyrimidinyl, triazinyl, morpholinyl, dithianyl, thiomorpholinyl, piperazinyl, trithianyl, azocanyl, dihydropyrrolyl, dihydroimidazolyl, and azacyclooctenyl. The heterocyclyl is optionally substituted with one or more (such as 1 to 3) suitable substituents (e.g., halogen, OH, NH₂, oxo (=O), C₁₋₆ alkyl, and C₁₋₆ haloalkyl).

As used herein, the term "nitrogen-containing heterocyclic ring" refers to a saturated or unsaturated monocyclic or bicyclic group, which has 2, 3, 4, 5, 6, 7, 8, 9, 10, 11, 12, or 13 carbon atoms and at least one nitrogen atom in the ring and can further comprise optionally one or more (e.g., 1, 2, 3, or 4) ring members selected from N, O, C=O, S, S=O, and S(=O)₂. The nitrogen-containing heterocyclic ring is connected to the rest of the molecule via a nitrogen atom. The nitrogen-containing heterocyclic ring is preferably a saturated nitrogen-containing monocyclic ring. In particular, a 3- to -14-membered nitrogen-containing heterocyclic ring is a group having 3-14 ring atoms, including carbon atoms and heteroatoms (at least one of which is a nitrogen atom) in the ring, including but not limited to a 3-membered nitrogen-containing heterocyclic ring (such as aziridinyl), a 4-membered nitrogen-containing heterocyclic ring (such as azetidinyl), a 5-membered nitrogen-containing heterocyclic ring (such as pyrrolyl, pyrrolidinyl (pyrrolidine ring), pyrrolinyl, pyrrolidonyl, imidazolyl, imidazolidinyl, imidazolinyl, pyrazolyl, and pyrazolinyl), a 6-membered nitrogen-containing heterocyclic ring (such as piperidinyl (piperidine ring), morpholinyl, thiomorpholinyl, and piperazinyl), a 7-membered nitrogen-containing heterocyclic ring, etc.

As used herein, the term "heterocyclyl" covers a fused ring structure, and the point of connection of the fused ring structure to other groups can be on any ring in the fused ring structure. Therefore, the heterocyclyl of the present invention also includes, but is not limited to, heterocyclyl fused heterocyclyl, heterocyclyl fused cycloalkyl, monoheterocyclyl fused monoheterocyclyl, and monoheterocyclyl fused monocycloalkyl, e.g., 3- to 7-membered (mono)heterocyclyl fused 3- to 7-membered (mono)heterocyclyl, 3- to 7-membered (mono)heterocyclyl fused (mono) cycloalkyl, and 3- to 7-membered (mono)heterocyclyl fused C₄₋₆(mono)cycloalkyl, and examples thereof include but are not limited to pyrrolidinyl fused cyclopropyl, cyclopentyl fused aziridinyl, pyrrolidinyl fused cyclobutyl, pyrrolidinyl fused pyrrolidinyl, pyrrolidinyl fused piperidinyl, pyrrolidinyl fused piperazinyl, piperidinyl fused morpholinyl, 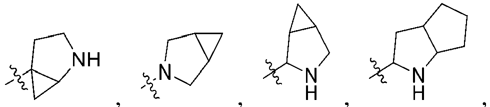 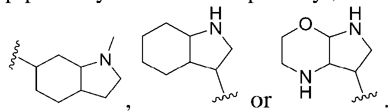 In some embodiments, the heterocyclyl also includes heteroaryl fused heterocyclyl or cyclohydrocarbyl, and aryl fused heterocyclyl, as long as the entire ring system is non-aromatic. In some embodiments, the heterocyclyl includes 5- to 6-membered monocyclic heteroaryl fused C₅₋₆ monocyclic cyclohydrocarbyl, 5- to 6-membered monocyclic heteroaryl fused 5- to 6-membered monocyclic heterocyclyl, and benzo5- to 6-membered monocyclic heterocyclyl, e.g., pyrrolotetrahydropyridinyl, pyrazolotetrahydropyridinyl, and imidazolotetrahydropyridinyl.

As used herein, the term "aryl" refers to an all-carbon monocyclic or fused polycyclic aromatic group with a conjugated π electron system. For example, as used herein, the term "C₆₋₁₄ aryl" means an aromatic group containing 6 to 14 (e.g., 6 to 12) carbon atoms, such as phenyl or naphthyl. The aryl is optionally substituted with one or more (such as 1 to 3) suitable substituents (e.g., halogen, -OH, -CN, -NO₂, and C₁₋₆ alkyl).

As use herein, the term "heteroaryl" refers to a monocyclic or polycyclic (e. g., bicyclic or tricyclic) aromatic ring system having 5 to 14 ring atoms, such as 5, 6, 7, 8, 9, 10, 11, 12, 13, or 14 ring atoms, especially having 1, 2, 3, 4, 5, 6, 7, 8, 9, 10, 11, 12, or 13 carbon atoms and 1, 2, 3, 4, or 5 identical or different heteroatoms independently selected from N, O, S, and S(O)₂. One or more ring carbon atoms in heteroaryl can be replaced by C(O). The heteroaryl may be benzo. Examples of heteroaryl include, but are not limited to: pyridinyl, pyridonyl, pyrimidinyl, pyrimidonyl, pyrazinyl, pyridazinyl, thiazolyl, thienyl, oxazolyl, furyl, pyrrolyl, pyrazolyl, triazolyl, tetrazolyl, isoxazolyl, isothiazolyl, imidazolyl, triazinyl, oxadiazolyl, thiadiazolyl, benzothiazolyl, benzoisothiazolyl, imidazolopyridinyl, quinolyl, indolyl, pyrrolopyridazinyl, benzofuryl, benzothienyl, indazolyl, benzooxazolyl, benzoisoxazolyl, quinazolinyl, pyrrolopyridinyl, pyrazolopyrimidinyl, imidazopyridazinyl, pyrazolopyridinyl, triazolopyridinyl, isoquinolinyl, tetrahydroisoquinolinyl, benzimidazolyl, cinnolinyl, indolizinyl, phthalazinyl, isoindolyl, pteridinyl, purinyl, furazanyl, benzofurazanyl, quinoxalinyl, naphthyridinyl, or furopyridinyl. Heteroaryl can be optionally substituted with one or more (e.g., 1, 2, 3, or 4) suitable substituents.

As used herein, the term "halo" or "halogen" group is defined to include F, Cl, Br, or I.

As used herein, the term "haloalkyl" refers to alkyl substituted with one or more (such as 1 to 3) identical or different halogen atoms, wherein the alkyl is as defined herein. The terms "C₁₋₈ haloalkyl", "C₁₋₆ haloalkyl", and "C₁₋₄ haloalkyl" respectively refer to haloalkyl groups having 1 to 8 carbon atoms, 1 to 6 carbon atoms, and 1 to 4 carbon atoms, e.g., -CF₃, -C₂F₅, -CHF₂, -CH₂F, -CH₂CF₃, -CH₂Cl, or -CH₂CH₂CF₃.

The term "substituted" means that one or more (e.g., one, two, three, or four) hydrogens on the specified atom are replaced by selection from the indicated groups, provided that the normal valence of the specified atom in the current situation is not exceeded and the substitution forms a stable compound. Combinations of substituents and/or variables are allowed only when such combinations form stable compounds.

If a group is described as being "optionally substituted with" or "optionally substituted", the group can be: (1) unsubstituted or (2) substituted. If a carbon of a group is described as being optionally substituted with one or more substituents in a list, one or more hydrogens on the carbon (to the extent of any hydrogen present) can be individually and/or collectively replaced by independently selected optional substituents. If the nitrogen of a group is described as being optionally substituted by one or more substituents in a list, one or more hydrogens on the nitrogen (to the extent of any hydrogen present) can each be replaced by independently selected optional substituents.

If a substituent is described as being "independently selected from" a group, each substituent is selected independently of another. Therefore, each substituent may be the same as or different from another (other) substituent.

As used herein, the term "one or more" means one or more than one, such as 2, 3, 4, 5, or 10, under reasonable conditions.

Unless otherwise specified, as used herein, the point of connection of a substituent can be any suitable position of the substituent.

When the bond to a substituent is shown as passing through a bond connecting two atoms in a ring ("floating bond"), such a substituent can be bonded to any ring-forming atom in the substitutable ring, unless otherwise specified. Where an available ring member is shown to carry replaceable hydrogen atoms, when the floating bond is bonded to the available ring member, the replaceable hydrogen atom is substantially replaced (i.e., does not exist).

The present invention further comprises all pharmaceutically acceptable isotopically labeled compounds, which are the same as the compounds of the present invention, except that one or more atoms are replaced by atoms that have the same atomic number but different atomic masses or mass numbers from those prevailing in nature. Examples of isotopes suitable for inclusion in the compounds of the present invention include, but are not limited to, isotopes of hydrogen (e.g., deuterium (D, ²H) and tritium (T, ³H)); isotopes of carbon (e.g., ¹¹C, ¹³C, and ¹⁴C); isotopes of chlorine (e.g., ³⁶Cl); isotopes of fluorine (e.g., ¹⁸F); isotopes of iodine (e.g., ¹²³I and ¹²⁵I); isotopes of nitrogen (e.g., ¹³N and ¹⁵N); isotopes of oxygen (e.g., ¹⁵O, ¹⁷O, and ¹⁸O); isotopes of phosphorus (e.g., ³²P); and isotopes of sulfur (e.g., ³⁵S). Some isotopically labeled compounds of the present invention (e.g., those incorporated with radioisotopes) can be used in the study (e.g., analysis) of drug and/or substrate tissue distribution. Radioisotopes tritium (i.e., ³H) and carbon-14 (i.e., ¹⁴C) are particularly useful for this purpose because of their easy incorporation and easy detection. Substitution with positron emission isotopes (e.g., ¹¹C, ¹⁸F, ¹⁵O, and ¹³N) can be used to test the occupation of substrate receptors in positron emission tomography (PET) research. Isotopically labeled compounds of the present invention can be prepared by using appropriate isotopically labeled reagents instead of previously used unlabeled reagents by methods similar to those described in the attached routes and/or examples and preparation. Pharmaceutically acceptable solvates of the present invention include those in which the crystallization solvent can be substituted with isotopes, for example, D₂O, acetone-d₆, or DMSO-d₆. In some embodiments, the isotopically labeled compound of the present invention is a deuterated compound.

The term "stereoisomer" means an isomer formed by at least one asymmetric center, which has the same chemical composition but different spatial arrangement of atoms or groups. In compounds with one or more (e.g., 1, 2, 3, or 4) asymmetric centers, racemic mixtures, single enantiomers, diastereomer mixtures, and individual diastereomers can be produced. Specific individual molecules can also exist as geometric isomers (cis/trans). Similarly, the compounds of the present invention can exist as a mixture of two or more structurally different forms in rapid equilibrium (commonly referred to as tautomers). Representative examples of tautomers include ketone-enol tautomers, phenol-ketone tautomers, nitroso-oxime tautomers, imine-enamine tautomers, etc. It needs to be understood that the scope of the present application covers all such isomers or mixtures thereof in any ratio (e.g., 60%, 65%, 70%, 75%, 80%, 85%, 90%, 95%, 96%, 97%, 98%, and 99%).

"Diastereomers" refer to stereoisomers that have two or more chiral centers and whose molecules are not mirror images of each other. Diastereomers have different physical properties, such as melting points, boiling points, spectral properties and reactivity. Mixtures of diastereomers can be separated by high-resolution analytical methods such as electrophoresis and chromatography.

"Enantiomers" refer to two stereoisomers of a compound that are non-superimposable mirror images of each other.

The term "chiral" refers to molecules that have non-superimposable mirror images, whereas the term "achirality" refers to molecules that can be superimposed on their mirror images.

The compound of the present invention can be prepared into a racemic form, or a single enantiomer can be prepared by enantioselective synthesis or resolution.

As used herein, the term "cis/trans isomer" or "geometric isomer" is due to the fact that double bonds or single bonds of ring-forming carbon atoms cannot rotate freely. The compounds provided herein include all cis, trans, syn, anti, entgegen (E), and zusammen (Z) isomers and corresponding mixtures thereof.

Solid lines ( 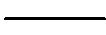 ), solid wedges ( 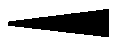 ), dashed wedges ( 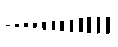 ) and cross bonds ( 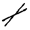 ) can be used herein to describe the chemical bonds of the compounds of the present invention. The use of solid lines to depict the bonds connected to asymmetric carbon atoms is intended to indicate that all possible stereoisomers at the carbon atoms (such as specific enantiomers and racemic mixtures) are included. The use of solid or dashed wedges to depict the bonds connected to asymmetric carbon atoms is intended to indicate the presence of the stereoisomers shown. When present in a racemic mixture, solid and dashed wedges are used to define the relative stereochemistry rather than the absolute stereochemistry. The use of cross bonds to depict double bonds is intended to indicate one of cis/trans isomers or E/Z isomers, or a mixture of cis/trans isomers or E/Z isomers. Unless otherwise indicated, it is intended that the compounds of the present invention may exist in the form of stereoisomers, including cis and trans isomers, optical isomers (e.g., R- and S-enantiomers), diastereomers, geometric isomers, rotamers, conformers, atropisomers, and mixtures thereof. The compounds of the present invention may exhibit more than one type of isomerism, and consist of mixtures thereof (such as racemic mixtures and diastereomeric pairs).

It should also be understood that certain compounds of the present invention may exist in free form for the treatment or, where appropriate, in the form of pharmaceutically acceptable derivatives thereof. In the present invention, pharmaceutically acceptable derivatives include, but are not limited to, pharmaceutically acceptable salts, esters, solvates, metabolites, or prodrugs, which can directly or indirectly provide the compounds of the present invention or metabolites or residues thereof after they are administered to patients in need thereof. Therefore, reference to "the compound of the present invention" herein is also intended to cover the above various derivative forms of the compound.

The term "pharmaceutically acceptable" means that a substance or composition is necessarily chemically and/or toxicologically compatible with other components constituting a formulation and/or the mammal to be treated therewith.

The pharmaceutically acceptable salt of the compound of the present invention includes acid addition salts and base addition salts thereof.

Suitable acid addition salts are formed from acids that form pharmaceutically acceptable salts. Examples include aspartate, benzoate, bicarbonate/carbonate, bisulfate/sulfate, fumarate, glucoheptonate, gluconate, glucuronate, hexafluorophosphate, hydrobromide/bromide, hydroiodide/iodide, maleate, malonate, methyl sulfate, naphthylate, nicotinate, nitrate, orotate, oxalate, palmitate, and other similar salts.

Suitable base addition salts are formed from bases that form pharmaceutically acceptable salts. Examples include aluminum salt, arginate, choline salt, diethylamine salt, lysinate, magnesium salt, meglumine salt, potassium salt, and other similar salts.

The review of suitable salts can be found in Stahl and Wermuth, "Handbook of Pharmaceutical Salts: Properties, Selection, and Use" (Wiley-VCH, 2002). Methods for preparing pharmaceutically acceptable salts of the compounds of the present invention are known to those skilled in the art.

As use herein, that term "ester" means esters derived from various compounds of the general formula in the present application, including physiologically hydrolyzable esters (compounds of the present invention that can be hydrolyzed under physiological conditions to release free acid or alcohol forms). The compound of the present invention itself may also be an ester.

The present invention covers all possible crystalline forms or polymorphs of the compound of the present invention, which can be single polymorphs or a mixture of more than one polymorph in any ratio.

The compound of the present invention may exist in the form of a solvate (preferably a hydrate), wherein the compound of the present invention comprises a polar solvent as a structural element of the crystal lattice of the compound, especially for example, water, methanol, or ethanol. The amount of the polar solvent, especially water, can exist in a stoichiometric or non-stoichiometric ratio.

Those skilled in the art will understand that since nitrogen requires an available lone pair of electrons for oxidization into oxide, not all nitrogen-containing heterocyclic rings can form *N*-oxides. Those skilled in the art will recognize nitrogen-containing heterocyclic rings capable of forming *N-*oxides. Those skilled in the art will also recognize that tertiary amines can form *N*-oxides. Synthesis methods for preparing *N*-oxides of heterocyclic rings and tertiary amines are well known to those skilled in the art, including oxidation of heterocyclic rings and tertiary amines with peroxy acids such as peracetic acid and m-chloroperoxybenzoic acid (MCPBA), hydrogen peroxide, alkyl hydrogen peroxide such as tert-butyl hydrogen peroxide, sodium perborate and dioxirane such as dimethyl dioxirane. These methods for preparing *N*-oxides have been widely described and reviewed in literatures, see, for example: T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

Also included within the scope of the present invention are metabolites of the compounds of the present invention, that is, substances formed in vivo when the compounds of the present invention are administered. Such products can be produced by, for example, oxidation, reduction, hydrolysis, amidation, deamidation, esterification, degreasing, and enzymolysis of the administered compound. Therefore, the present invention includes metabolites of the compounds of the present invention, including compounds prepared by a method of bringing the compounds of the present invention into contact with mammals for a time sufficient to produce metabolites thereof.

The present invention further comprises, within its scope, prodrugs of the compound of the present invention, which are certain derivatives of the compound of the present invention that may have little or no pharmacological activity per se and can be converted into the compound of the present invention with desired activity by, for example, hydrolytic cleavage when administered into or on the body. Generally, such prodrugs can be functional derivatives of the compounds, which can be easily converted into the desired therapeutically active compounds in vivo. Other information about the use of prodrugs can be found in "Pro-drugs as Novel Delivery Systems", vol. 14, ACS Symposium Series (T. Higuchi and V. Stella) and "Bioreversible Carriers in Drug Design," Pergamon Press, 1987 (edited by E. B. Roche, American Pharmaceutical Association). The prodrugs of the present invention can be prepared, for example, by replacing appropriate functional groups present in the compound of the present invention with some moieties known to those skilled in the art as "pro-moiety" (described in e.g., "Design of Prodrugs", H. Bundgaard (Elsevier, 1985)).

The present invention further covers compounds of the invention containing protecting groups. During any process of preparing the compound of the present invention, it may be necessary and/or desirable to protect sensitive groups or reactive groups on any relevant molecules, thereby forming a chemically protected form of the compound of the present invention. This can be achieved by conventional protecting groups, for example, those protecting groups as described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, which are incorporated herein by reference. The protecting group can be removed at an appropriate subsequent stage by using a method known in the art.

As used herein, the term "about" means within ±10%, preferably within ±5%, and more preferably within ±2% of the stated numerical value.

### Compounds

**In** one aspect, the present invention provides a compound of formula (I): 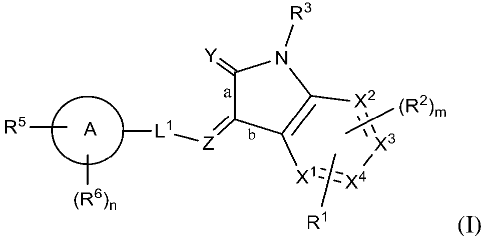
or a stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof,
wherein:
   ring 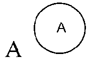 is selected from: C₃₋₁₀ cyclohydrocarbyl, 5- to 10-membered heterocyclyl, C₆₋₁₀ aryl, and 5- to 10-membered heteroaryl;
   X¹, X², X³, and X⁴ are each independently N or CH; and at least one of X¹, X², X³, and X⁴ is not N;
   Y is O or S;
   Z is N or CR⁴;
   the characters "a" and "b" respectively indicate bonds between a ring carbon atom connected to Z and two adjacent ring carbon atoms;
   wherein: when Z is CR⁴, the moiety following structure of formula (i) or (ii): 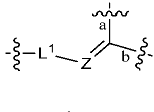 is represented by the 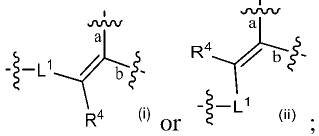
   when Z is N, the moiety 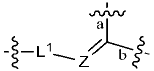 is represented by the following structure of formula (iii) or (iv): 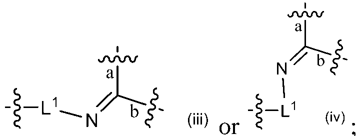
   L¹ is selected from: a direct bond, O, S, C(O), S(O), S(O)₂, and NR⁷;
   R¹ is -L²-R^{1a};
   L² is -NR^{1b}-*, -S(O)₂NR^{1b}-*, -C(O)-NR^{1b}-*, -CR^{1c}R^{1d}-NR^{1b}-*, -NR^{1b}-C(O)-*, or -NR^{1b}-S(O)₂-*, wherein the bond indicated by * is connected to R^{1a};
   R^{1a} is selected from: C₁₋₆ alkyl, C₁₋₆ haloalkyl, C₂₋₆ alkenyl, C₂₋₆ haloalkenyl, - C₁₋₆ alkylene-O-C₁₋₆ alkyl, -OC₁₋₆ alkylene-OC₁₋₆ alkyl, -C₁₋₆ alkylene-OH, -C₁₋₆ alkylene-SH, -C₁₋₆ alkylene-CN, -C₁₋₆ alkylene-NR^{1e}R^{1f}, and the cyclic group Cy1, wherein the cyclic group Cy1 is selected from C₃₋₁₀ cyclohydrocarbyl, 3- to 10-membered heterocyclyl, C₆₋₁₀ aryl, and 5- to 10-membered heteroaryl, wherein the C₃₋₁₀ cyclohydrocarbyl, 3- to 10-membered heterocyclyl, C₆₋₁₀ aryl, and 5- to 10-membered heteroaryl are each optionally substituted with one or more substituents independently selected from the following groups: halogen, OH, SH, -NR^{1e}R^{1f}, CN, C₁₋₆ alkyl, C₁₋₆ haloalkyl, C₂₋₆ alkenyl, C₂₋₆ haloalkenyl, -O-C₁₋₆ alkyl, -O-C₁₋₆ haloalkyl, -S-C₁₋₆ alkyl, -C₁₋₆ alkylene-O-C₁₋₆ alkyl, -OC₁₋₆ alkylene-OC₁₋₆ alkyl, - C₁₋₆ alkylene-OH, -C₁₋₆ alkylene-SH, -C₁₋₆ alkylene-CN, and -C₁₋₆ alkylene-NR^{1e}R^{1f};
   R^{1c} and R^{1d} and each R² are independently selected from: H, halogen, OH, SH, -NR^{2a}R^{2b}, CN, C₁₋₆ alkyl, C₁₋₆ haloalkyl, C₂₋₆ alkenyl, C₂₋₆ haloalkenyl, -O-C₁₋₆ alkyl, -O-C₁₋₆ haloalkyl, -S-C₁₋₆ alkyl, -C₁₋₆ alkylene-O-C₁₋₆ alkyl, -OC₁₋₆ alkylene-OC₁₋₆ alkyl, -C₁₋₆ alkylene-OH, -C₁₋₆ alkylene-SH, -C₁₋₆ alkylene-CN, and -C₁₋₆ alkylene-NR^{2a}R^{2b};
   m is 0, 1, 2, or 3;
   R³ and R⁷ are each independently selected from: H, C₁₋₆ alkyl, C₁₋₆ haloalkyl, C₂₋₆ alkenyl, C₂₋₆ haloalkenyl, -C₁₋₆ alkylene-O-C₁₋₆ alkyl, -C₁₋₆ alkylene-OH, -C₁₋₆ alkylene-SH, -C₁₋₆ alkylene-CN, and -C₁₋₆ alkylene-NR^{3a}R^{3b};
   R⁴ is selected from: H, D, halogen, OH, SH, -NR^{4a}R^{4b}, CN, C₁₋₆ alkyl, C₁₋₆ haloalkyl, C₂₋₆ alkenyl, C₂₋₆ haloalkenyl, -C₁₋₆ alkylene-O-C₁₋₆ alkyl, -C₁₋₆ alkylene-OH, -C₁₋₆ alkylene-SH, -C₁₋₆ alkylene-CN, and -C₁₋₆ alkylene-NR^{4a}R^{4b};
   R⁵ is selected from: halogen, OH, SH, -NR^{5a}R^{5b}, CN, C₁₋₆ alkyl, C₁₋₆ haloalkyl, C₂₋₆ alkenyl, C₂₋₆ haloalkenyl, -O-C₁₋₆ alkyl, -O-C₁₋₆ haloalkyl, -S-C₁₋₆ alkyl, -C₁₋₆ alkylene-O-C₁₋₆ alkyl, -OC₁₋₆ alkylene-OC₁₋₆ alkyl, -C₁₋₆ alkylene-OH, -C₁₋₆ alkylene-SH, -C₁₋₆ alkylene-CN, -C₁₋₆ alkylene-NR^{5a}R^{5b}, the cyclic group Cy2, and -C₁₋₆ alkylene-Cy2, wherein the cyclic group Cy2 is selected from C₃₋₁₀ cyclohydrocarbyl, 3- to 10-membered heterocyclyl, C₆₋₁₀ aryl, and 5- to 10-membered heteroaryl, wherein the C₃₋₁₀ cyclohydrocarbyl, 3- to 10-membered heterocyclyl, C₆₋₁₀ aryl, and 5- to 10-membered heteroaryl are each optionally substituted with one or more substituents independently selected from the following groups: halogen, OH, SH, -NR^{5a}R^{5b}, CN, C₁₋₆ alkyl, C₁₋₆ haloalkyl, C₂₋₆ alkenyl, C₂₋₆ haloalkenyl, -O-C₁₋₆ alkyl, -O-C₁₋₆ haloalkyl, -S-C₁₋₆ alkyl, -C₁₋₆ alkylene-O-C₁₋₆ alkyl, -OC₁₋₆ alkylene-OC₁₋₆ alkyl, -C₁₋₆ alkylene-OH, -C₁₋₆ alkylene-SH, -C₁₋₆ alkylene-CN, and -C₁₋₆ alkylene-NR^{5a}R^{5b};
   R⁶ is selected from: halogen, OH, SH, -NR^{6a}R^{6b}, CN, C₁₋₆ alkyl, C₁₋₆ haloalkyl, C₂₋₆ alkenyl, C₂₋₆ haloalkenyl, -O-C₁₋₆ alkyl, -O-C₁₋₆ haloalkyl, -S-C₁₋₆ alkyl, -C₁₋₆ alkylene-O-C₁₋₆ alkyl, -OC₁₋₆ alkylene-OC₁₋₆ alkyl, -C₁₋₆ alkylene-OH, -C₁₋₆ alkylene-SH, -C₁₋₆ alkylene-CN, and -C₁₋₆ alkylene-NR^{6a}R^{6b};
   n is 0, 1, 2, 3, or 4; and
   R¹⁶, R^{1e}, R^{1f}, R^{2a}, R^{2b}, R^{3a}, R^{3b}, R^{4a}, R^{4b}, R^{5a}, R^{5b}, R^{6a}, and R^{6b} are, at each occurrence, independently selected from H and C₁₋₆ alkyl.

In some embodiments, the present invention provides the compound of formula (I) according to the present invention, wherein:
the compound of formula (I) is not 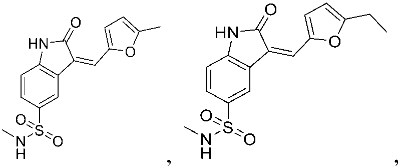 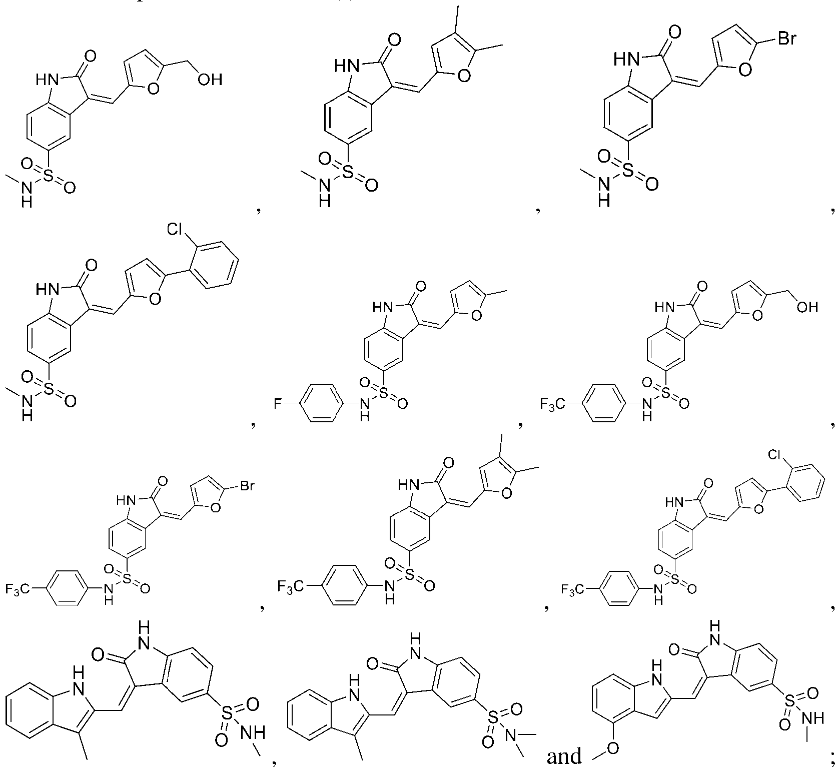 and when ring A is indolyl, R⁵ is not

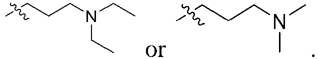

In some embodiments, the present invention provides the compound of formula (I) according to the present invention, wherein:
the compound of formula (I) satisfies at least one of the following (1) to (5): 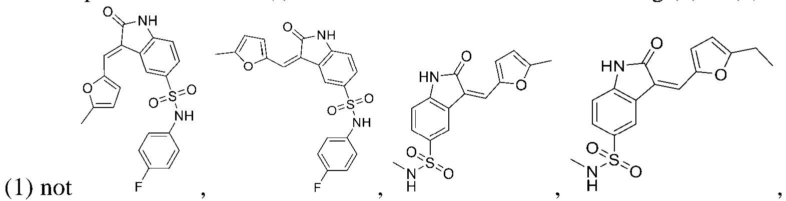 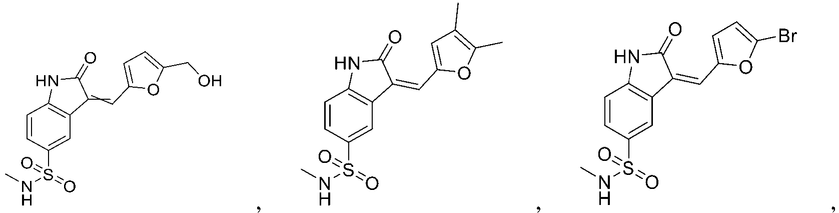 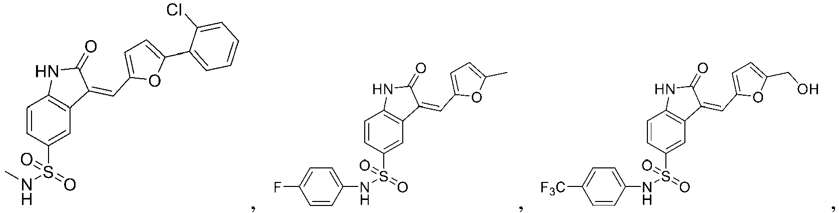 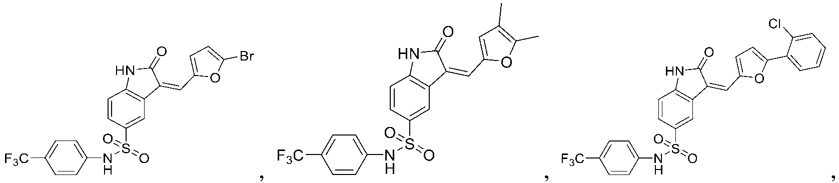 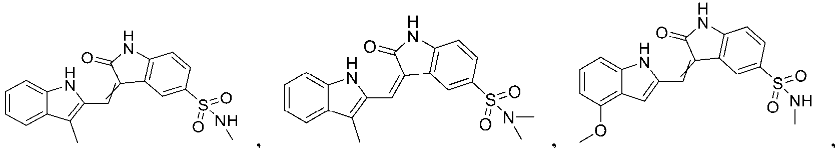 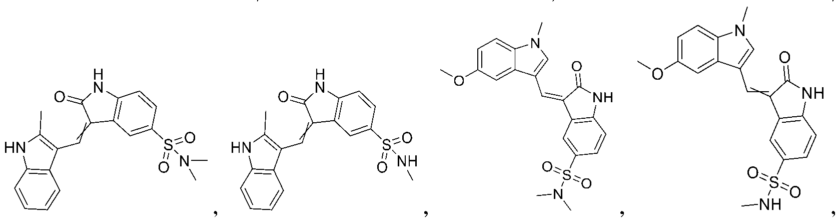 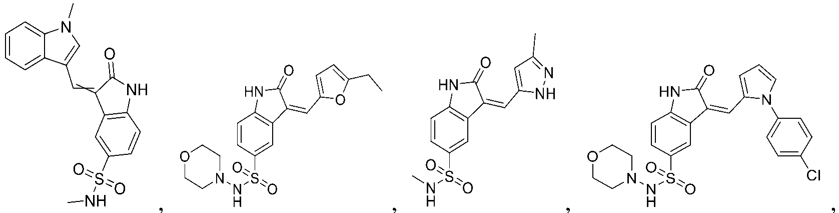 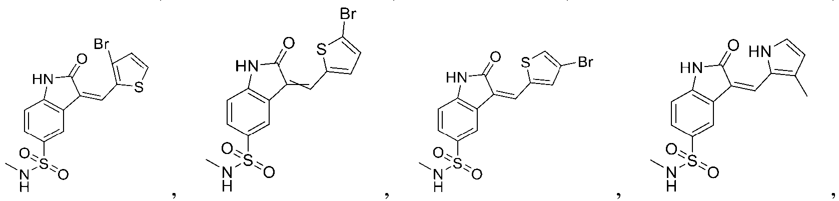 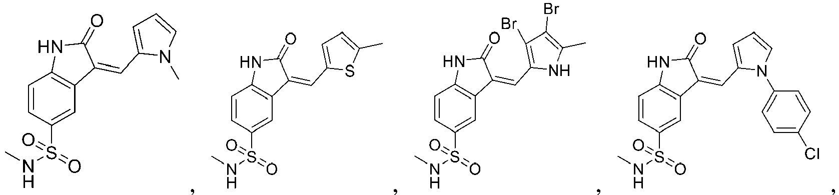 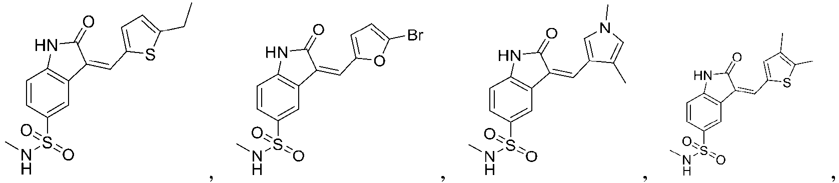 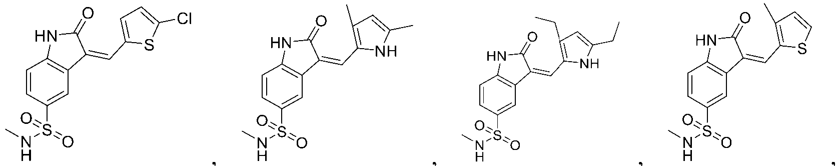 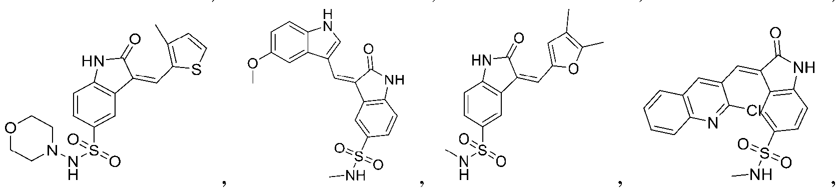 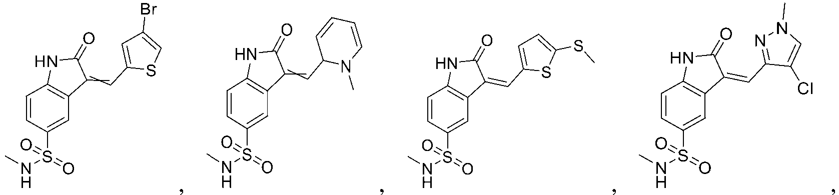 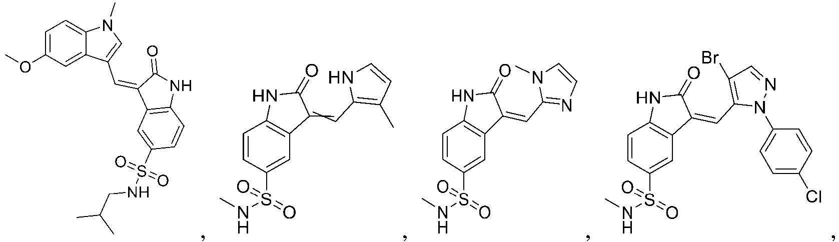 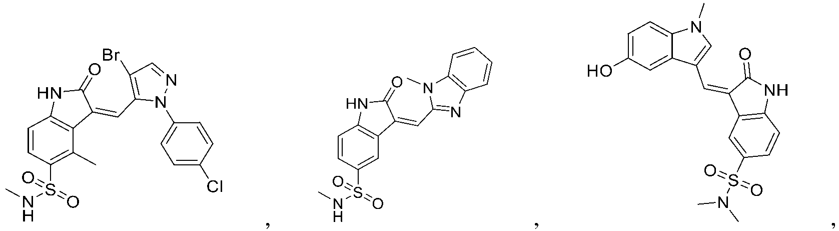 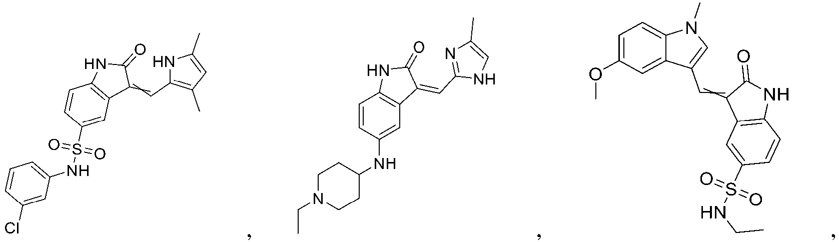 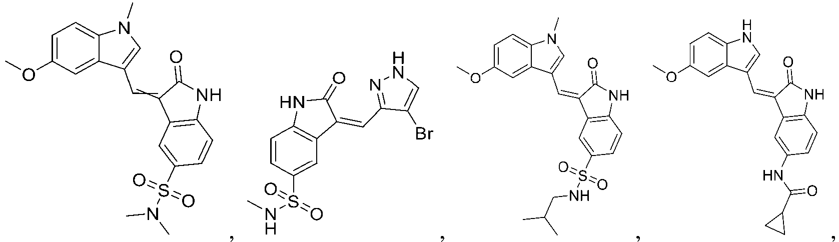 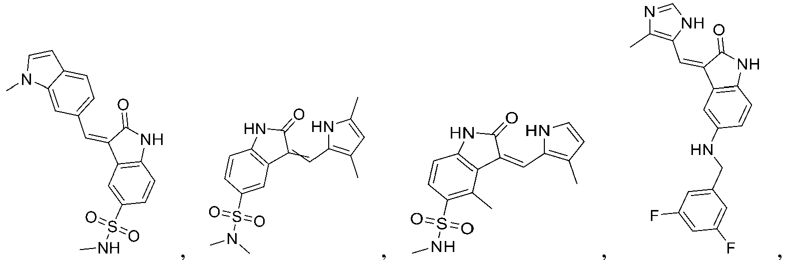 and 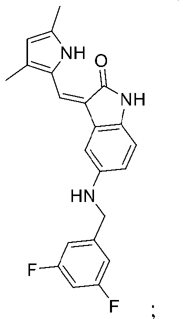
(2) when ring A is indolyl, pyrrolyl, 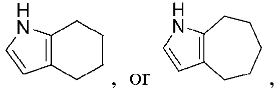 R⁵ is not 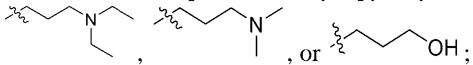
(3) -L²-R^{1a} is not 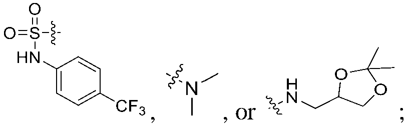
(4) ring A is not tetrahydropyrrolyl, tetrahydrofuryl, tetrahydrothienyl, 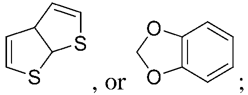
(5) when ring A is thienyl or pyrrolyl, -L²-R^{1a} is not

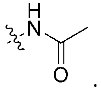

In some embodiments, the present invention provides the compound of formula (I) according to the present invention, wherein:
the compound of formula (I) satisfies the above conditions (1) to (5).

In some embodiments, the present invention provides the compound of formula (I) according to the present invention, wherein the compound has a structure of formula (II):

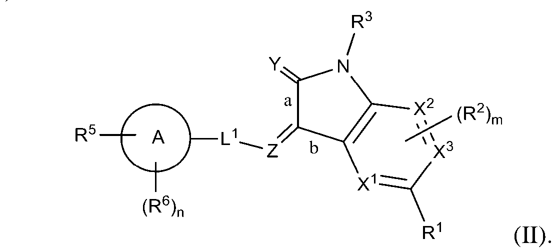

In some embodiments, the present invention provides the compound of formula (I) according to the present invention, including a compound of formula (II), wherein Y is O.

In some embodiments, the present invention provides the compound of formula (I) according to the present invention, including the compound of formula (II), wherein ring A is selected from C₃₋₁₀ monocyclic or fused bicyclic cyclohydrocarbyl, 5- to 10-membered monocyclic or fused bicyclic heterocyclyl, C₆₋₁₀ aryl, and 5- to 10-membered monocyclic or fused bicyclic heteroaryl.

In some preferred embodiments, ring A is selected from: C₅₋₆ monocyclic cyclohydrocarbyl, C₉₋₁₀ fused bicyclic cyclohydrocarbyl, 5- to 6-membered monocyclic heterocyclyl, 9- to 10-membered fused bicyclic heterocyclyl, phenyl, naphthyl, 5- to 6-membered monocyclic heteroaryl, and 9- to 10-membered fused bicyclic heteroaryl.

In some more preferred embodiments, ring A is selected from: 9- to 10-membered fused bicyclic heterocyclyl, 5- to 6-membered monocyclic heteroaryl, and 9- to 10-membered fused bicyclic heteroaryl.

In some more preferred embodiments, ring A is selected from: 5- to 6-membered monocyclic heteroaryl, C₅₋₆ monocyclic cyclohydrocarbyl fused 5- to 6-membered monocyclic heteroaryl, 5- to 6-membered monocyclic heterocyclyl fused 5- to 6-membered monocyclic heteroaryl, benzo 5- to 6-membered monocyclic heteroaryl, and 5- to 6-membered monocyclic heteroaryl fused 5- to 6-membered monocyclic heteroaryl. In some preferred embodiments, the ring A is connected to L¹ via the 5- to 6-membered monocyclic heteroaryl ring.

In some more preferred embodiments, ring A is selected from: 5-membered monocyclic heteroaryl, and BiCy which is selected from the following fused bicyclic groups: C₆ monocyclic cyclohydrocarbyl fused 5-membered monocyclic heteroaryl, 6-membered monocyclic heterocyclyl fused 5-membered monocyclic heteroaryl, benzo 5-membered monocyclic heteroaryl, and 6-membered monocyclic heteroaryl fused 5-membered monocyclic heteroaryl. In some preferred embodiments, the ring A is connected to L¹ via the 5-membered monocyclic heteroaryl ring. In some preferred embodiments, the 5-membered monocyclic heteroaryl is selected from furyl, thienyl, oxazolyl, isoxazolyl, thiazolyl, isothiazolyl, oxadiazolyl, thiadiazolyl, pyrrolyl, pyrazolyl, imidazolyl, triazolyl, and tetrazolyl.

In some more preferred embodiments, ring A is selected from: furyl, thienyl, oxazolyl, isoxazolyl, thiazolyl, isothiazolyl, pyrrolyl, pyrazolyl, imidazolyl, and triazolyl, and BiCy which is selected from the following fused bicyclic groups: C₆ monocyclic cyclohydrocarbyl fused 5-membered monocyclic heteroaryl, 6-membered monocyclic heterocyclyl fused 5-membered monocyclic heteroaryl, benzo 5-membered monocyclic heteroaryl, and 6-membered monocyclic heteroaryl fused 5-membered monocyclic heteroaryl, wherein the 5-membered monocyclic heteroaryl is selected from furyl, thienyl, oxazolyl, isoxazolyl, thiazolyl, isothiazolyl, pyrrolyl, pyrazolyl, imidazolyl, and triazolyl. In some preferred embodiments, ring A is connected to L¹ via the furyl, thienyl, oxazolyl, isoxazolyl, thiazolyl, isothiazolyl, pyrrolyl, pyrazolyl, imidazolyl, or triazolyl.

In some preferred embodiments, the bicyclic group BiCy has a structure of formula (a), (b), or (c): wherein: 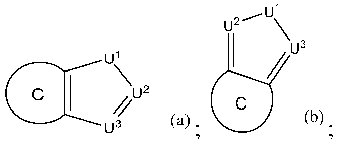
U¹ is O, S, or NH,
U² and U³ are each CH or N; and
at least one of U¹, U², and U³ is CH and connected to L¹ (in this case, H in the CH is replaced); and
ring C is C₆ monocyclic cyclohydrocarbyl, 6-membered monocyclic heterocyclyl, phenyl, or 6-membered monocyclic heteroaryl;
or 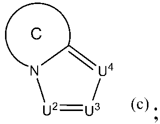 wherein:
   U², U³, and U⁴ are each CH or N; and
   at least one of U², U³, and U⁴ is CH and connected to L¹ (in this case, H in the CH is replaced); and
   ring C is C₆ monocyclic cyclohydrocarbyl, 6-membered monocyclic heterocyclyl, or 6-membered monocyclic heteroaryl.

In some more preferred embodiments, ring A is selected from:
(1) 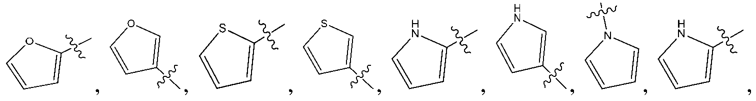 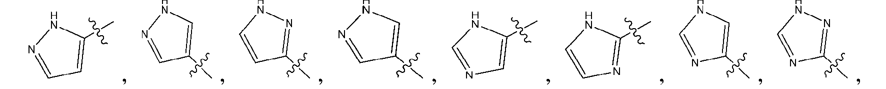 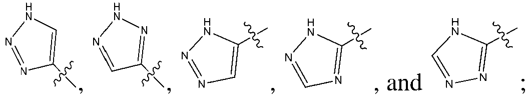 and
(2) the bicyclic group BiCy having a structure of one of formulas (a-1) - (a-16), formulas (b-1) - (b-12), and formulas (c-1) - (c-12):

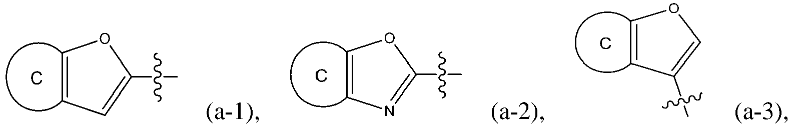

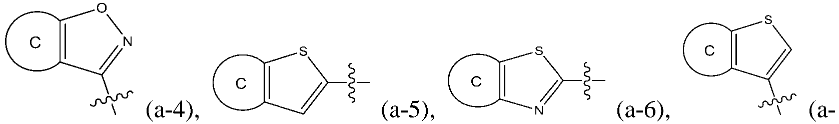

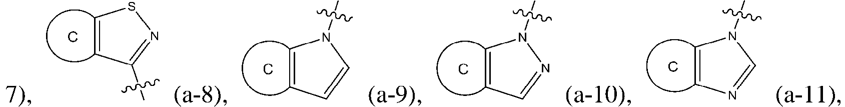

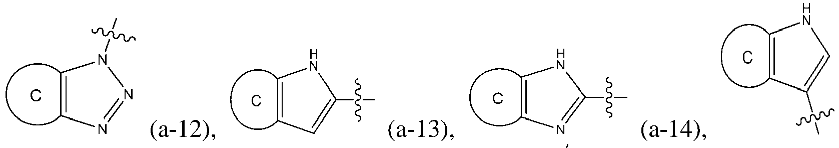

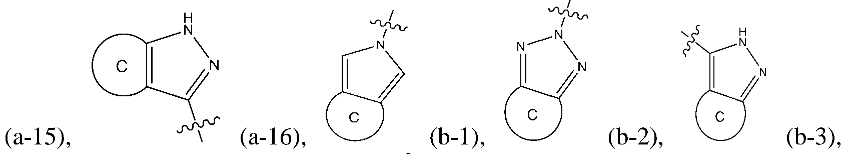

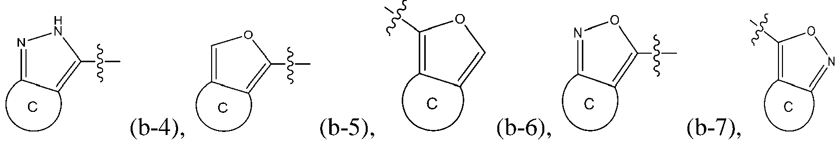

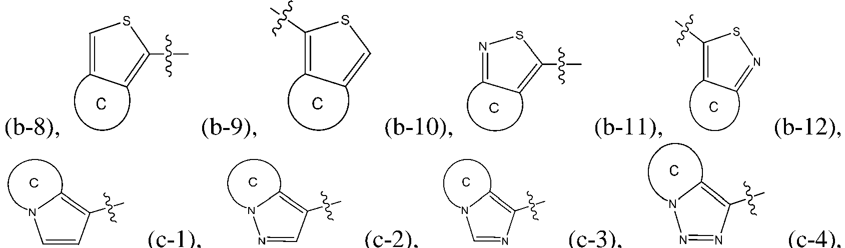

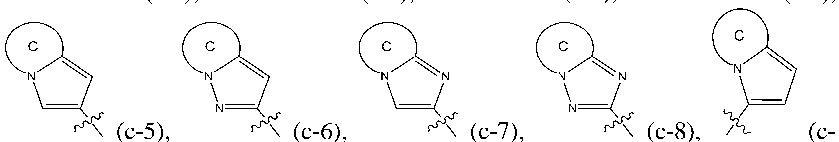

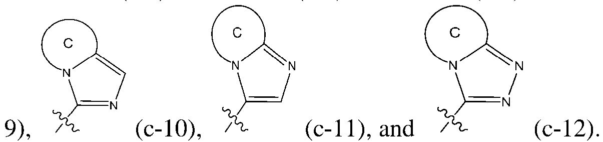

In some embodiments, the C₆ monocyclic cyclohydrocarbyl is cyclohexyl.

In some embodiments, the 6-membered monocyclic heterocyclyl is selected from tetrahydropyridinyl, piperidinyl, tetrahydropyrazinyl, piperazinyl, dihydropyranyl, tetrahydropyranyl, dihydrothiopyranyl, tetrahydrothiopyranyl, dihydrooxazinyl, morpholinyl, dihydrothiazinyl, and thiomorpholinyl, preferably tetrahydropyridinyl or piperidinyl.

In some embodiments, the 6-membered monocyclic heteroaryl is selected from pyridinyl, pyrazinyl, pyridazinyl, pyrimidinyl, and triazinyl, preferably pyridinyl.

In some embodiments, ring C is phenyl, pyridinyl, tetrahydropyridinyl, or piperidinyl.

In some embodiments, the bicyclic group BiCy is selected from benzofuryl, benzothienyl, indolyl, indazolyl, benzimidazolyl, benzotriazolyl, pyrrolopyridinyl, pyrazolopyridinyl, imidazolopyridinyl, pyrrolotetrahydropyridinyl, pyrazolotetrahydropyridinyl, and imidazolotetrahydropyridinyl.

In some embodiments, the present invention provides the compound of formula (I) according to the present invention, including a compound of formula (II), wherein:
the moiety 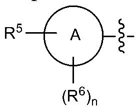 is selected from: 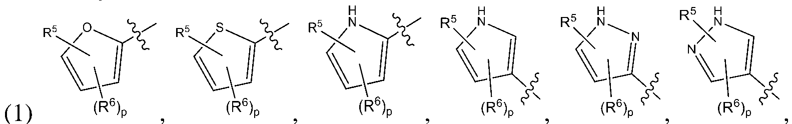 and 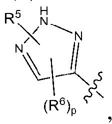 wherein p is 0, 1, or 2;
preferably 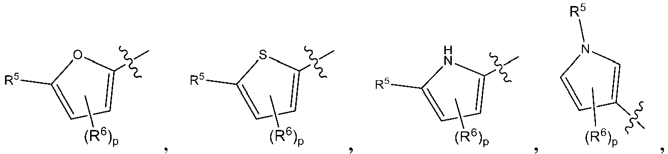 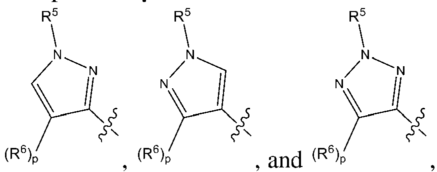 wherein is 0 or 1;
(2) a structure of formula (a-1'), (a-5'), or (c-1'): 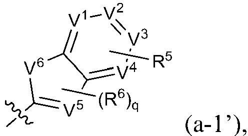 wherein V⁶ is NH, O, or S;
preferably 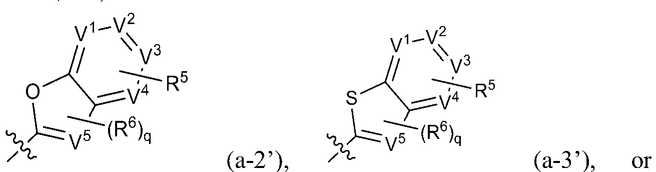 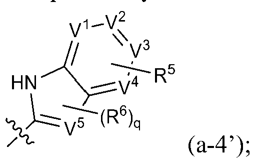 wherein in any one of formulas (a-1') - (a-4'), V¹, V², V³, V⁴, and V⁵ are each independently N or CH; In some preferred embodiments, V¹, V², and V³, or V², V³, and V⁴ are not simultaneously N; in some preferred embodiments, V¹, V², V³, and V⁴ are each CH; or in some other preferred embodiments, one of V¹, V², V³, and V⁴, preferably V⁴ is N, and the rest are all CH;
or 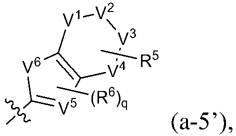 wherein V⁶ is NH, O, or S;
preferably 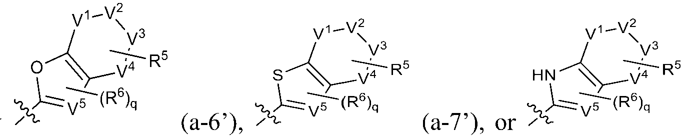 (a-8'),
wherein in any one of formulas (a-5') - (a-8'), V¹, V², V³, and V⁴ are each independently NH or CH₂, and V⁵ is N or CH; in some preferred embodiments, V¹, V², and V³, or V², V³, and V⁴ are not simultaneously NH; in some preferred embodiments, V¹, V², V³, and V⁴ are each CH₂; or in some other preferred embodiments, one of V¹, V², V³, and V⁴, preferably V³ is NH, and the rest are all CH₂; in some preferred embodiments, V⁵ is CH;
or 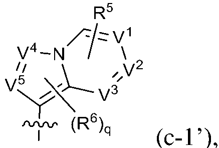 wherein V⁴ and V⁵ are each independently N or CH, preferably V⁴ and V⁵ are not simultaneously N;
preferably 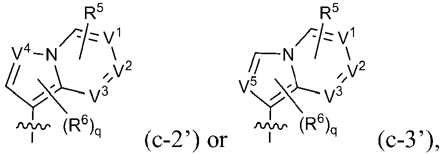
wherein in any one of formulas (c-1') - (c-3'), V¹, V², and V³ are each independently N or CH; in some preferred embodiments, V¹, V², and V³ are not simultaneously N; in some other preferred embodiments, V¹, V², and V³ are each CH; or in some other preferred embodiments, one of V¹, V², and V³ is N, and the rest are both CH; and
q is 0, 1, 2, 3, or 4.

In some preferred embodiments, the moiety 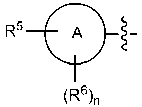 is selected from: 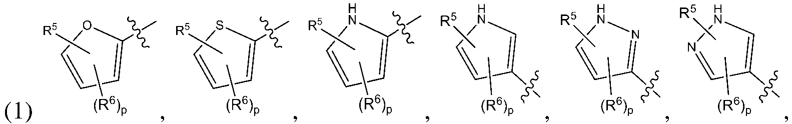 and 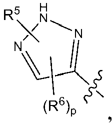 wherein p is 0, 1, or 2; and (2) a structure of one of formulas (a-1') - (a-6') and formulas (c-1') - (c-2').

In some preferred embodiments, the moiety 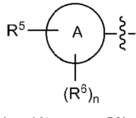 is selected from structures of formulas (a-9') - (a-14') and formulas (c-4') - (c-5'): 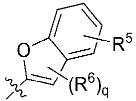 (preferably 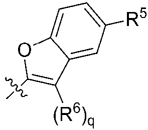 ) (a-9'), 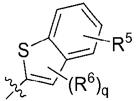 (preferably 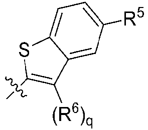 ) (a-10'), 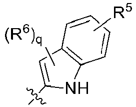 (preferably 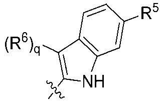 ) (a-11"), 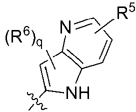 ( 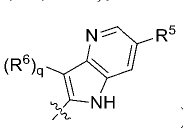 ) (a-12'), 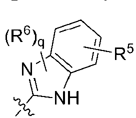 (preferably 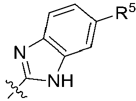 ) (a-13'), 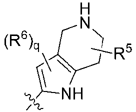 (preferably 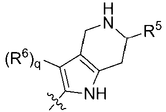 ) (a-14'), 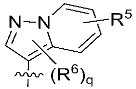 (preferably 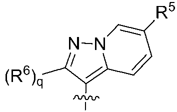 ) (c-4'), 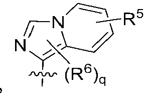 (preferably 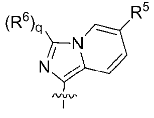 ) (c-5'),
wherein q is 0, 1, 2, 3, or 4, preferably 0 or 1.

In some embodiments, the present invention provides the compound of formula (I) according to the present invention, including the compound of formula (II), wherein the compound has a structure of one of formulas (III) - (X): 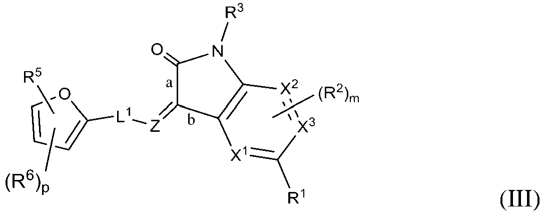 (preferably 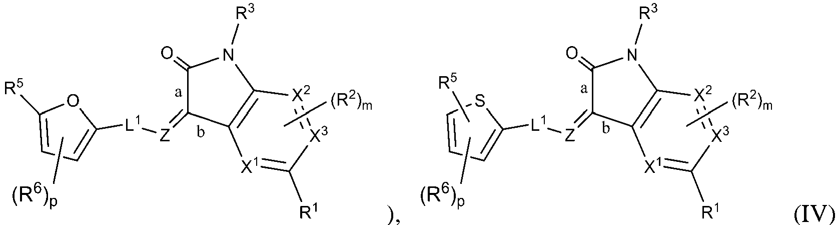 (preferably 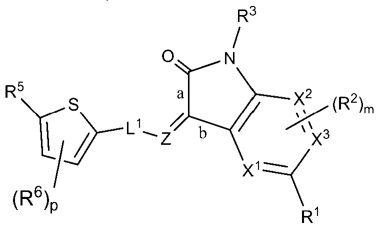 ), 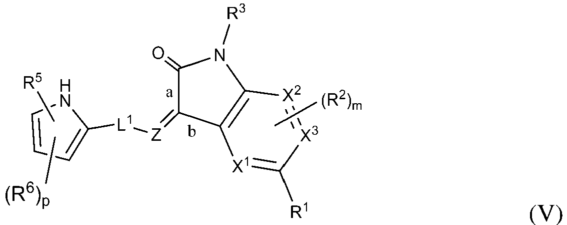 (preferably 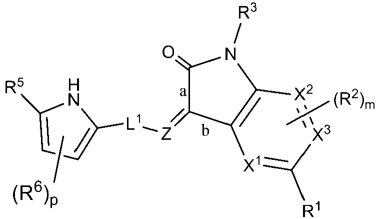 ), 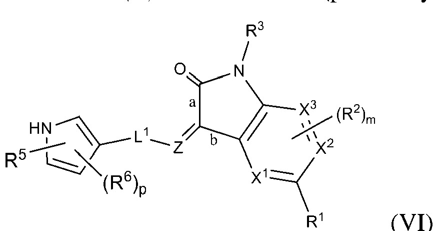 (preferably 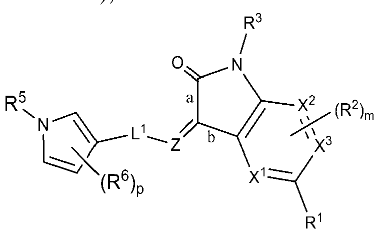 ), 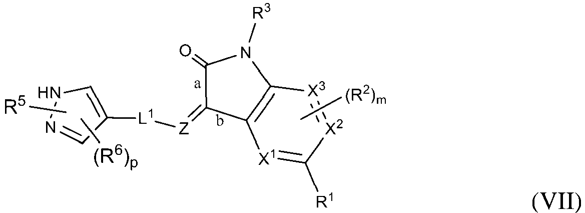 (preferably 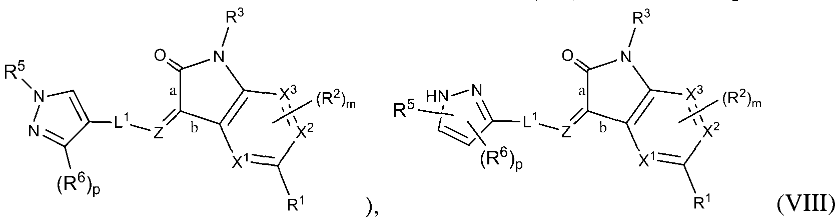 (preferably 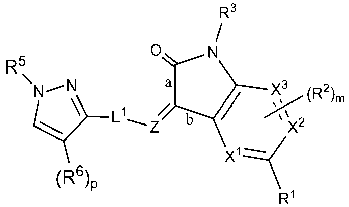 ), 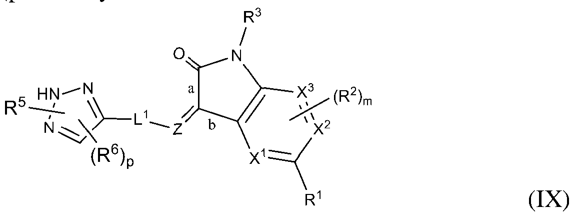 (preferably 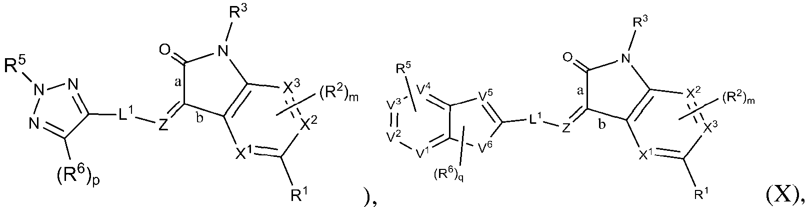
wherein V¹, V², V³, V⁴, V⁵, and V⁶ are each as defined hereinbefore with respect to formulas (a-1') - (a-4');
preferably 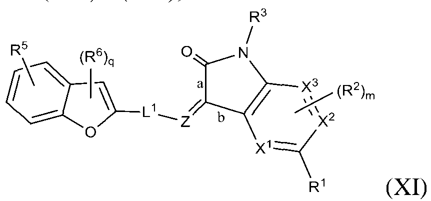 (preferably 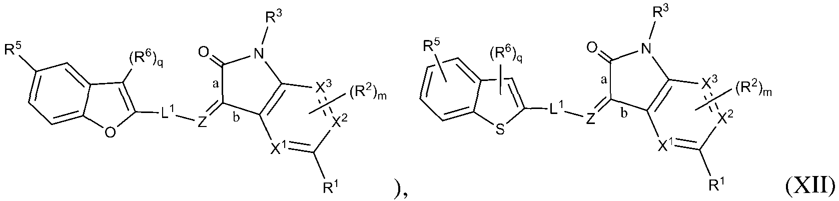 (preferably 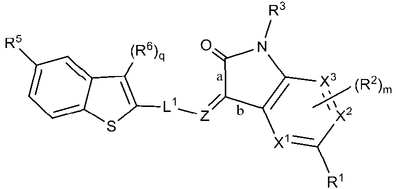 ), 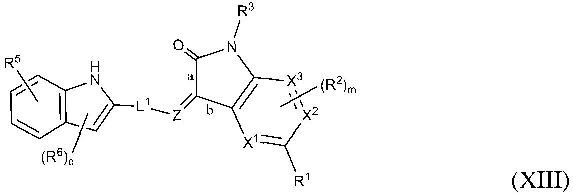 (preferably 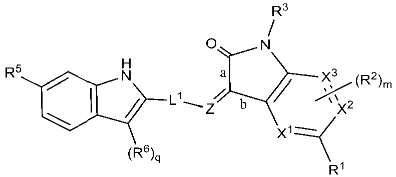 ), 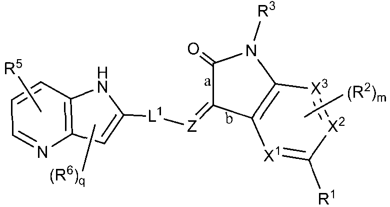 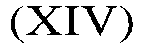 (preferably 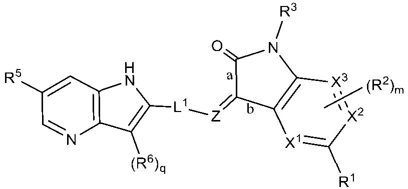 ), 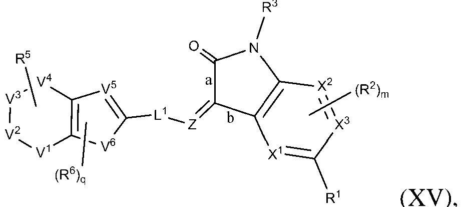
wherein V¹, V², V³, V⁴, V⁵, and V⁶ are each as defined hereinbefore with respect to formulas (a-5') - (a-8');
preferably 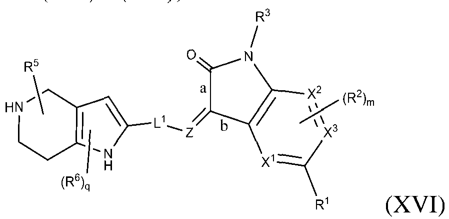 (preferably 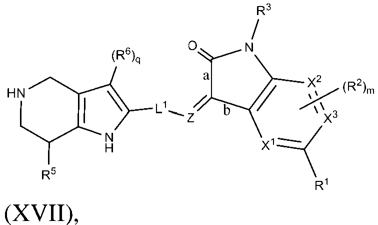 ) 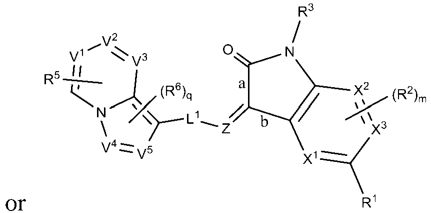
wherein V¹, V², V³, V⁴, and V⁵ are each as defined hereinbefore with respect to the formulas (c-1') - (c-3');
preferably 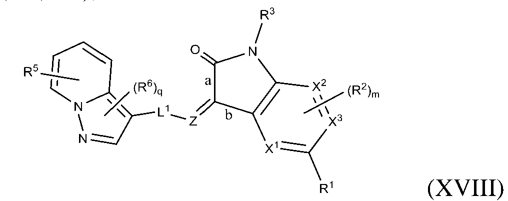 (preferably 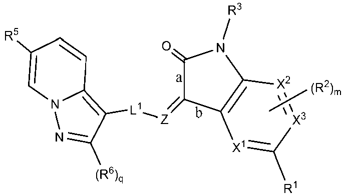 ),
wherein p is 0, 1, or 2, preferably 0 or 1; and
q is 0, 1, 2, 3, or 4, preferably 0 or 1.

In some embodiments, the present invention provides the compound of formula (I) according to the present invention, including the compound of formula (II), wherein the compound has a structure of one of formulas (III) - (XVIII).

In some embodiments, the present invention provides the compound of formula (I) according to the present invention, including the compounds of formulas (II) - (XVIII), wherein R^{1a} is cyclic group Cy1, and the cyclic group Cy1 is selected from C₃₋₁₀ cyclohydrocarbyl, 3- to 10-membered heterocyclyl, C₆₋₁₀ aryl, and 5- to 10-membered heteroaryl, wherein the C₃₋₁₀ cyclohydrocarbyl, 3- to 10-membered heterocyclyl, C₆₋₁₀ aryl, and 5- to 10-membered heteroaryl are each optionally substituted with one or more substituents independently selected from the following groups: halogen, OH, SH, -NR^{1e}R^{1f}, CN, C₁₋₄ alkyl, C₁₋₄ haloalkyl, C₂₋₄ alkenyl, C₂₋₄ haloalkenyl, -O-C₁₋₄ alkyl, -O-C₁₋₄ haloalkyl, -S-C₁₋₄ alkyl, -C₁₋₄ alkylene-O-C₁₋₄ alkyl, -OC₁₋₄ alkylene-OC₁₋₄ alkyl, -C₁₋₄ alkylene-OH, -C₁₋₄ alkylene-SH, -C₁₋₄ alkylene-CN, and -C₁₋₄ alkylene-NR^{1e}R^{1f}.

In some preferred embodiments, the cyclic group Cy1 is selected from C₃₋₆ cyclohydrocarbyl, 5- to 10-membered heterocycloalkyl, phenyl, and 5- to 10-membered heteroaryl, wherein the C₃₋₆ cyclohydrocarbyl, 5- to 10-membered heterocycloalkyl, phenyl, and 5- to 10-membered heteroaryl are each optionally substituted with one or more substituents independently selected from the following groups: halogen, OH, -NR^{1e}R^{1f}, CN, C₁₋₄ alkyl, C₁₋₄ haloalkyl, -O-C₁₋₄ alkyl, and - O-C₁₋₄ haloalkyl.

In some more preferred embodiments, the cyclic group Cy1 is selected from 5-to 6-membered heterocycloalkyl, phenyl, and 5- to 6-membered heteroaryl, wherein the 5- to 6-membered heterocycloalkyl, phenyl, and 5- to 6-membered heteroaryl are each optionally substituted with one or more substituents independently selected from the following groups: F, Cl, OH, -NH₂, CN, methyl, and ethyl.

In some more preferred embodiments R^{1a} is selected from

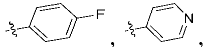

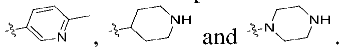

In some embodiments, the present invention provides the compound of formula (I) according to the present invention, including the compounds of formulas (II) - (XVIII), wherein the compound has a structure of one of formulas (XIX) - (XXVII): 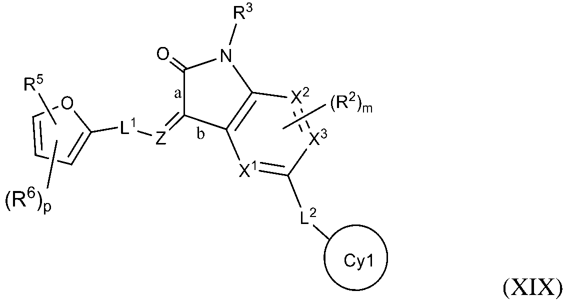 (preferably 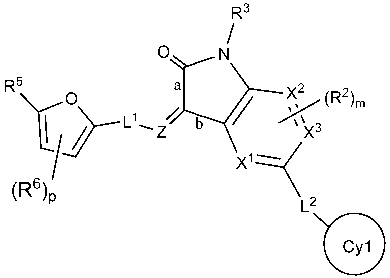 ), 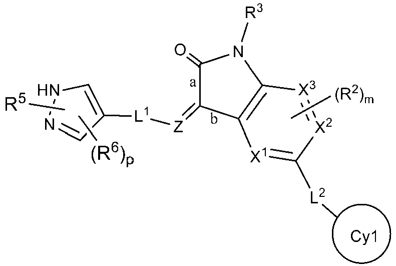 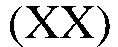 (preferably 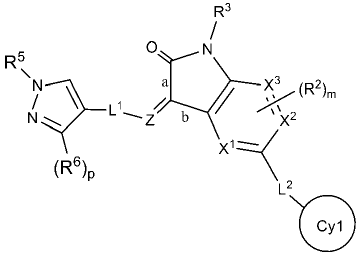 ), 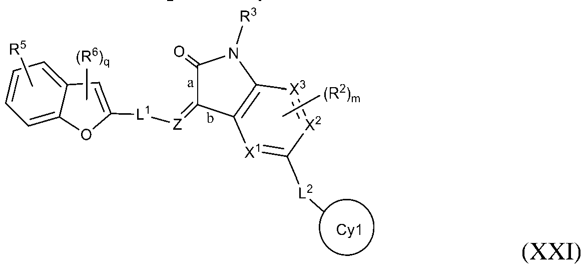 (preferably 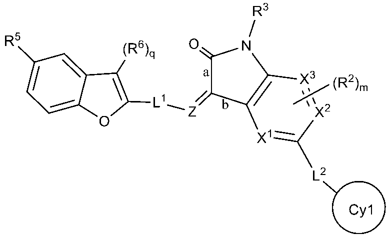 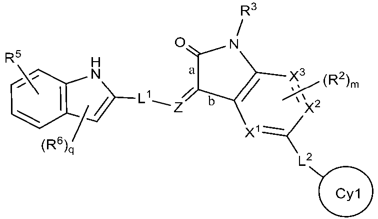 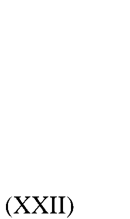 (preferably 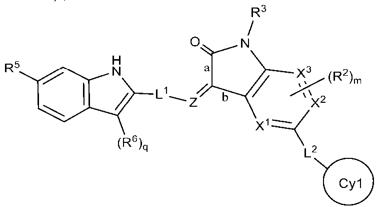 ), 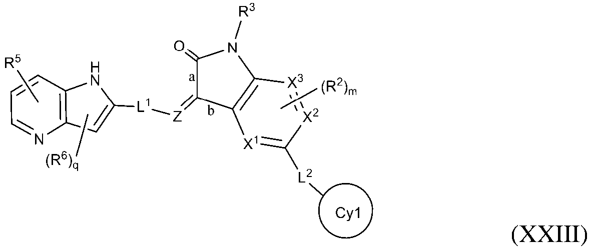 (preferably 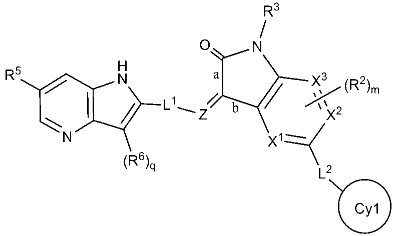 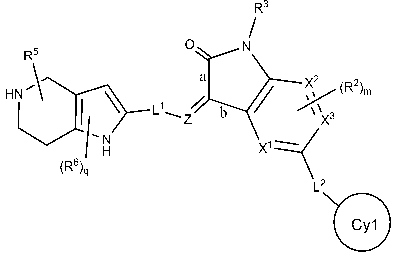 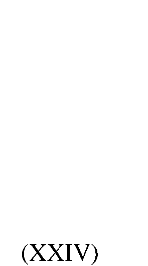 (preferably 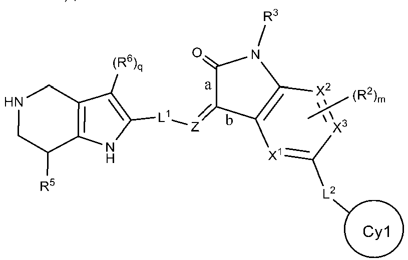 ), 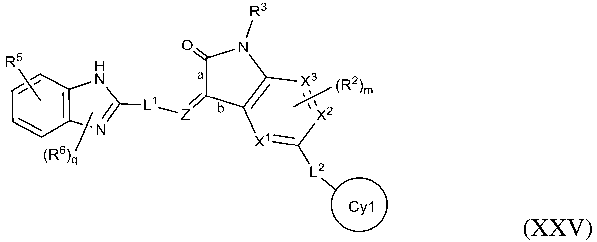 (preferably 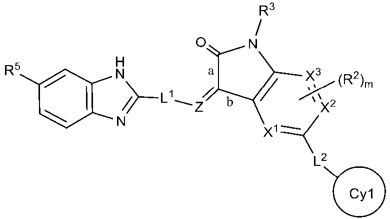 ), 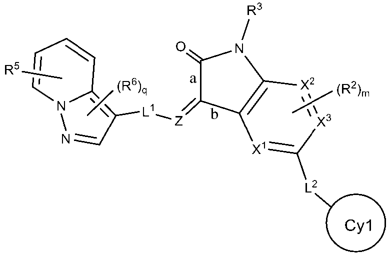 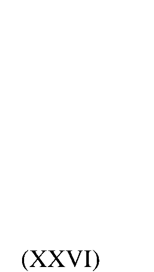 (preferably 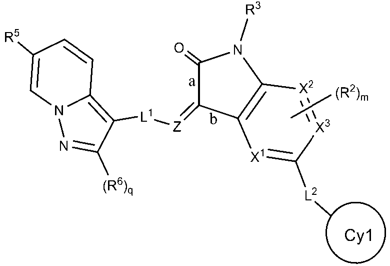 ), 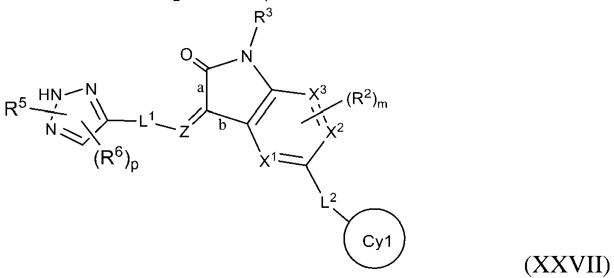 (preferably 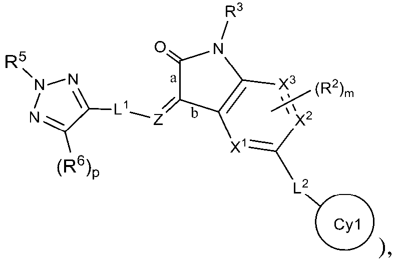 wherein:
p is 0, 1, or 2, preferably 0 or 1; and
q is 0, 1, 2, 3, or 4, preferably 0 or 1.

In some other embodiments, the present invention provides the compound of formula (I) according to the present invention, including the compounds of formulas (II) - (XVIII), wherein:
R^{1a} is selected from: C₁₋₄ alkyl, C₁₋₄ haloalkyl, C₂₋₄ alkenyl, C₂₋₄ haloalkenyl, - C₁₋₄ alkylene-O-C₁₋₄ alkyl, -OC₁₋₄ alkylene-OC₁₋₄ alkyl, -C₁₋₄ alkylene-OH, -C₁₋₄ alkylene-SH, -C₁₋₄ alkylene-CN, and -C₁₋₄ alkylene-NR^{1e}R^{1f}.

In some preferred embodiments, R^{1a} is selected from: C₁₋₄ alkyl and C₁₋₄ haloalkyl. More preferably, R^{1a} is selected from methyl and ethyl.

In some embodiments, the present invention provides the compound of formula (I) according to the present invention, including the compounds of formulas (II) - (XXII), wherein Z is N. In some such embodiments, the moiety 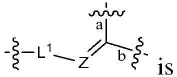 represented by a structure of formula (iii). In some such embodiments, the moiety 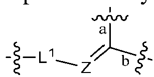 is represented by a structure of formula (iv).

In some other embodiments, Z is CR⁴. In some such embodiments, the moiety 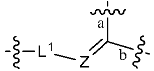 is represented by a structure of formula (i). In some such embodiments, the moiety 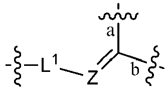 is represented by a structure of formula (ii).

In some embodiments, the present invention provides the compound of formula (I) according to the present invention, including the compounds of formulas (II) - (XXVII), wherein L¹ is selected from: a direct bond and NR ⁷. In some preferred embodiments, L¹ is a direct bond. In some other preferred embodiments, L¹ is NR⁷.

In some embodiments, the present invention provides the compound of formula (I) according to the present invention, including the compounds of formulas (II) - (XXVII), wherein the compound has a structure of one of formulas (1) - (37): 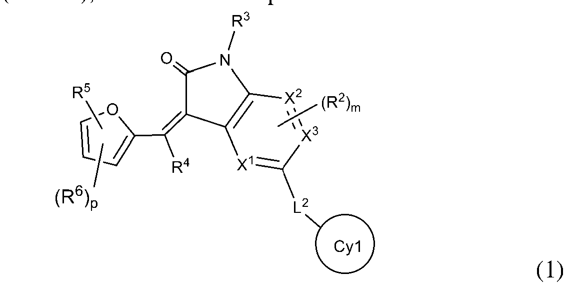 (preferably 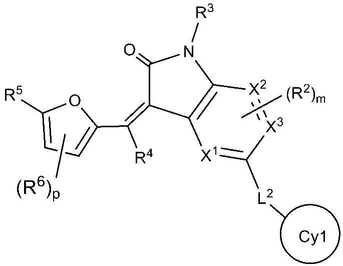 ), 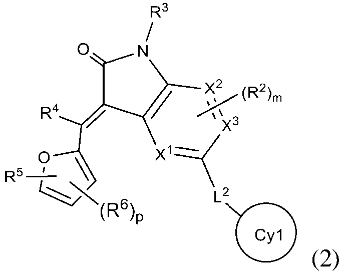 (preferably 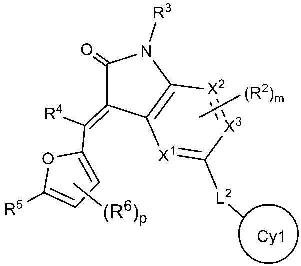 ), 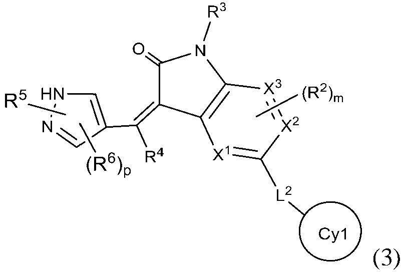 (preferably 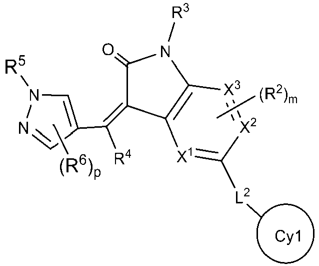 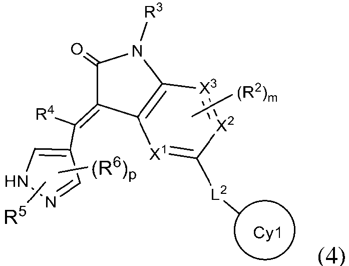 (preferably 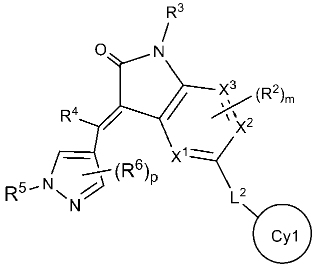 ), 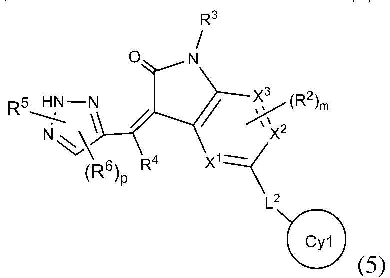 (preferably 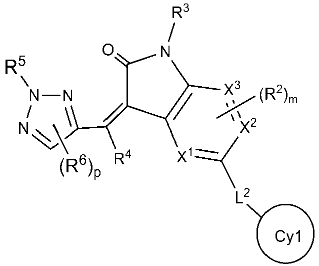 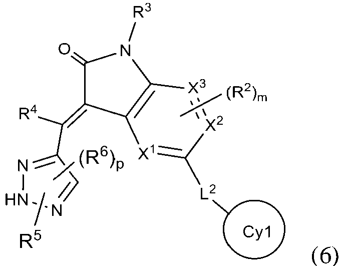 (preferably 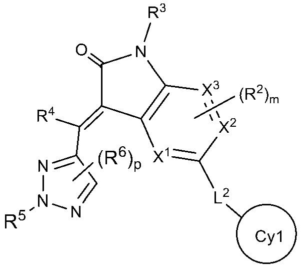 ), 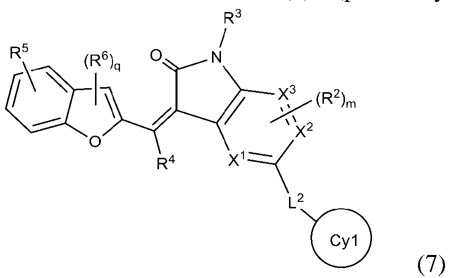 (preferably 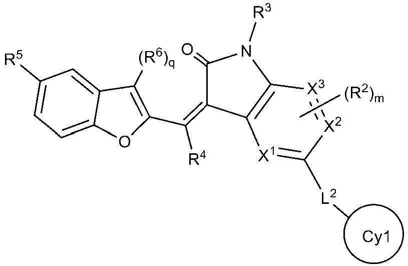 ), 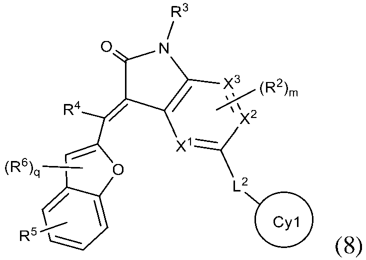 (preferably 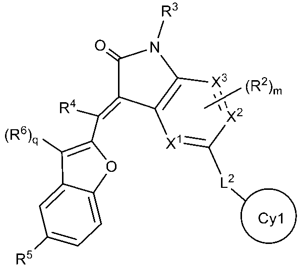 ), 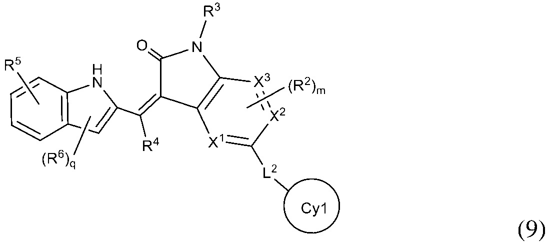 (preferably 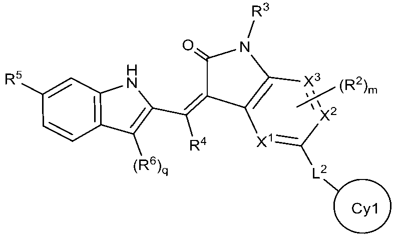 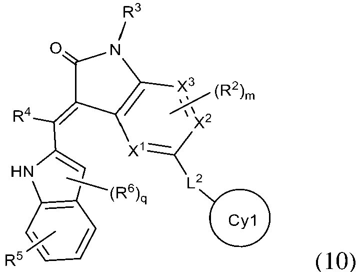 (preferably 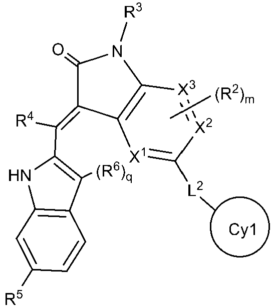 ), 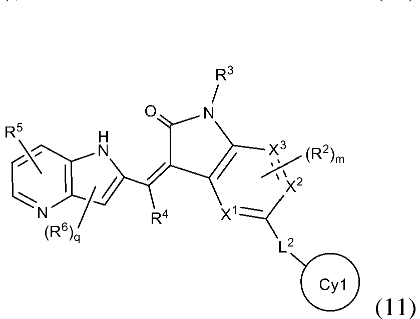 (preferably 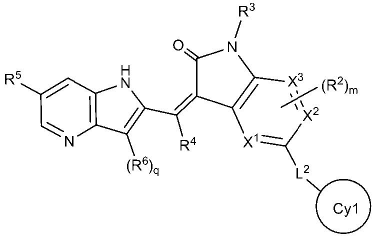 ), 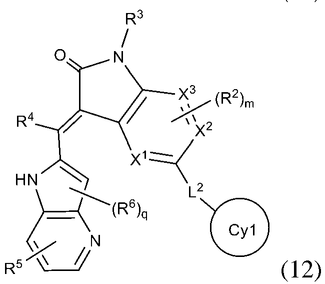 (preferably 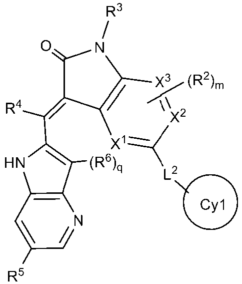 ), 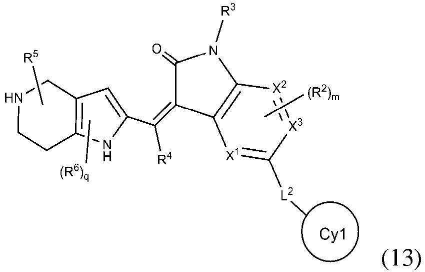 (preferably 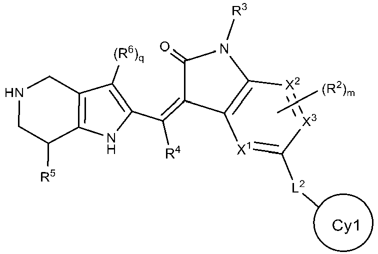 ), 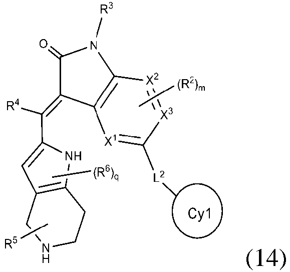 (preferably 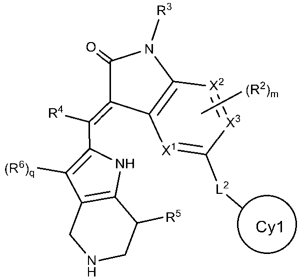 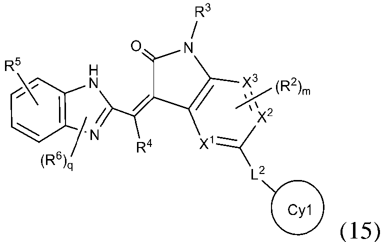 (preferably 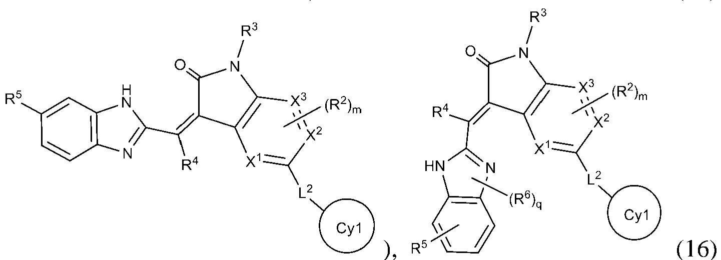 (preferably 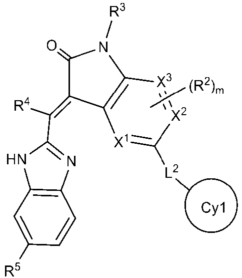 ), 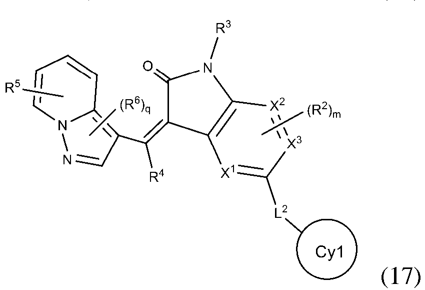 (preferably 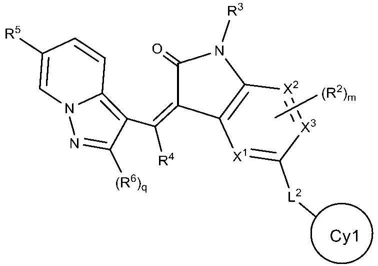 ), 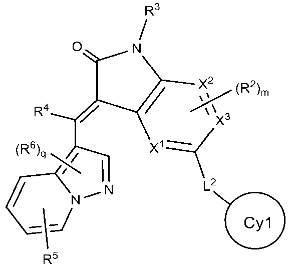 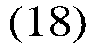 (preferably 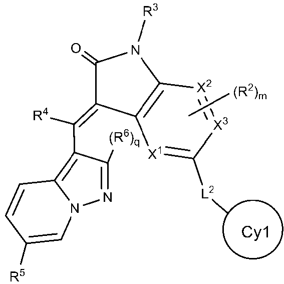 ), 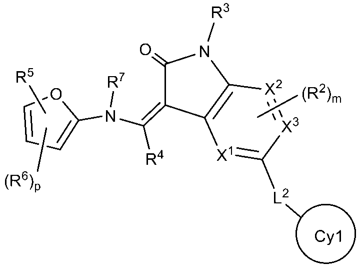 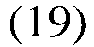 (preferably 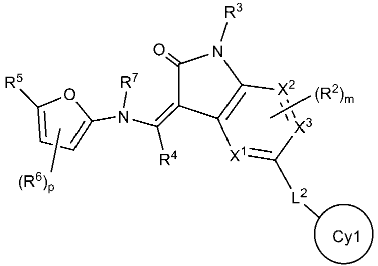 ), 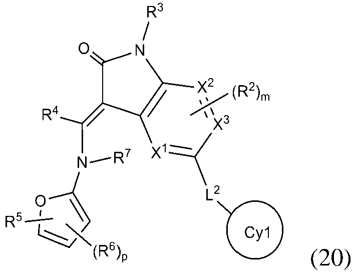 (preferably 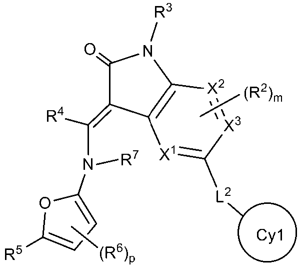 ), 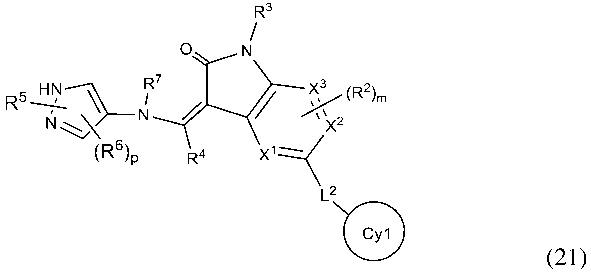 (preferably 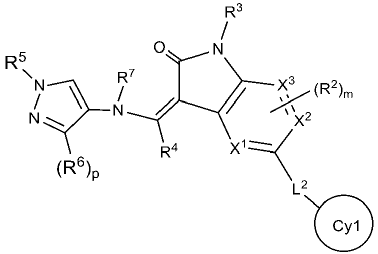 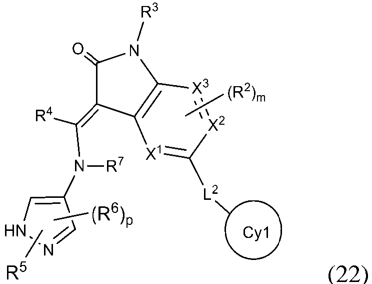 (preferably 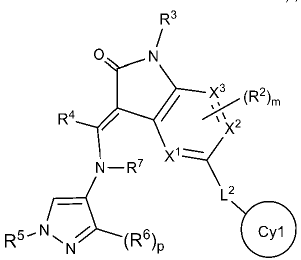 ), 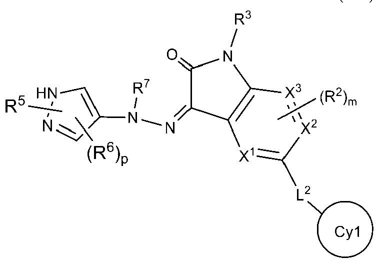 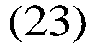 (preferably 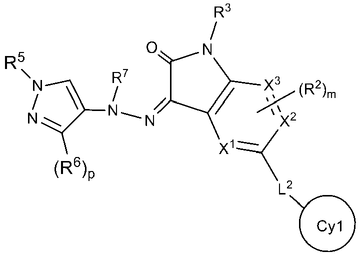 ), 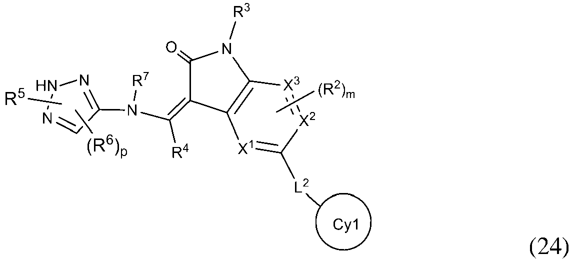 (preferably 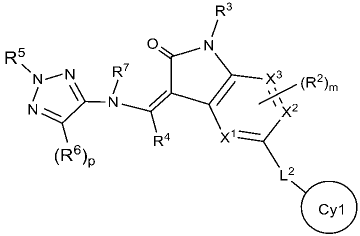 ), 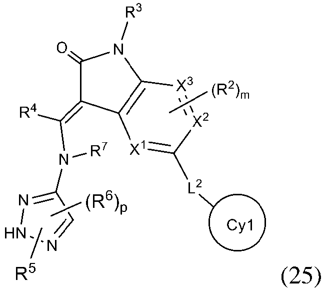 (preferably 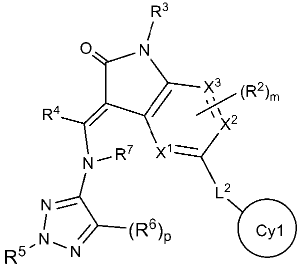 ), 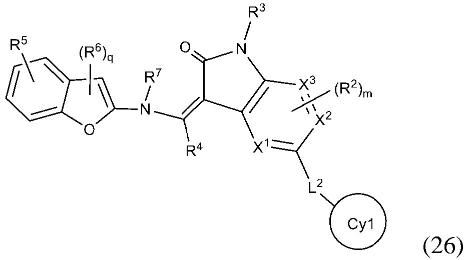 (preferably 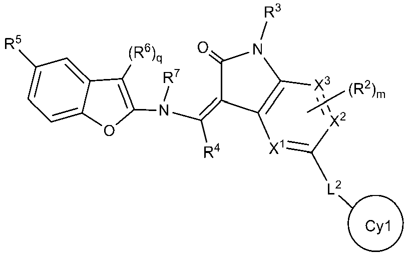 ), 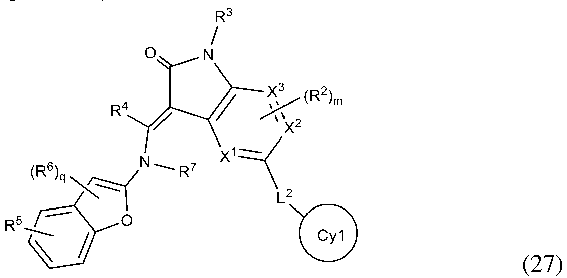 (preferably 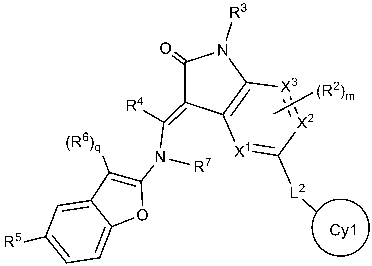 ), 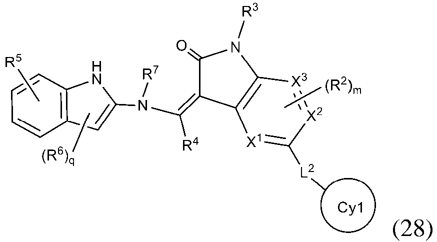 (preferably 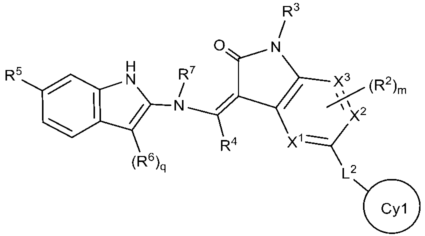 ), 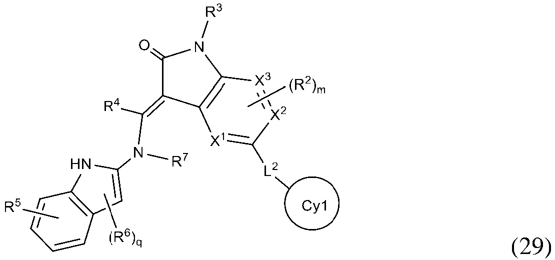 (preferably 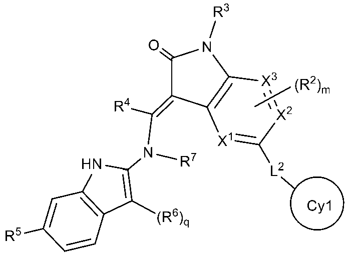 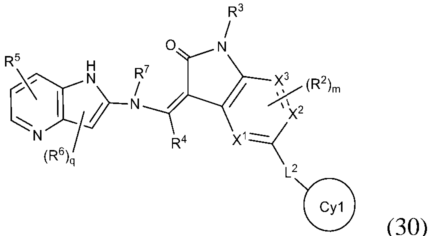 (preferably 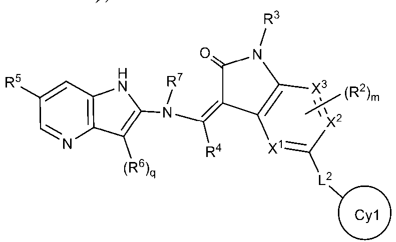 ), 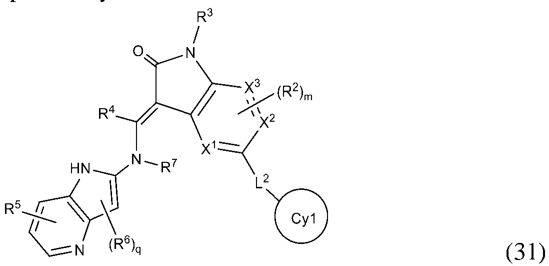 (preferably 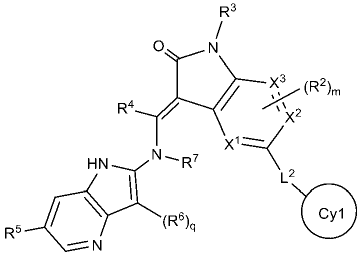 ), 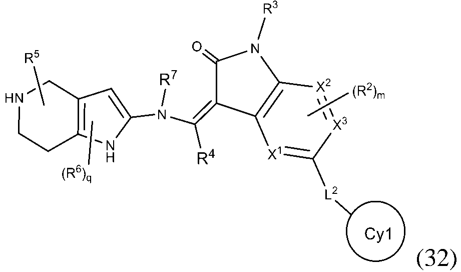 (preferably 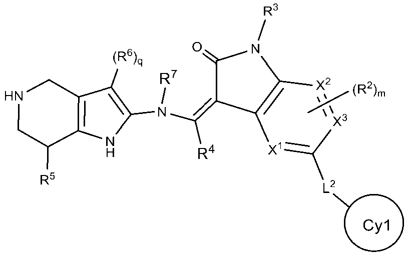 ), 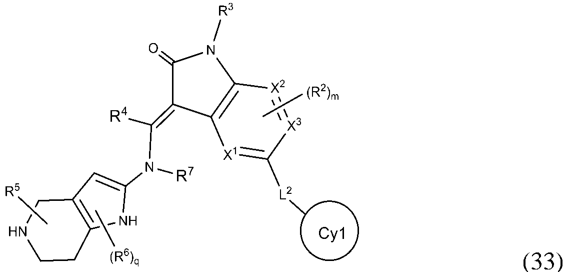 (preferably 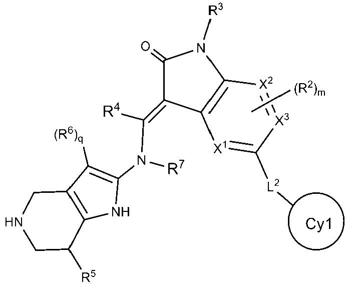 ), 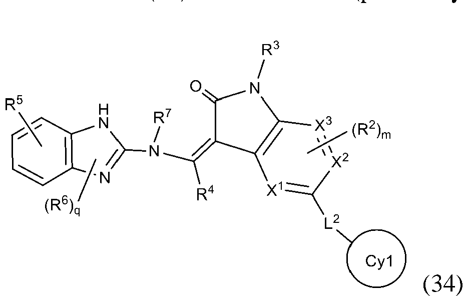 (preferably 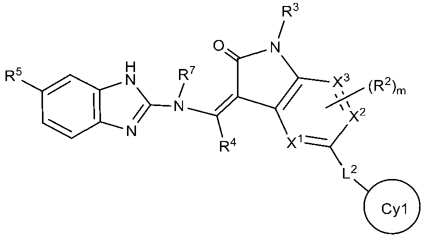 ), 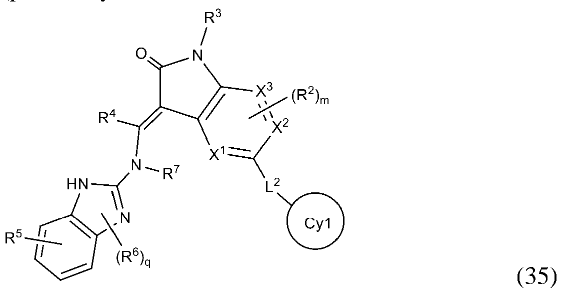 (preferably 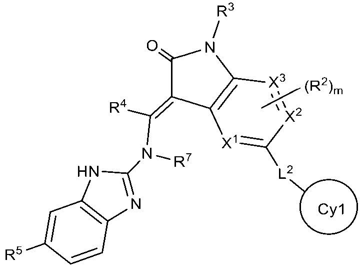 ), 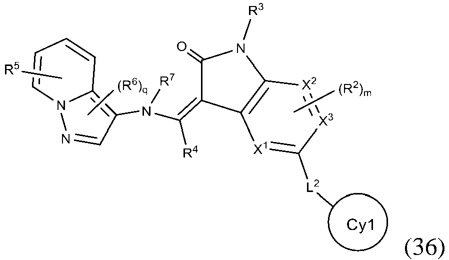 (preferably 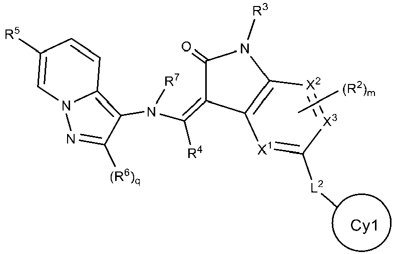 ), 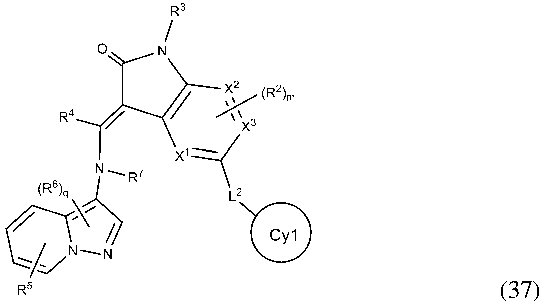 (preferably 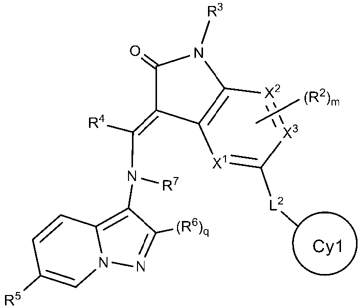 ), wherein:
p is 0, 1, or 2, preferably 0 or 1; and
q is 0, 1, 2, 3, or 4, preferably 0 or 1.

In some embodiments, the present invention provides the compound of formula (I) according to the present invention, including the compounds of formulas (II) - (XXII) and formulas (1) - (19), wherein X¹, X², and X³ are each CH. In some other embodiments, X¹ is N, and X² and X³ are each CH. In some other embodiments, X² is N, and X¹ and X³ are each CH. In some other embodiments, X³ is N, and X¹ and X² are each CH. In some other embodiments, X¹ and X² are each N, and X³ is CH. In some other embodiments, X¹ and X³ are each N, and X² is CH. In some other embodiments, X² and X³ are each N, and X¹ is CH. In some other embodiments, X¹, X², and X³ are each N.

In some embodiments, R³ and R⁷ are each independently selected from: H, C₁. ₄ alkyl, C₁₋₄ haloalkyl, C₂₋₄ alkenyl, C₂₋₄ haloalkenyl, -C₁₋₄ alkylene-O-C₁₋₄ alkyl, - C₁₋₄ alkylene-OH, -C₁₋₄ alkylene-SH, -C₁₋₄ alkylene-CN, and -C₁₋₄ alkylene-NR^{3a}R^{3b}. In some preferred embodiments, R³ and R⁷ are each independently selected from H and C₁₋₄ alkyl, more preferably H and methyl.

In some embodiments, R⁴ is selected from: H, D, halogen, OH, SH, -NR^{4a}R^{4b}, CN, C₁₋₄ alkyl, C₁₋₄ haloalkyl, C₂₋₄ alkenyl, C₂₋₄ haloalkenyl, -C₁₋₄ alkylene-O-C₁₋₄ alkyl, -C₁₋₄ alkylene-OH, -C₁₋₄ alkylene-SH, -C₁₋₄ alkylene-CN, and -C₁₋₄ alkylene-NR^{4a}R^{4b}, In some preferred embodiments, R⁴ is selected from: H, D, halogen, OH, - NR^{4a}R^{4b}, CN, C₁₋₄ alkyl, and C₁₋₄ haloalkyl. In some preferred embodiments, R⁴ is selected from: H, D, F, Cl, OH, -NH₂, CN, methyl, ethyl, -CHF₂, and -CF₃. In some more preferred embodiments, R⁴ is selected from: H, D, F, Cl, methyl, and ethyl.

In some embodiments, L² is -NR^{1b}-*, -S(O)₂NR^{1b}-*, -C(O)-NR^{1b}-*, -CR^{1c}R^{1d}-NR^{1b}-*, or -NR^{1b}-C(O)-*, wherein the bond indicated by * is connected to R^{1a}. In some preferred embodiments, L² is -S(O)₂NR^{1b}-*, wherein the bond indicated by * is connected to R^{1a}.

In some embodiments, R^{1c} and R^{1d} are each independently selected from: H, halogen, OH, SH, -NR^{2a}R^{2b}, CN, C₁₋₄ alkyl, C₁₋₄ haloalkyl, C₂₋₄ alkenyl, C₂₋₄ haloalkenyl, -O-C₁₋₄ alkyl, -O-C₁₋₄ haloalkyl, -S-C₁₋₄ alkyl, -C₁₋₄ alkylene-O-C₁₋₄ alkyl, -OC₁₋₄ alkylene-OC₁₋₄ alkyl, -C₁₋₄ alkylene-OH, -C₁₋₄ alkylene-SH, -C₁₋₄ alkylene-CN, and -C₁₋₄ alkylene-NR^{2a}R^{2b}. In some preferred embodiments, R^{1c} and R^{1d} are each independently selected from: H, F, and Cl.

In some embodiments, each R² is independently selected from: H, halogen, OH, SH, -NR^{2a}R^{2b}, CN, C₁₋₄ alkyl, C₁₋₄ haloalkyl, C₂₋₄ alkenyl, C₂₋₄ haloalkenyl, -O-C₁₋₄ alkyl, -O-C₁₋₄ haloalkyl, -S-C₁₋₄ alkyl, -C₁₋₄ alkylene-O-C₁₋₄ alkyl, -OC₁₋₄ alkylene-OC₁₋₄ alkyl, -C₁₋₄ alkylene-OH, -C₁₋₄ alkylene-SH, -C₁₋₄ alkylene-CN, and -C₁₋₄ alkylene-NR^{2a}R^{2b}. In some preferred embodiments, each R² is independently H.

In some embodiments, R⁵ is selected from: halogen, OH, SH, -NR^{5a}R^{5b}, CN, C₁₋₄ alkyl, C₁₋₄ haloalkyl, C₂₋₄ alkenyl, C₂₋₄ haloalkenyl, -O-C₁₋₄ alkyl, -O-C₁₋₄ haloalkyl, -S-C₁₋₄ alkyl, -C₁₋₄ alkylene-O-C₁₋₄ alkyl, -OC₁₋₄ alkylene-OC₁₋₄ alkyl, - C₁₋₄ alkylene-OH, -C₁₋₄ alkylene-SH, -C₁₋₄ alkylene-CN, -C₁₋₄ alkylene-NR^{5a}R^{5b}, the cyclic group Cy2, and -C₁₋₄ alkylene-Cy2, wherein the group Cy2 is selected from C₃₋₁₀ cyclohydrocarbyl, 3- to 10-membered heterocyclyl, C₆₋₁₀ aryl, and 5- to 10-membered heteroaryl, wherein the C₃₋₁₀ cyclohydrocarbyl, 3- to 10-membered heterocyclyl, C₆₋₁₀ aryl, and 5- to 10-membered heteroaryl are each optionally substituted with one or more substituents independently selected from the following groups: halogen, OH, SH, -NR^{5a}R^{5b}, CN, C₁₋₄ alkyl, C₁₋₄ haloalkyl, C₂₋₄ alkenyl, C₂₋₄ haloalkenyl, -O-C₁₋₄ alkyl, -O-C₁₋₄ haloalkyl, -S-C₁₋₄ alkyl, -C₁₋₄ alkylene-O-C₁₋₄ alkyl, -OC₁₋₄ alkylene-OC₁₋₄ alkyl, -C₁₋₄ alkylene-OH, -C₁₋₄ alkylene-SH, -C₁₋₄ alkylene-CN, and -C₁₋₄ alkylene-NR^{5a}R^{5b}

In some preferred embodiments, R⁵ is selected from: C₁₋₄ alkyl, C₁₋₄ haloalkyl, C₂₋₄ alkenyl, C₂₋₄ haloalkenyl, and the cyclic group Cy2, wherein the group Cy2 is selected from C₃₋₆ cycloalkyl, 5- to 10-membered heterocycloalkyl, C₆₋₁₀ aryl, and 5- to 10-membered heteroaryl, wherein the C₃₋₆ cycloalkyl, 5- to 10-membered heterocycloalkyl, C₆₋₁₀ aryl, and 5- to 10-membered heteroaryl are each optionally substituted with one or more substituents independently selected from the following groups: halogen, OH, SH, -NR^{5a}R^{5b}, CN, C₁₋₄ alkyl, C₁₋₄ haloalkyl, C₂₋₄ alkenyl, C₂₋₄ haloalkenyl, -O-C₁₋₄ alkyl, -O-C₁₋₄ haloalkyl, -S-C₁₋₄ alkyl, -C₁₋₄ alkylene-O-C₁₋₄ alkyl, -OC₁₋₄ alkylene-OC₁₋₄ alkyl, -C₁₋₄ alkylene-OH, -C₁₋₄ alkylene-SH, -C₁₋₄ alkylene-CN, and -C₁₋₄ alkylene-NR^{5a}R^{5b}.

In some more preferred embodiments, R⁵ is selected from: C₁₋₄ alkyl, C₁₋₄ haloalkyl, C₂₋₄ alkenyl, C₂₋₄ haloalkenyl, and the cyclic group Cy2, wherein the group Cy2 is selected from C₃₋₆ cycloalkyl, 5- to 10-membered heterocycloalkyl, C₆₋₁₀ aryl, and 5- to 10-membered heteroaryl, wherein the C₃₋₆ cycloalkyl, 5- to 10-membered heterocycloalkyl, C₆₋₁₀ aryl, and 5- to 10-membered heteroaryl are each optionally substituted with one or more substituents independently selected from the following groups: C₁₋₄ alkyl, C₁₋₄ haloalkyl, -C₁₋₄ alkylene-O-C₁₋₄ alkyl, -OC₁₋₄ alkylene-OC₁₋₄ alkyl, -C₁₋₄ alkylene-OH, -C₁₋₄ alkylene-SH, -C₁₋₄ alkylene-CN, and -C₁₋₄ alkylene-NR^{5a}R^{5b}.

In some more preferred embodiments R⁵ is selected from methyl, cyclopentyl, cyclohexyl,

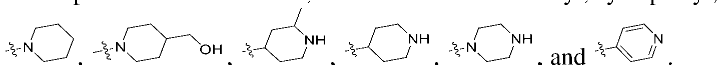

In some embodiments, R⁶ is selected from: halogen, OH, SH, -NR^{6a}R^{6b}, CN, C₁₋₄ alkyl, C₁₋₄ haloalkyl, C₂₋₄ alkenyl, C₂₋₄ haloalkenyl, -O-C₁₋₄ alkyl, -O-C₁₋₄ haloalkyl, -S-C₁₋₄ alkyl, -C₁₋₄ alkylene-O-C₁₋₄ alkyl, -OC₁₋₄ alkylene-OC₁₋₄ alkyl, - C₁₋₄ alkylene-OH, -C₁₋₄ alkylene-SH, -C₁₋₄ alkylene-CN, and -C₁₋₄ alkylene-NR^{6a}R^{6b}.

In some preferred embodiments, R⁶ is selected from: halogen, OH, SH, - NR^{6a}R^{6b}, CN, C₁₋₄ alkyl, C₁₋₄ haloalkyl, -C₁₋₄ alkylene-OH, -C₁₋₄ alkylene-SH, -C₁₋₄ alkylene-CN, and -C₁₋₄ alkylene-NR^{6a}R^{6b}.

In some more preferred embodiments, R⁶ is selected from: F, Cl, OH, -NH₂, - NHCH₃, -N(CH₃)₂, CN, methyl, ethyl, -CHF₂, and -CF₃.

In some more preferred embodiments, R⁶ is selected from: -NH₂, -NHCH₃, - N(CH₃)₂, methyl, ethyl, -CHF₂, and -CF₃.

In some embodiments, n is 0 or 1.

In some embodiments , R^{1b}, R^{1e}, R^{1f}, R^{2a} , R^{2b} , R^{3a} , R^{3b}, R^{4a}, R^{4b}, R^{5a}, R^{5b}, R^{6a}, and R^{6b} are, at each occurrence, independently selected from H and C₁₋₄ alkyl, preferably H, methyl, and ethyl.

The present invention covers compounds obtained by any combination of various embodiments.

In some embodiments, the present invention provides a compound of formula (I), or a stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof, wherein the compound is selected from:

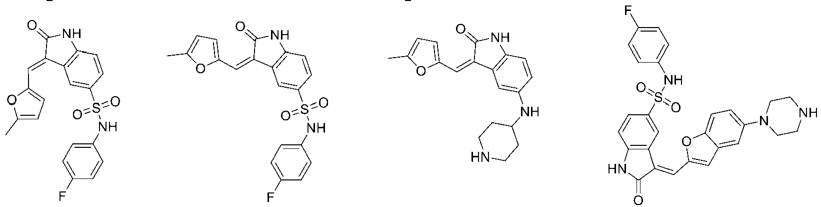

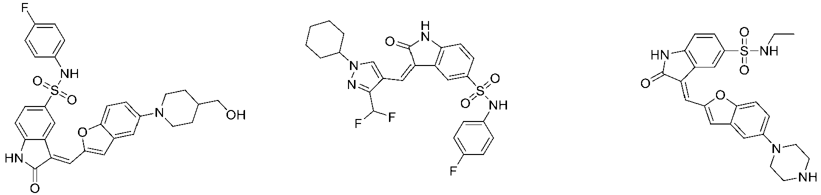

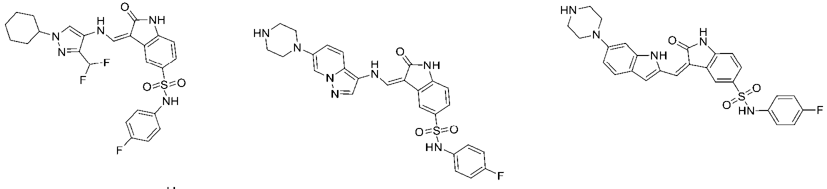

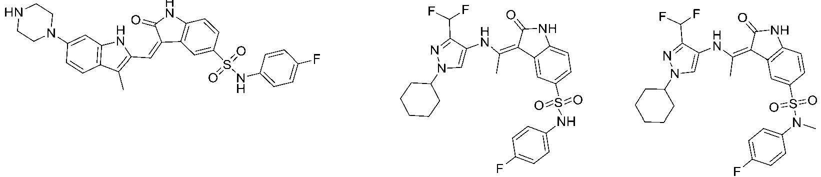

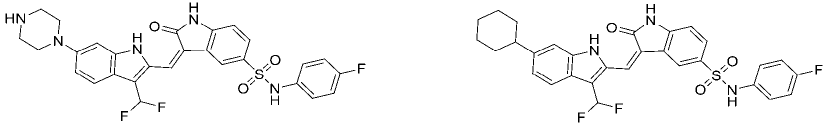

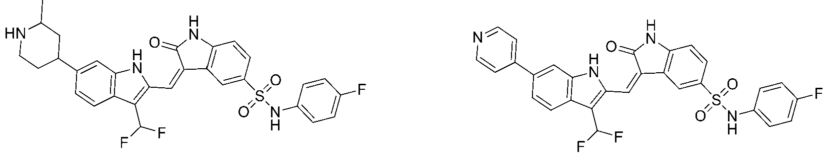

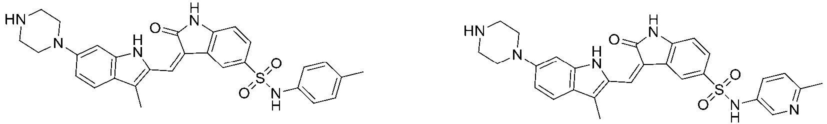

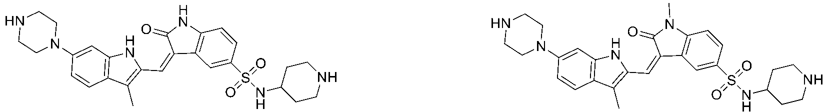

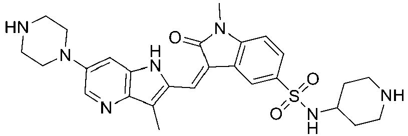

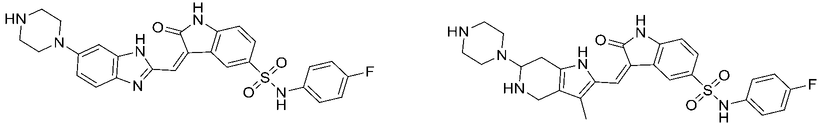

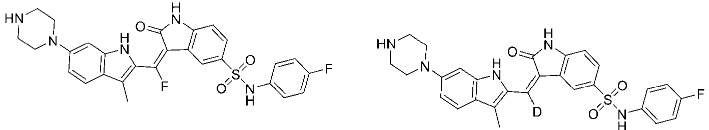

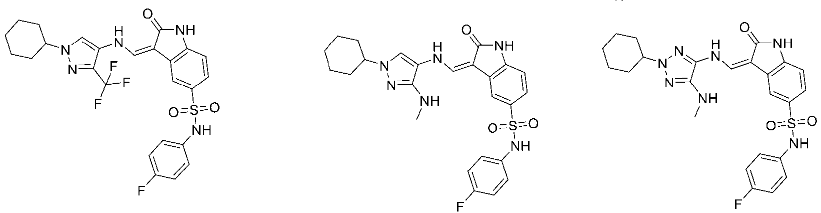

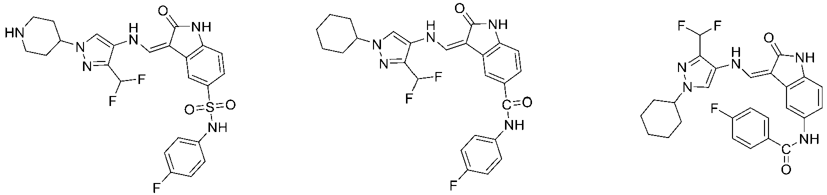

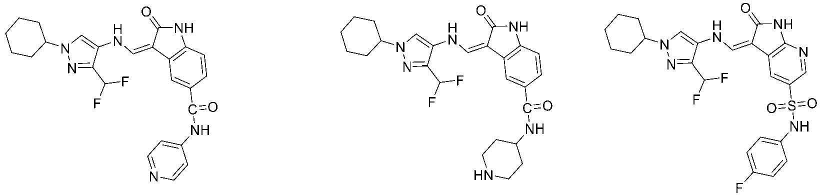

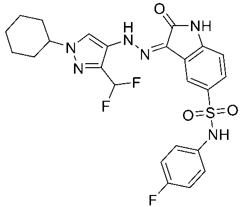

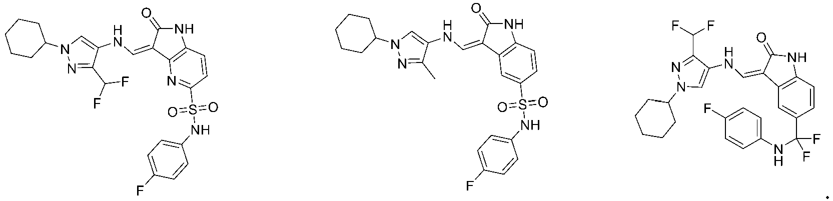

### PROTAC molecule capable of inhibiting and degrading IRAK4

In another aspect, the present invention provides the use of the compound or the stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof according to the present invention, as described above, in the preparation of a proteolysis targeting chimera (PROTAC).

In another aspect, the present invention provides a proteolysis targeting chimera (PROTAC), comprising a moiety with IRAK4 protein kinase inhibitory activity, wherein the moiety is derived from the compound or the stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof according to the present invention, as described above.

### Pharmaceutical composition and treatment method

In another aspect, the present invention provides a pharmaceutical composition, comprising a therapeutically effective amount of the compound or the stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof according to the present invention, or the PROTAC molecule of the present invention, and one or more pharmaceutically acceptable carriers. The pharmaceutical composition is preferably a solid formulation, a liquid formulation, or a transdermal formulation.

In another aspect, the present invention provides the use of the compound or the stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof according to the present invention, or the PROTAC molecule of the present invention, or the pharmaceutical composition of the present invention for the preparation of a medicament.

In some embodiments, the compound of the present invention, the PROTAC molecule of the present invention, the pharmaceutical composition of the present invention, or the medicament is used for the treatment of a disease, disorder or condition associated with IRAK4 protein kinase.

In another aspect, the present invention further provides a method for treating a disease, disorder or condition associated with IRAK4 protein kinase, alleviating a symptom thereof, and delaying the development or onset thereof, which comprises administering to an individual in need thereof an effective amount of the compound or the stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof according to the present invention, the PROTAC molecule of the present invention, or the pharmaceutical composition of the present invention.

In a further aspect, the present invention provides the compound or the stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof according to the present invention, the PROTAC molecule of the present invention, or the pharmaceutical composition of the present invention, which is used for treating a disease, disorder or condition associated with IRAK4 protein kinase.

In another aspect, the present invention further provides the use of the compound or the stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof according to the present invention, the PROTAC molecule of the present invention, or the pharmaceutical composition of the present invention for the preparation of a medicament as an IRAK4 inhibitor.

In a further aspect, the present invention provides a method for inhibiting IRAK4 activity in an individual, which comprises administering to an individual in need thereof an effective amount of the compound or the stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof according to the present invention, the PROTAC molecule of the present invention, or the pharmaceutical composition of the present invention.

In some embodiments, the disease, disorder or condition associated with IRAK4 protein kinase is selected from: autoimmune condition, inflammatory condition, cancer, graft rejection, thromboembolism, atherosclerosis, myocardial infarction, and metabolic syndrome.

In some embodiments, the inflammatory condition is selected from: osteoarthritis, gout, gouty arthritis, chronic obstructive pulmonary disease, periodic fever, atopic dermatitis, allergic eczema, lymphadenectasis, sepsis, irritable bowel syndrome (IBD), ulcerative colitis, asthma, and allergy.

In some embodiments, the autoimmune condition is selected from: Crohn's disease, rheumatoid arthritis, systemic lupus erythematosus, lupus nephritis, cutaneous lupus, psoriasis, psoriatic arthritis, multiple sclerosis, neuropathic pain, ankylosing spondylitis, reactive arthritis, and systemic juvenile idiopathic arthritis.

In some embodiments, the graft rejection is selected from graft-versus-host disease and allograft rejection.

In some embodiments, the cancer is selected from: brain cancer, renal cancer, liver cancer, gastric cancer, vaginal cancer, ovarian cancer, gastric tumor, breast cancer, bladder cancer, colon cancer, prostate cancer, pancreatic cancer, lung cancer, cervical cancer, testicular cancer, skin cancer, bone cancer, thyroid cancer, sarcoma, glioblastoma, neuroblastoma, multiple myeloma, gastrointestinal cancer, neck and head tumor, adenoma, adenocarcinoma, keratoacanthoma, epidermoid carcinoma, large cell carcinoma, non-small cell lung cancer, Hodgkin's lymphoma and non-Hodgkin's lymphoma, mastocarcinoma, follicular carcinoma, papillary carcinoma, seminoma, melanoma, acute myeloid leukaemia, chronic myeloid leukemia, diffuse large B-cell lymphoma, activated B cell-like diffuse large B-cell lymphoma, chronic lymphocytic leukemia, chronic lymphocytic lymphoma, primary exudative lymphoma, Burkitt's lymphoma/leukemia, acute lymphoblastic leukemia, B-cell prolymphocytic leukemia, lymphoplasmacytic lymphoma, Waldenstrom's macroglobulinemia, splenic marginal zone lymphoma, intravascular large B-cell lymphoma, plasmacytoma, and multiple myeloma.

In the present invention, "pharmaceutically acceptable carrier" refers to a diluent, adjuvant, excipient, or vehicle administered together with a therapeutic agent, and it is suitable for use in contact with tissues of human beings and/or other animals within the scope of sound medical judgment without undue toxicity, irritation, allergic reaction or other problems or complications commensurate with a reasonable benefit/risk ratio.

Unless otherwise specified, as used herein, the term "treatment" means reversing, alleviating, or inhibiting a disorder or condition to which such a term applies or the progression of one or more symptoms of such a disorder or condition, or preventing such a disorder or condition or one or more symptoms of such a disorder or condition.

As used herein, "individual" includes human or non-human animals. Exemplary human individuals include human individuals (referred to as patients) suffering from diseases (e.g., diseases described herein) or normal individuals. "Non-human animals" in the present invention includes all vertebrates, such as non-mammals (e.g., birds, amphibians, and reptiles) and mammals, such as non-human primates, domestic animals and/or domesticated animals (e.g., sheep, dogs, cats, cows, and pigs).

In another embodiment, the pharmaceutical composition of the present invention can further comprise one or more additional therapeutic or prophylactic agents.

### Examples

Embodiments of the present invention will be described in detail below with reference to examples; however, those skilled in the art will understand that the following examples are only used to illustrate the present invention and should not be regarded as limiting the scope of the present invention. For examples in which specific conditions are not specified, they are carried out according to the conventional conditions or the conditions recommended by the manufacturer. The reagents or instruments used without indicating the manufacturer are all commercially available conventional products.

NMR was measured by Bruker Avance III 400 nuclear magnetic analyzer, and the chemical shift (δ) was given in 10⁻⁶ (ppm). The solvent was deuterated methanol (CD₃OD), deuterated chloroform (CDCl₃), hexadeuterated dimethyl sulfoxide (DMSO-d6), etc., and the internal standard is tetramethylsilane (TMS).

MS was measured by an Agilent (ESI) mass spectrometer (Agilent 1260, Agilent 6125B).

Determination conditions of high performance liquid chromatography (HPLC): Gilson high-pressure liquid chromatograph (Gilson GX-281), C18 column (10 µM, 19 mm × 250 mm), ultraviolet detection wave bands: 220 and 254 nm, and elution conditions: gradient elution with 5-95% acetonitrile (containing 0.05% v/v formic acid or ammonium bicarbonate) for 15 min.

For reverse phase purification, a Biotage Isolera rapid purification system is used.

Thin-layer chromatography separation and purification is carried out by using a thin-layer chromatography silica gel plate (aluminum plate (20 cm × 20 cm × 1 mm) produced by Meck or GF 254 produced in Yantai).

Biotage Initiator + (400 W, RT to 300°C) microwave reactor is used for a microwave reaction.

TLC or LCMS is commonly used for reaction monitoring. Commonly used developing agent systems are: dichloromethane/methanol, n-hexane/ethyl acetate, and petroleum ether/ethyl acetate. The volume ratio of the solvents is adjusted according to the polarity of the compound or by adding triethylamine, etc.

The silica gel used in column chromatography is generally 100-200 mesh silica gel. Commonly used eluent systems are: dichloromethane/methanol and petroleum ether/ethyl acetate. The volume ratio of the solvents is adjusted according to the polarity of the compound, or may also be adjusted by adding a small amount of triethylamine.

The reagents, solvents, etc. of the present invention are purchased from Aldrich Chemical Company, Energy Chemical, J&K Scientific Ltd., Shanghai Bide Pharmatech Ltd., PharmaBlock, Shanghai Titan Scientific Co., Ltd., etc.

In conventional synthesis methods and synthesis examples of the compounds and intermediates of the present invention, the meanings of abbreviations are as shown below.

| Abbreviation | Meaning |
|---|---|
| EtOH | Ethanol |
| LAH or LiAlH₄ | Lithium aluminum hydride |
| DMF-DMA | Dimethylformamide dimethyl acetal |
| THF | Tetrahydrofuran |
| DCM | Dichloromethane |
| TFA | Trifluoroacetic acid |
| Pd₂(dba)₃ | Tris(dibenzylideneacetone)dipalladium |
| MW | Microwave |
| MsOH | Methanesulfonic acid |
| X-Phos | 2-Dicyclohexylphosphino-2,4,6-triisopropylbiphenyl |
| LiHMDS | Lithium bis(trimethylsilyl)amide |
| tBuONa | Sodium tert-butoxide |
| LC-MS | Liquid chromatography-mass spectrometry instrument |
| NMR | Nuclear magnetic resonance spectroscopy |
| HPLC | High performance liquid chromatography |

### Synthesis Examples

### Example 1: Preparation of compound 1

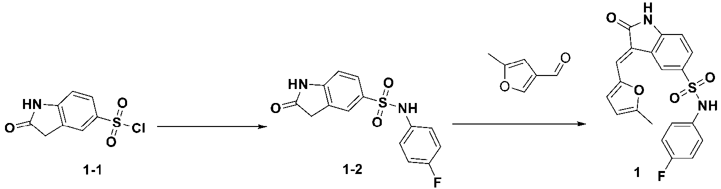

Step 1: At room temperature, compound 1-1 (1.00 g, 4.32 mmol), pyridine (0.68 g, 8.64 mmol), p-fluoroaniline (0.96 g, 8.64 mmol), and tetrahydrofuran (10 mL) were added to a single-necked flask, and the reaction solution was stirred at 25°C for 2 hours. Water (50 mL) was added to the reaction solution, and the reaction solution was extracted with ethyl acetate (40 mL × 3). The organic phase was dried over anhydrous sodium sulfate and concentrated under reduced pressure, and the crude product was slurried with petroleum ether/ethyl acetate (5/1) to obtain compound 1-2. LC-MS: m/z: 307.0 (M+H)⁺.

Step 2: At room temperature, compound 1-2 (200 mg, 0.65 mmol), piperidine (166 mg, 1.95 mmol), 5-methylfuran-3-carbaldehyde (143 mg, 1.30 mmol), and ethanol (8 mL) were added to a 100 mL single-necked flask, and the reaction solution was stirred for 4 hours at room temperature. The reaction solution was concentrated under reduced pressure, and the residue was slurried with petroleum ether/ethyl acetate (5/1) to obtain compound 1.

LC-MS: m/z: 399 (M+H)⁺.

¹H NMR (400 MHz, DMSO-*d₆*) δ 10.98 (s, 1H), 9.99 (s, 1H), 8.78 (d, *J* = 1.6 Hz, 1H),7.56 (s, 1H), 7.35 (s, 1H), 7.28 (d, *J =* 3.2 Hz, 2H), 7.08 (t, *J =* 5.6 Hz, 2H), 7.00 (d, *J =* 8.4 Hz, 1H), 6.53 (d, *J =* 3.2 Hz, 1H), 2.54 (s, 3H).

### Example 2: Preparation of compound 2

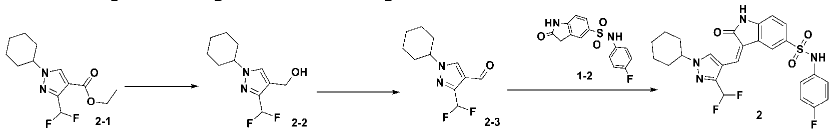

Step 1: Compound 2-1 (2.00 g, 7.34 mol) was added to a three-necked flak and dissolved in tetrahydrofuran (150 mL). At 0°C, lithium aluminum hydride (22.0 ml, 22.0 mmol, 1.0 M THF solution) was added to the reaction. The reaction solution was stirred at room temperature for 1 hour, and sodium sulfate decahydrate (5.0 g) was then slowly added. The mixture was stirred and filtered. The filter cake was washed with methanol (30 mL) three times. The filtrates were combined and concentrated under reduced pressure to obtain compound 2-2. LC-MS: m/z: 231 (M+H)⁺.

Step 2: Compound 2-2 (1.77 g, 7.30 mol) was added to a three-necked flask and dissolved in dichloromethane (150 mL). At room temperature, Dess-Martin periodinane (7.19 ml, 23.07 mmol) was added to the reaction. The reaction solution was stirred at room temperature for 3 hours, quenched with a saturated sodium bicarbonate solution (100 mL), and extracted with dichloromethane (100 mL × 3). The organic layers were combined, washed with a saturated aqueous sodium chloride solution (200 mL), dried over anhydrous sodium sulfate, filtered, and concentrated under reduced pressure, and the residue was separated and purified by column chromatography (ethyl acetate/petroleum ether = 1/3) to obtain compound 2-3. LC-MS: m/z: 229.0 (M+H)⁺.

¹H NMR (400 MHz, DMSO-*d₆*) δ 9.89 (s, 1H), 8.66 (s, 1H), 7.20 (t, *J =* 53.56 Hz, 1H), 4.35 - 4.21 (m, 1H), 2.09 - 2.01 (m, 2H), 1.86 - 1.77 (m, 2H), 1.77 -1.61 (m, 3H), 1.47 - 1.32 (m, 2H), 1.28 - 1.15 (m, 1H).

Step 3: At room temperature, compound 2-3 (200 mg, 0.88 mmol), compound 1-2 (268 mg, 0.88 mmol), piperdine (374 mg, 4.40 mmol), and ethanol (6 mL) were added to a single-necked flask, and the reaction solution was stirred at room temperature for 4 hours. The reaction solution was concentrated under reduced pressure, and the crude product was slurried with petroleum ether/ethyl acetate (5/1) to obtain compound 2. LC-MS: m/z: 517.2 (M+H)⁺.

¹H NMR (400 MHz, DMSO-*d₆*) δ 11.14 (s, 1H), 10.11 (s, 1H), 9.38 (s, 1H), 7.97 (s, 1H), 7.72 (s, 1H), 7.55-7.51 (m, 2H), 7.11-7.08 (m, 4H), 6.95 (d, *J =* 8.4 Hz, 1H), 4.36 (t, *J =* 3.6 Hz, 1H), 2.08 (d, *J =* 10.4 Hz, 2H), 1.83 (d, *J =* 13.2 Hz, 2H), 1.78-1.66 (m, 3H), 1.45-1.42 (m, 2H), 1.26-1.23 (m, 1H).

### Example 3: Preparation of compound 3

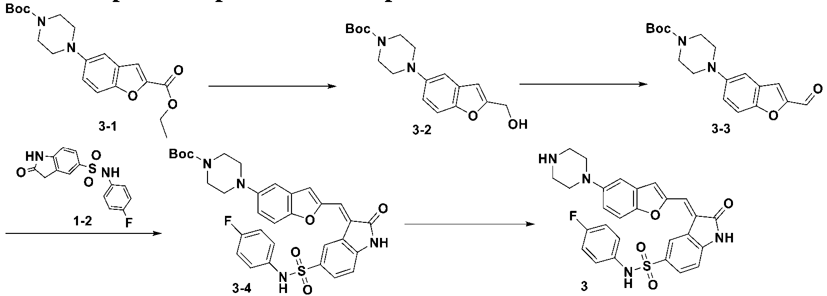

Step 1: Compound 3-1 (500 mg, 1.34 mmol) and anhydrous tetrahydrofuran (10 mL) were added to a single-necked flask and cooled to 0°C, and a lithium aluminum hydride solution (0.81 mL, 1.61 mmol, 2.0 M in THF) was dropwise added. The reaction solution was stirred at 0°C for 1 hour. Sodium sulfate decahydrate (5.0 g) was added to the reaction solution, and the mixture was filtered. The filtrate was concentrated under reduced pressure, and the crude product was purified by column chromatography (0-50%, petroleum ether/ethyl acetate) to obtain compound 3-2. LC-MS: m/z: 333.2 (M+H)⁺.

Step 2: Compound 3-2 (170 mg, 0.51 mmol), Dess-Martin periodinane (650 mg, 1.53 mmol), and dichloromethane (6 mL) were added to a 100 mL single-necked flask at room temperature, and the reaction solution was stirred at room temperature for 16 hours. The reaction solution was filtered by a Buchner funnel. The filtrate was concentrated, and the crude product was purified by column chromatography (0-50%, petroleum ether/ethyl acetate) to obtain compound 3-3. LC-MS: m/z: 331.0 (M+H)⁺.

Step 3: At room temperature, compound 3-3 (100 mg, 0.30 mmol), compound 1-2 (92.7 mg, 0.30 mmol), piperdine (129 mg, 1.51 mmol), and ethanol (6 mL) were added to a single-necked flask, and the reaction solution was stirred at 25°C for 18 hours. The reaction solution was concentrated, diluted with water (20 mL), extracted with ethyl acetate (20 mL×2), dried over anhydrous sodium sulfate, and concentrated under reduced pressure, and the crude product was purified by column chromatography (0-50%, petroleum ether/ethyl acetate) to obtain compound 3-4. LC-MS: m/z: 619.0 (M+H)⁺.

Step 4: At room temperature, compound 3-4 (100 mg, 0.16 mmol), trifluoroacetic acid (2 mL), and dichloromethane (6 mL) were added to a single-necked flask, and the reaction solution was stirred at 25°C for 1 hour. The reaction solution was concentrated under reduced pressure, and the crude product was subjected to high performance liquid chromatography (formic acid/acetonitrile/water system) to prepare compound 3. LC-MS: m/z: 519.2 (M+H)⁺.

¹H NMR (400 MHz, DMSO-*d₆*) δ 9.00 (s, 1H), 8.28 (s, 1H), 7.64 (d, *J* = 6.8 Hz, 2H), 7.54 (d, *J* = 8.8 Hz, 2H), 7.38 (d, *J* = 9.2 Hz, 1H), 7.23 (s, 1H), 7.15 (d, *J* = 4.8 Hz, 2H), 7.13-6.98 (m, 3H), 3.20 (s, 4H), 3.04 (s, 4H).

### Example 4: Preparation of compound 4

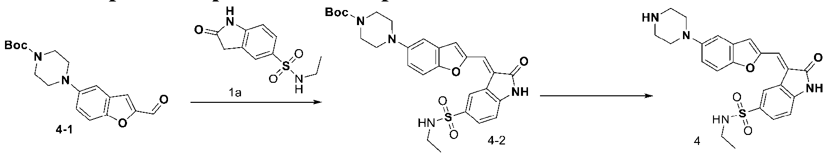

Step 1: At room temperature, compound 4-1 (100 mg, 0.30 mmol), compound 1a (72 mg, 0.30 mmol), piperdine (77 mg, 0.90 mmol), and ethanol (6 mL) were added to a single-necked flask, and the reaction solution was stirred at 25°C for 18 hours. The reaction solution was concentrated, and water (20 mL) was added to the reaction solution. The reaction solution was extracted with ethyl acetate (20 mL ×2). The organic phase was dried over anhydrous sodium sulfate and concentrated under reduced pressure, and the residue was separated by column chromatography (0-50%, petroleum ether/ethyl acetate) to obtain compound 4-2. LC-MS: m/z: 554.2 (M+H)⁺.

Step 2: At room temperature, compound 4-2 (150 mg, 0.27 mmol), trifluoroacetic acid (1 mL), and dichloromethane (3 mL) were added to a 100 mL single-necked flask, and the reaction solution was stirred at 25°C for 2 hours. The reaction solution was concentrated under reduced pressure, and the residue was subjected to high performance liquid chromatography (formic acid/acetonitrile/water system) to prepare compound 4. LC-MS: m/z: 453.0 (M+H)⁺.

¹H NMR (400 MHz, DMSO-*d6*) δ 11.06 (brs, 1H), 9.11 (s, 1H), 7.73 (d, *J =* 6.8 Hz, 1H), 7.71 (s, 1H), 7.64 (s, 1H), 7.55 (d, *J =* 16 Hz, 1H), 7.49 (d, *J =* 16 Hz, 1H), 7.33 (d, *J =* 9.2 Hz, 1H), 7.20(d, *J =* 1.6 Hz, 1H), 7.07 (d, *J =* 8.4 Hz, 1H), 3.11 (s, 4H), 2.94 (s, 4H), 2.90 - 2.80 (m, 2H), 1.01 (t*, J =* 7.2 Hz, 3H).

### Example 5: Preparation of compound 5

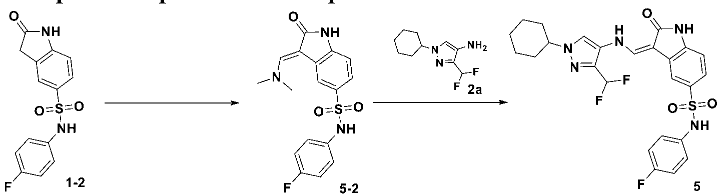

Step 1: At 25°C, compound 1-2 (100 mg, 0.33 mmol) and dimethylformamide dimethyl acetal (2 mL) were added to a 50 mL single-necked flask, and the reaction solution was stirred at 25°C for 2 hours. The reaction solution was evaporated to dryness under reduced pressure to obtain compound 5-2, and the residue was directly used in the next reaction without further purification. LC-MS: m/z: 362.0 (M+H)⁺.

Step 2: At 25°C, compound 5-2 (100 mg, 0.280 mmol), compound 2a (59.5 mg, 0.28 mmol), and ethanol (1 mL) were added to a single-necked flask, and the reaction solution was stirred at 80°C for 18 hours. The reaction solution was evaporated to dryness under reduced pressure, and the residue was purified by preparative chromatography (acetonitrile/water (0.1% formic acid)) to obtain compound 5. LC-MS: m/z 532.1 (M+H)⁺.

¹H NMR (400 MHz, DMSO-*d₆*) δ 10.88 (s, 1H), 10.60 (d, *J* = 12.9 Hz, 1H), 9.99 (s, 1H), 8.45 (d, *J =* 12.5 Hz, 1H), 8.29 (s, 1H), 7.83 (d, *J =* 1.7 Hz, 1H), 7.35 (dd, *J =* 8.3*,* 1.9 Hz, 1H), 7.05 (s, 2H), 7.03 (d, *J =* 1.3 Hz, 2H), 6.93 (d, *J =* 8.2 Hz, 1H), 4.12 (t, *J =* 11.7 Hz, 1H), 2.04 (d, *J* = 12.9 Hz, 2H), 1.96 (d, *J =* 7.4 Hz, 1H), 1.80 (d, *J =* 13.0 Hz, 2H), 1.65 (d, *J =* 12.5 Hz, 3H), 1.42 - 1.30 (m, 2H).

### Example 6: Preparation of compound 6

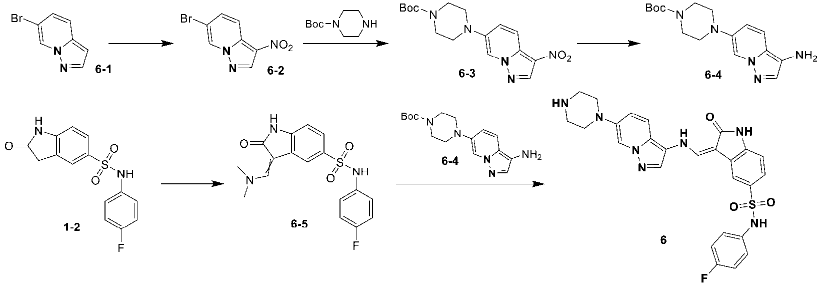

Step 1: At room temperature, compound 6-1 (2.00 g, 10.15 mmol) and concentrated H₂SO₄ (10 mL) were added to a three-necked flask and cooled to 0°C in an ice-water bath, and concentrated HNO₃ (0.71 mL, 11.2 mmol) was then slowly dropwise added to the reaction flask. The reaction solution was stirred at 0-5°C for 3 hours. As monitored by LCMS, the raw material was completely reacted. The reaction solution was slowly poured into ice water (30 mL), and a solid was precipitated and filtered to obtain compound 6-2. LC-MS: m/z: 244.1 (M+H)⁺.

Step 2: At room temperature, compound 6-2 (700 mg, 2.89 mmol), 1-Boc-piperazine (700 mg, 3.76 mmol), Pd₂(dba)₃ (234 mg, 0.29 mmol), BINAP (540 mg, 0.87 mmol), cesium carbonate (1413 mg, 4.34 mmol), and DMA (7 mL) were added to an IKA flask. After complete nitrogen displacement, the reaction solution was stirred at 110°C for 12 hours. As monitored by LCMS, the raw material was completely reacted. Water (10 mL) was added to the reaction solution, and the reaction solution was extracted with ethyl acetate (30 mL × 3). The organic phase was concentrated under reduced pressure, and the residue was purified by column chromatography (PE/EA = 1/0-1/1) to obtain compound 6-3. LC-MS: m/z: 348.4 (M+H)⁺.

¹H NMR (400 MHz, DMSO-*d*₆) δ 8.79 (s, 1H), 8.46 (d, *J*=1.6 Hz, 1H), 8.11 (d, *J*=9.6 Hz, 1H), 7.92 - 7.84 (m, 1H), 3.52 - 3.44 (m, 4H), 3.22 - 3.16 (m, 4H), 1.48 - 1.38 (m, 9H).

Step 3: At room temperature, compound 6-3 (400 mg, 1.15 mmol), ethanol (4 mL), and water (1 mL) were added to an IKA flask, and iron powder (257 mg, 4.61 mmol) and anhydrous calcium chloride (63.9 mg, 0.58 mmol) were then added. After nitrogen displacement, the reaction solution was stirred at 80°C for 5 hours. As monitored by LCMS, the raw material was completely reacted. The reaction solution was cooled to room temperature, filtered, and concentrated under reduced pressure to obtain compound 6-4, which was immediately used in the next step. LC-MS: m/z: 318.4 (M+H)⁺.

Step 4: At room temperature, compound 1-2 (100 mg, 0.33 mmol) and DMF (1 mL) were added to an IKA flask, followed by DMF-DMA (0.09 mL, 0.66 mmol), and after nitrogen displacement, the reaction solution was stirred at 90°C for 1 hour. As monitored by TLC (PE/EA = 1/1), the raw material was completely reacted. The reaction solution was cooled to room temperature, and concentrated under reduced pressure to obtain compound 6-5, which was immediately used in the next step.

Step 5: At room temperature, compound 6-5 (52.69 mg, 0.17 mmol), ethanol (2 mL), compound 6-4 (50 mg, 0.14 mmol), and methanesulfonic acid (0.01 mL, 0.15 mmol) were added to a 5 mL microwave flask, and after nitrogen displacement, the reaction solution was heated under microwave at 150°C for 5 minutes. As monitored by LCMS, the raw material was completely reacted. The reaction solution was cooled to room temperature and concentrated under reduced pressure. After preparative purification, compound 6 was obtained.

LC-MS: m/z: 534.4 (M+H)⁺.

¹H NMR (400 MHz, DMSO-*d*₆) δ 10.86 (s, 1H), 10.81 - 10.52 (m, 1H), 8.54 (brs, 1H), 8.26 (brs, 1H), 8.15 (s, 1H), 7.98 (d, *J*=10.8 Hz, 2H), 7.62 (d, *J*=9.8 Hz, 1H), 7.40 - 7.25 (m, 2H), 7.16 - 7.02 (m, 5H), 6.94 (d, *J*=8.1 Hz, 1H), 3.04 (br s, 4H), 2.91 (br s, 4H).

### Example 7: Preparation of compound 7

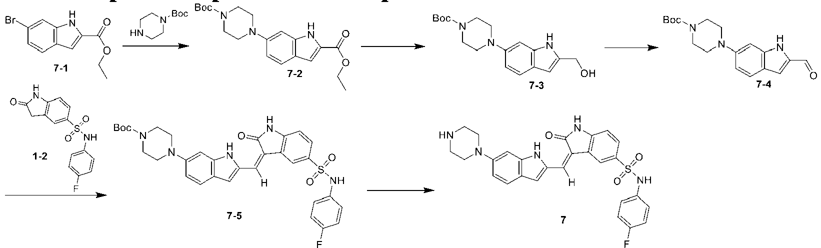

Step 1: At room temperature, ethyl 6-bromoindole -2-carboxylate (800 mg, 2.98 mmol), 1-Boc-piperazine (945 mg, 5.07 mmol), 2-dicyclohexylphosphino-2,4,6-triisopropyl biphenyl (42.7 mg, 0.090 mmol), tris(dibenzylideneacetone)dipalladium (27.3 mg, 0.030 mmol), and tetrahydrofuran (20 mL) were successively added to an IKA flask. After nitrogen displacement three times, a 1.0 M tetrahydrofuran solution (7.46 mL) of lithium bis(trimethylsilyl)amide was added, and the reaction solution was then stirred at 65°C for 12 hours. After the reaction was complete as detected by LCMS, 100 mL of water was added to the reaction solution, and the reaction solution was then extracted with 50 mL of ethyl acetate three times. The organic phase was concentrated under reduced pressure, and the residue was subjected to column chromatography (0-50% petroleum ether and ethyl acetate) to obtain compound 7-2. LC-MS: m/z: 374.2 (M+H)⁺.

¹H NMR (400 MHz, DMSO-*d*₆) δ 11.55 (s, 1H), 7.50 (d, *J =* 8.9 Hz, 1H), 7.03 (d, *J* = 1.6 Hz, 1H), 6.91 (dd, *J =* 2.1, 8.9 Hz, 1H), 6.81 (s, 1H), 4.30 (q, *J =* 7.1 Hz, 2H), 3.54 - 3.44 (m, 4H), 3.15 - 3.05 (m, 4H), 1.43 (s, 9H), 1.32 (t, *J =* 7.1 Hz, 3H).

Step 2: At room temperature, compound 7-2 (300 mg, 0.80 mmol) and tetrahydrofuran (10 mL) were successively added to a reaction flask and stirred until dissolved. The reaction solution was cooled to 0°C, lithium aluminum hydride (67.07 mg, 1.77 mmol) was added, and the reaction solution was stirred at 25°C for 1 hour. After the reaction was complete as detected by TLC, 10 mL of water was added to the reaction solution, followed by an aqueous sodium hydroxide solution (1.0 M, 10 mL), and the reaction solution was then extracted with ethyl acetate (10 mL × 3). The organic phase was concentrated under reduced pressure, and the residue was subjected to column chromatography (0-50% petroleum ether and ethyl acetate) to obtain compound 7-3.

¹H NMR (500 MHz, DMSO-*d*₆) δ 10.73 (s, 1H), 7.31 (d, *J =* 8.5 Hz, 1H), 6.81 (s, 1H), 6.74 (dd, *J =* 2.1, 8.5 Hz, 1H), 6.13 (s, 1H), 5.14 (t, *J =* 5.6 Hz, 1H), 4.53 (d, *J =* 5.5 Hz, 2H), 3.52 - 3.46 (m, 4H), 3.04 - 2.96 (m, 4H), 1.43 (s, 9H).

Step 3: At room temperature, compound 7-3 (190 mg, 0.57 mmol) and dichloromethane (5 mL) were successively added to a flask and stirred until dissolved. Manganese dioxide (249 mg, 2.87 mmol) was added, and the reaction solution was stirred at 25°C for 2 hours. After the reaction was complete as detected by LCMS, the reaction solution was filtered. The organic phase was concentrated under reduced pressure, and the residue was subjected to column chromatography (0-50% petroleum ether and ethyl acetate) to obtain compound 7-4.

¹H NMR (400 MHz, DMSO-*d*₆) δ 11.65 (s, 1H), 9.67 (s, 1H), 7.58 (d, *J* = 9.1 Hz, 1H), 7.26 (d, *J =* 1.3 Hz, 1H), 6.95 (dd, *J =* 2.0, 8.9 Hz, 1H), 6.75 (s, 1H), 3.59 - 3.44 (m, 4H), 3.19 - 3.06 (m, 4H), 1.43 (s, 9H).

Step 4: At room temperature, compound 7-4 (50 mg, 0.15 mmol), piperidine (51.14 mg, 0.17 mmol), and ethanol (1 mL) were successively added to an 8 mL flask and stirred until dissolved. compound 1-2 (51.1 mg, 0.17 mmol) was added, and the reaction solution was stirred at 70°C for 3 hours. After the reaction was complete as detected by LCMS, the reaction solution was filtered to obtain compound 7-5. LC-MS: m/z: 618.2 (M+H)⁺.

Step 5: At room temperature, compound 7-5 (100 mg, 0.13 mmol) and hydrogen chloride dioxane (2 mL) were successively added to a 40 mL IKA flask. The reaction solution was stirred at 25°C for 1 hour. The reaction solution was filtered and concentrated under reduced pressure, and the residue was purified by high performance liquid chromatography (water/aqueous ammonia/acetonitrile) to obtain compound 7. LC-MS: m/z: 518.1 (M+H)⁺.

¹H NMR (400 MHz, DMSO-*d*₆) δ 12.61 (s, 1H), 8.06 (d, *J =* 1.6 Hz, 1H), 7.97 (s, 1H), 7.57 - 7.46 (m, 2H), 7.16 (s, 1H), 7.14 - 7.03 (m, 5H), 7.00 (d, *J =* 8.2 Hz, 1H), 6.96 - 6.90 (m, 2H), 3.19 - 3.15 (m, 4H), 2.90 - 2.82 (m, 4H).

### Example 8: Preparation of compound 8

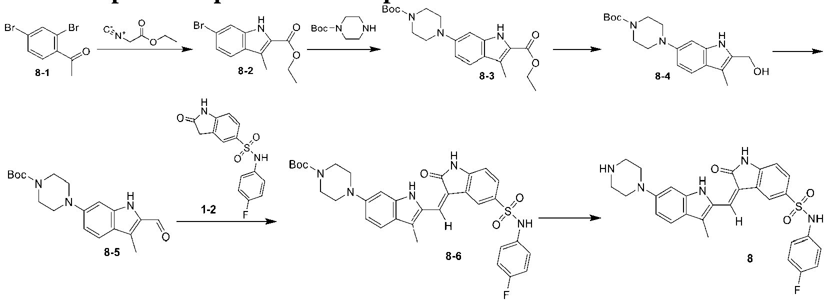

Step 1: At room temperature, compound 8-1 (2.00 g, 7.20 mmol) and dimethylsulfoxide (15 mL) were successively added to a 50 mL single-necked flask and stirred until dissolved. Ethyl isocyanatoacetate (895 mg, 7.92 mmol), cuprous iodide (137 mg, 0.72 mmol), and cesium carbonate (4.69 g, 14.4 mmol) were successively added. The reaction solution was stirred under nitrogen protection at 50°C for 16 hours. 150 mL of water was added to the reaction solution, and the reaction solution was extracted with ethyl acetate (30 mL × 3). The organic phases were combined, dried over anhydrous sodium sulfate, and filtered, the filtrate was concentrated under reduced pressure, and the crude product was purified by column chromatography (petroleum ether/ethyl acetate = 10/1) to obtain compound 8-2. LC-MS: m/z: 284.0 (M+H)⁺.

¹H NMR (400 MHz, DMSO-*d*₆) δ 11.59 (br s, 1H), 7.62 (d, *J =* 8.6 Hz, 1H), 7.56 (d, *J* = 1.6 Hz, 1H), 7.19 (dd, *J* = 1.7, 8.6 Hz, 1H), 4.35 (q, *J* = 7.1 Hz, 2H), 2.52 (s, 3H), 1.35 (t, *J =* 7.1 Hz, 3H).

Step 2: At room temperature, compound 8-2 (1.20 g, 4.25 mmol) and tetrahydrofuran (18 mL) were successively added to a 50 mL single-necked flask and stirred until dissolved. 1-Boc-piperazine (1.19 g, 6.38 mmol), Pd₂(dba)₃ (194.7 mg, 0.21 mmol), X-phos (202.76 mg, 0.43 mmol), and sodium tert-butoxide (834.1 mg, 9.36 mmol) were successively added. The reaction solution was stirred under nitrogen protection at 65°C for 16 hours. Water (80 mL) was added to the reaction solution, and the reaction solution was extracted with ethyl acetate (30 mL × 3). The organic phases were combined, dried over anhydrous sodium sulfate, and filtered, the filtrate was concentrated under reduced pressure and the crude product was purified by column chromatography (petroleum ether/ethyl acetate = 3/1) to obtain compound 8-3. LC-MS: m/z: 388.1 (M+H)⁺.

¹H NMR (400 MHz, CDCl₃) δ 8.54 (br s, 1H), 7.53 (d, *J* = 8.8 Hz, 1H), 6.89 (dd, *J* = 2.0, 8.9 Hz, 1H), 6.76 (s, 1H), 4.39 (q, *J =* 7.1 Hz, 2H), 3.68 - 3.54 (m, 4H), 3.23 - 3.08 (m, 5H), 2.56 (s, 3H), 1.49 (s, 9H), 1.41 (t, *J* = 7.1 Hz, 3H).

Step 3: At room temperature, lithium aluminum hydride (117.5 mg, 3.10 mmol) and tetrahydrofuran (5 mL) were added to a single-necked flask, stirred, and cooled to 0°C. Compound 8-3 (600 mg, 1.55 mmol) was dissolved in tetrahydrofuran (5 mL) and stirred until dissolved. At 0°C, the solution of compound 8-3 in tetrahydrofuran was slowly dropwise added to the solution of lithium aluminum hydride in tetrahydrofuran. The reaction solution was stirred at 0°C for 5 minutes and at 25°C for 20 minutes. Saturated ammonium chloride (50 mL) was added to the reaction solution, and the reaction solution was extracted with ethyl acetate (30 mL × 3). The organic phases were combined, dried over anhydrous sodium sulfate, and filtered, the filtrate was concentrated under reduced pressure, and the crude product was purified by column chromatography (petroleum ether/ethyl acetate = 2/1) to obtain compound 8-4.

LC-MS: m/z :346.1(M+H)⁺.

¹H NMR (400 MHz, CDCl₃) δ 8.12 (br s, 1H), 7.41 (br d, *J =* 8.5 Hz, 1H), 6.85 (dd, *J =* 1.8, 8.6 Hz, 1H), 6.82 - 6.71 (m, 1H), 4.77 (br s, 2H), 3.65 - 3.54 (m, 4H), 3.06 (br s, 4H), 2.24 (br s, 3H), 1.49 (s, 9H).

Step 4: At room temperature, compound 8-4 (600 mg, 1.74 mmol) and chloroform (15 mL) were successively added to a single-necked flask and stirred until dissolved. Manganese dioxide (1.51 g, 17.37 mmol) was added. The reaction solution was stirred at 25°C for 16 hours. The reaction solution was filtered, and the filtrate was concentrated under reduced pressure. The crude product was purified by column chromatography (petroleum ether/ethyl acetate = 4/1) to obtain compound 8-5. LC-MS: m/z :344.1(M+H)⁺.

¹H NMR (400 MHz, CDCl₃) δ 9.89 (s, 1H), 8.71 (br s, 1H), 7.56 (d, *J =* 8.8 Hz, 1H), 6.90 (br d, *J =* 8.4 Hz, 1H), 6.80 - 6.64 (m, 1H), 3.62 (br s, 4H), 3.21 (br s, 4H), 2.58 (s, 3H), 1.49 (s, 9H).

Step 5: At room temperature, compound 8-5 (120 mg, 0.35 mmol) and ethanol (2 mL) were added to a single-necked flask and stirred until dissolved. Compound 1-2 (117.1 mg, 0.38 mmol) and piperidine (29.8 mg, 0.35 mmol) were successively added. The reaction solution was stirred at 70°C for 2 hours. The reaction solution was then cooled to room temperature, filtered, and dried to obtain compound 8-6. LC-MS: m/z :632.1(M+H)⁺.

¹H NMR (400 MHz, DMSO-*d*₆) δ 12.76 (s, 1H), 11.32 (s, 1H), 10.05 (br s, 1H), 8.22 (s, 1H), 7.88 (s, 1H), 7.60 - 7.39 (m, 2H), 7.21 - 7.03 (m, 4H), 7.02 - 6.85 (m, 3H), 3.48 (br s, 4H), 3.23 (br s, 4H), 2.58 (s, 3H), 1.43 (s, 9H).

Step 6: Compound 8-6 (180 mg, 0.28 mmol) and tetrahydrofuran (2 mL) were successively added to the flask and stirred until dissolved, and hydrogen chloride dioxane (4 M, 15 mL) was then added. The reaction solution was stirred and reacted at 25°C for 0.5 hours. The reaction solution was concentrated, the residue was dissolved in tetrahydrofuran (40 mL), aqueous ammonia (6 mL) was added, and the solution was stirred at room temperature for 1 hour. After concentration, water (30 mL) was then added and stirred at room temperature for 1 hour. After post-treatment, compound 8 was obtained. LC-MS: m/z :532.1(M+H)⁺.

¹H NMR (400 MHz, DMSO-*d*₆) δ 12.74 (s, 1H), 8.21 (s, 1H), 7.86 (s, 1H), 7.55 - 7.44 (m, 2H), 7.17 - 7.04 (m, 4H), 6.97 (d, *J =* 8.2 Hz, 1H), 6.91 (br d, *J =* 9.2 Hz, 1H), 6.88 - 6.81 (m, 1H), 3.23 - 3.18 (m, 4H), 2.95 - 2.87 (m, 4H), 2.58 (s, 3H).

Other compounds of the present invention can be prepared by methods similar to those described in the above examples (with appropriate modifications, if necessary).

### Biological Assays:

### Experimental Example 1: IRAK4 kinase inhibition Assay

Cisbio HTRF KinEASE-STK SI kit (62S1PEB) was used in this experiment. The experiment/detection buffer, STK Substrate 1-biotin, Streptavidin-XL665, and STK Antibody-Crypatete were all contained in the kit.

The compound/DMSO was added to a 384 plate (PerkinElmer 6008280) using Echo 665. Subsequently, 5 µL of IRAK4 kinase protein dissolved in an experimental buffer [1 volume of 5 × kinase buffer + 20 mM magnesium chloride (Sigma) solution + 1 mM DTT (Alfa Aesar) solution + 4 volumes of deionized water] at 2 folds reaction concentration was added (final concentration: 10 nM). The shallow well plate was sealed, shaken, and then centrifuged at 1000 rpm for 1 minute and incubated at room temperature for 5 minutes. 5 µL of a 1 : 1 mixture of STK Substrate 1-biotin (final concentration: 1 µM) and ATP (Sigma A1852) (final concentration: 200 µM) at 2 folds reaction concentration prepared with the experimental buffer was then added. The shallow well plate was sealed, shaken, then centrifuged at 1000 rpm for 1 minute, and incubated at 37°C for 2 hours. Subsequently, 10 µL of a 1 : 1 mixed solution of Streptavidin-XL665 (final concentration: 62.5 nM) and STK-antibody-cryptate dissolved with the detection buffer was added. The shallow well plate was sealed, shaken, then centrifuged at 1000 rpm for 1 minute, and incubated at room temperature for 1 hour. Subsequently, numerical values were read at the wavelength of 620 nm/665 nm by a microplate reader. The data were analyzed by Prism 9.3 software, and the IC₅₀ value of IRAK4 kinase inhibition was fitted by four-parameter Logistic equation. After detection, the compound of the present invention has a better IRAK4 kinase inhibitory activity. The test results of the exemplary compounds are shown in Table 1.

**Table 1: IRAK4 inhibitory activity of compounds**

| Test compound | IC₅₀ (nM) |
|---|---|
| 1 | D |
| 2 | B |
| 3 | A |
| 4 | B |
| 5 | A |
| 6 | C |
| 7 | A |
| 8 | A |

| | |
|---|---|
| Note: A < 100 nM; 100 nM ≤ B ≤ 500 nM; 500 nM < C ≤ 1000 nM; and 1000 nM < D. | |

### Experimental Example 2: HiBit fluorescence detection of protein degradation

Using HiBiT tag technology, an HiBiT-containing tag was linked to IRAK4 plasmid (Beijing Tsingke Biotech Co., Ltd.), and the plasmid was transfected into HEK293T cells (Chinese Academy of Sciences, 1101HUM-PUMC000212). The HiBiT fluorescence level was detected to characterize the degradation level of the compound on IRAK4. With 1 mL of fresh culture medium, 300000 HEK293T cells were inoculated into a 6-well plate (Corning 3516) and placed in a thermostatic incubator at 37°C and 5% CO₂ overnight. 3 µL of X-tremeGENE^{™} 9 DNA Transfection Reagent (Roche #06365787001) and 1 µg of pcdna3.1-IRAK4-HiBiT (Beijing Tsingke Biotech Co., Ltd.) were added to 200 µL of Opti-MEM (Gibico 31985062) and gently mixed until uniform. A 6-well plate incubated overnight was taken out and left to stand at room temperature for 15 minutes, and the mixture was then dropwise added to the 6-well plate. After 6 hours of transient transfection, the cells were trypsinized and centrifuged at 1000 rpm for 3 minutes. Using 40 µL of fresh culture medium, 5000 HEK293T cells were inoculated into a 384-well plate (Corning 3765) and placed in a thermostatic incubator at 37°C and 5% CO₂ overnight. Then, 40 nL of the compound was added to a 384-well plate by using Echo 655 Liquid Handler, with the highest final concentration being 10 µM, followed by serial dilution at 1 : 3 to obtain a total of 10 different concentrations. Culture was performed at 37°C, 5% CO₂ for 24 hours. The 384-well plate was taken out and equilibrated to room temperature. 40 µL/per well of Nano-Glo^{®} HiBiT Lytic Detection System (Promega N3030) was added, followed by incubation at room temperature for 15 minutes, and fluorescence value was recorded using EnVision Xcite Multilabel Reader (PerkinElmer 2105-0020). The data were analyzed by Prism 9.3 software, and the DC₅₀ value of IRAK4 degradation was fitted by four-parameter Logistic equation.

The results show that the PROTAC molecule of the present invention has a better degradation activity for IRAK4.

In addition to those described herein, various modifications of the present invention will be apparent to those skilled in the art from the foregoing description. Such modifications are also intended to fall within the scope of the appended claims. All references cited in the present application (including all patents, patent applications, journal articles, books and any other publications) are incorporated herein by reference in their entirety.

## Claims

1. A compound of formula (I): 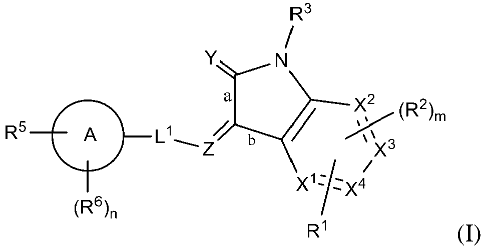
or a stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof,
**characterized in that**:
ring 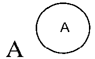
is selected from: C₃₋₁₀ cyclohydrocarbyl, 5- to 10-membered heterocyclyl, C₆₋₁₀ aryl, and 5- to 10-membered heteroaryl;
X¹, X², X³, and X⁴ are each independently N or CH; and at least one of X¹, X², X³, and X⁴ is not N;
Y is O or S; Z is N or CR⁴;
the characters "a" and "b" respectively indicate bonds between a ring carbon atom connected to Z and two adjacent ring carbon atoms;
wherein: when Z is CR⁴, the moiety 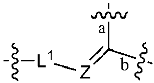
is represented by the following structure of formula (i) or (ii): 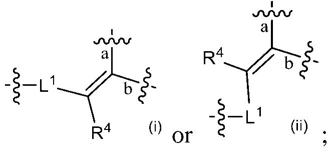
when Z is N, the moiety 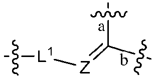
is represented by the following structure of formula (iii) or (iv): 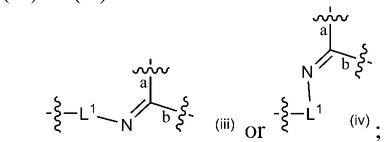
L¹ is selected from: a direct bond, O, S, C(O), S(O), S(O)₂, and NR⁷;
R¹ is -L²-R^{1a};
L² is -NR^{1b}-*, S(O)₂NR^{1b}-*, -C(O)-NR^{1b}-*, -CR^{1c}R^{1d}-NR^{1b}-*, -NR^{1b}-C(O)-*, or -NR^{1b}-S(O)₂-*, wherein the bond indicated by * is connected to R^{1a};
R^{1a} is selected from: C₁₋₆ alkyl, C₁₋₆ haloalkyl, C₂₋₆ alkenyl, C₂₋₆ haloalkenyl,-C₁₋₆ alkylene-O-C₁₋₆ alkyl, -OC₁₋₆ alkylene-OC₁₋₆ alkyl, -C₁₋₆ alkylene-OH, -C₁₋₆ alkylene-SH, -C₁₋₆ alkylene-CN, -C₁₋₆ alkylene-NR^{1e}R^{1f}, and cyclic group Cy1, wherein the cyclic group Cy1 is selected from C₃₋₁₀ cyclohydrocarbyl, 3- to 10-membered heterocyclyl, C₆₋₁₀ aryl, and 5- to 10-membered heteroaryl, wherein the C₃₋₁₀ cyclohydrocarbyl, 3- to 10-membered heterocyclyl, C₆₋₁₀ aryl, and 5- to 10-membered heteroaryl are each optionally substituted with one or more substituents independently selected from the following groups: halogen, OH, SH, -NR^{1e}R^{1f}, CN, C₁₋₆ alkyl, C₁₋₆ haloalkyl, C₂₋₆ alkenyl, C₂₋₆ haloalkenyl, -O-C₁₋₆ alkyl, -O-C₁₋₆ haloalkyl, -S-C₁₋₆ alkyl, -C₁₋₆ alkylene-O-C₁₋₆ alkyl, -OC₁₋₆ alkylene-OC₁₋₆ alkyl,-C₁₋₆ alkylene-OH, -C₁₋₆ alkylene-SH, -C₁₋₆ alkylene-CN, and -C₁₋₆ alkylene-NR^{1e}R^{1f};
R^{1c} and R^{1d} and each R² are independently selected from: H, halogen, OH, SH, -NR^{2a}R^{2b}, CN, C₁₋₆ alkyl, C₁₋₆ haloalkyl, C₂₋₆ alkenyl, C₂₋₆ haloalkenyl, -O-C₁₋₆ alkyl, -O-C₁₋₆ haloalkyl, -S-C₁₋₆ alkyl, -C₁₋₆ alkylene-O-C₁₋₆ alkyl, -OC₁₋₆ alkylene-OC₁₋₆ alkyl, -C₁₋₆ alkylene-OH, -C₁₋₆ alkylene-SH, -C₁₋₆ alkylene-CN, and -C₁₋₆ alkylene-NR^{2a}R^{2b};
m is 0, 1, 2, or 3;
R³ and R⁷ are each independently selected from: H, C₁₋₆ alkyl, C₁₋₆ haloalkyl, C₂₋₆ alkenyl, C₂₋₆ haloalkenyl, -C₁₋₆ alkylene-O-C₁₋₆ alkyl, -C₁₋₆ alkylene-OH, -C₁₋₆ alkylene-SH, -C₁₋₆ alkylene-CN, and -C₁₋₆ alkylene-NR^{3a}R^{3b};
R⁴ is selected from: H, D, halogen, OH, SH, -NR^{4a}R^{4b}, CN, C₁₋₆ alkyl, C₁₋₆ haloalkyl, C₂₋₆ alkenyl, C₂₋₆ haloalkenyl, -C₁₋₆ alkylene-O-C₁₋₆ alkyl, -C₁₋₆ alkylene-OH, -C₁₋₆ alkylene-SH, -C₁₋₆ alkylene-CN, and -C₁₋₆ alkylene-NR^{4a}R^{4b};
R⁵ is selected from: halogen, OH, SH, -NR^{5a}R^{5b}, CN, C₁₋₆ alkyl, C₁₋₆ haloalkyl, C₂₋₆ alkenyl, C₂₋₆ haloalkenyl, -O-C₁₋₆ alkyl, -O-C₁₋₆ haloalkyl, -S-C₁₋₆ alkyl, -C₁₋₆ alkylene-O-C₁₋₆ alkyl, -OC₁₋₆ alkylene-OC₁₋₆ alkyl, -C₁₋₆ alkylene-OH, -C₁₋₆ alkylene-SH, -C₁₋₆ alkylene-CN, -C₁₋₆ alkylene-NR^{5a}R^{5b}, cyclic group Cy2, and -C₁₋₆ alkylene-Cy2, wherein the group Cy2 is selected from C₃₋₁₀ cyclohydrocarbyl, 3-to 10-membered heterocyclyl, C₆₋₁₀ aryl, and 5- to 10-membered heteroaryl, wherein the C₃₋₁₀ cyclohydrocarbyl, 3- to 10-membered heterocyclyl, C₆₋₁₀ aryl, and 5- to 10-membered heteroaryl are each optionally substituted with one or more substituents independently selected from the following groups: halogen, OH, SH, -NR^{5a}R^{5b}, CN, C₁₋₆ alkyl, C₁₋₆ haloalkyl, C₂₋₆ alkenyl, C₂₋₆ haloalkenyl, -O-C₁₋₆ alkyl, -O-C₁₋₆ haloalkyl, -S-C₁₋₆ alkyl, -C₁₋₆ alkylene-O-C₁₋₆ alkyl, -OC₁₋₆ alkylene-OC₁₋₆ alkyl,-C₁₋₆ alkylene-OH, -C₁₋₆ alkylene-SH, -C₁₋₆ alkylene-CN, and -C₁₋₆ alkylene-NR^{5a}R^{5b}.
R⁶ is selected from: halogen, OH, SH, -NR^{6a}R^{6b}, CN, C₁₋₆ alkyl, C₁₋₆ haloalkyl, C₂₋₆ alkenyl, C₂₋₆ haloalkenyl, -O-C₁₋₆ alkyl, -O-C₁₋₆ haloalkyl, -S-C₁₋₆ alkyl, -C₁₋₆ alkylene-O-C₁₋₆ alkyl, -OC₁₋₆ alkylene-OC₁₋₆ alkyl, -C₁₋₆ alkylene-OH, -C₁₋₆ alkylene-SH, -C₁₋₆ alkylene-CN, and -C₁₋₆ alkylene-NR^{6a}R^{6b};
n is 0, 1, 2, 3, or 4; and
R^{1b}, R^{1e}, R^{1f}, R^{2a}, R^{2b}, R^{3a}, R^{3b}, R^{4a}, R^{4b}, R^{5a}, R^{5b}, R^{6a}, and R^{6b} are, at each occurrence, independently selected from H and C₁₋₆ alkyl.

2. The compound according to claim 1, **characterized in that**:
the compound of formula (I) satisfies at least one of the following (1) to (5):
(1) not 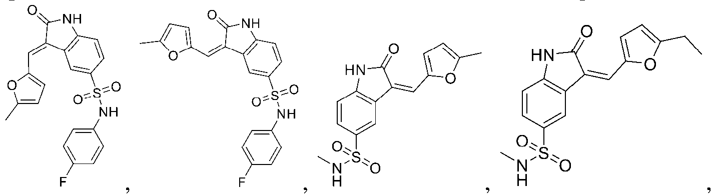 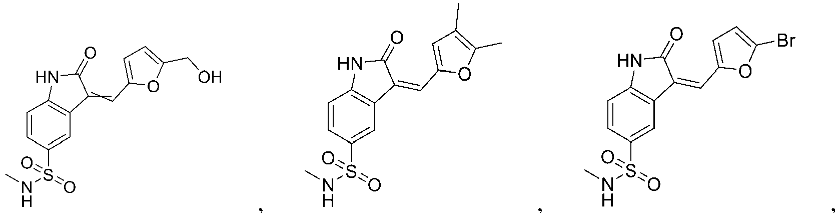 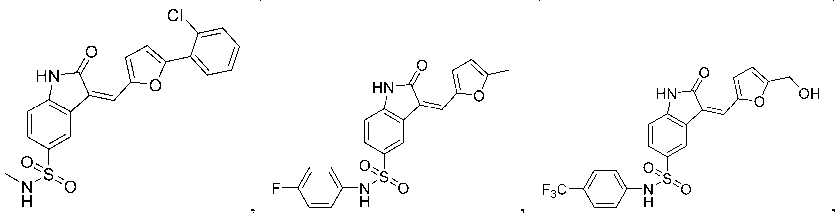 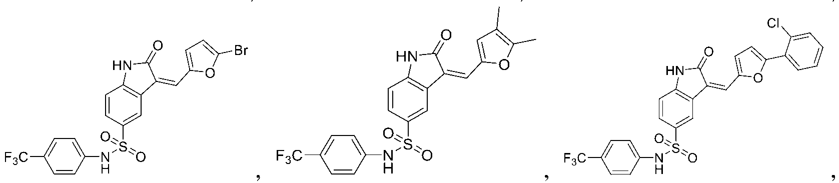 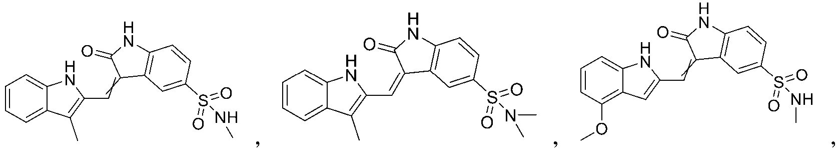 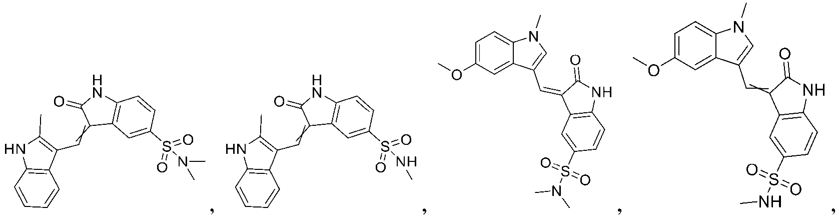 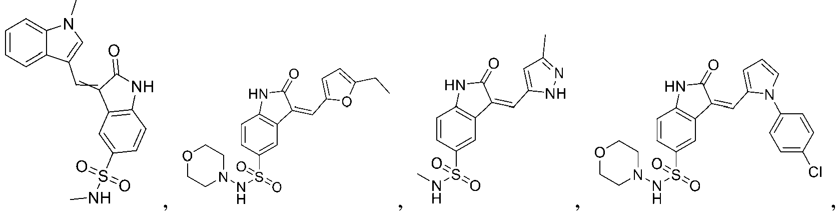 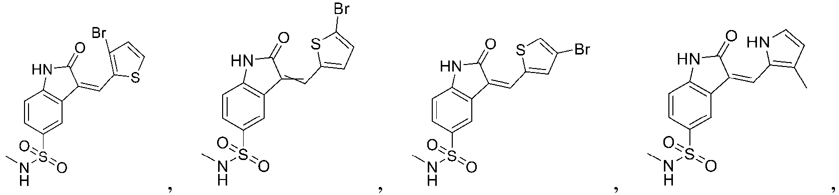 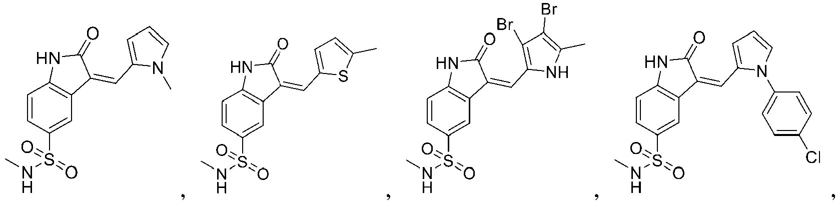 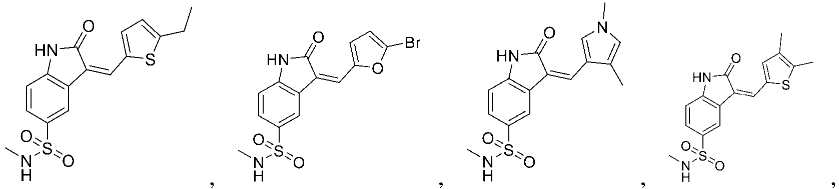 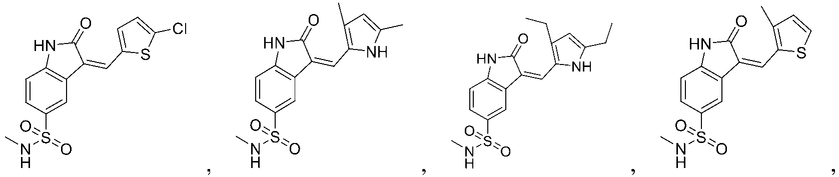 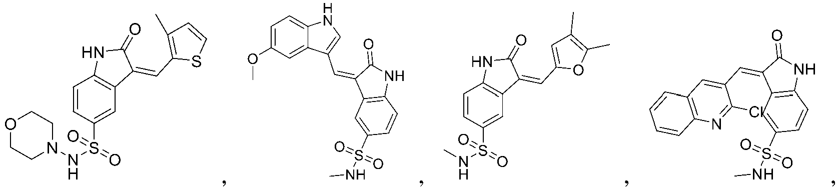 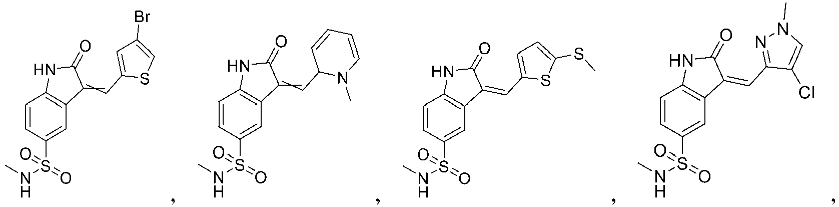 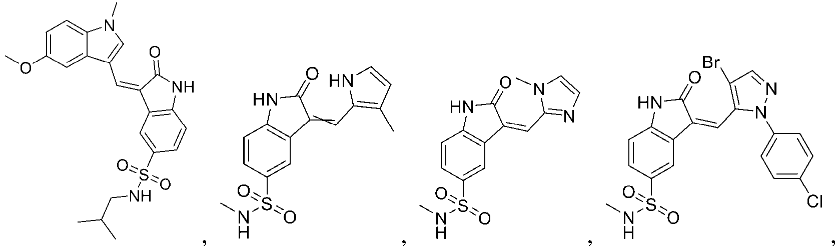 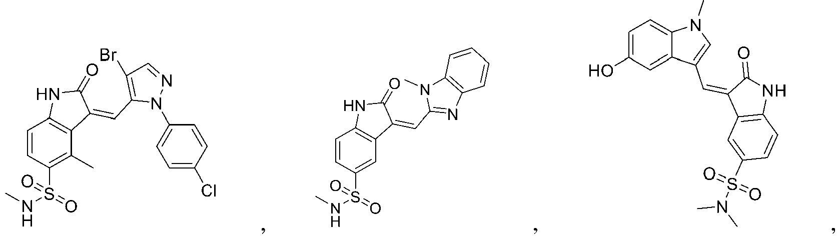 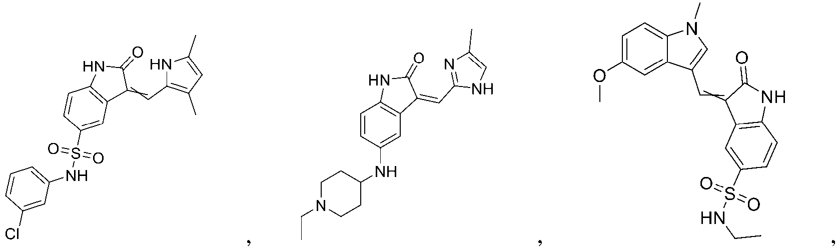 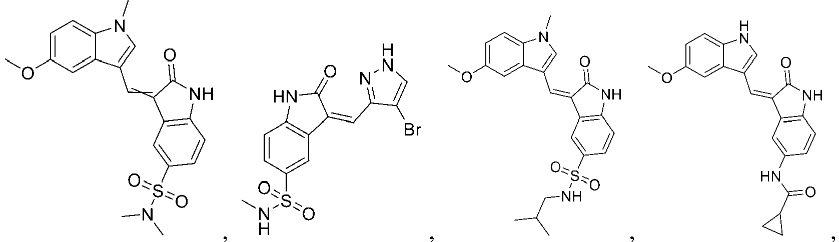 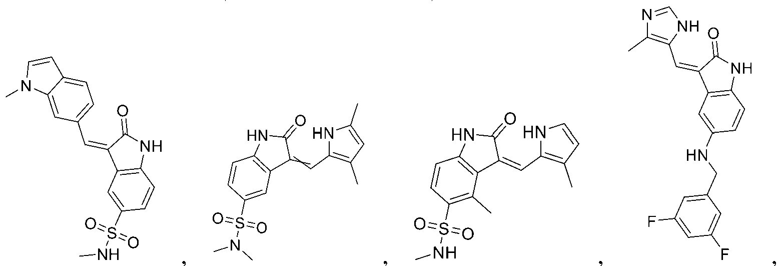 and 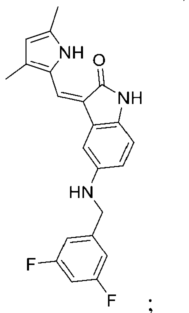
(2) when ring A is indolyl, pyrrolyl, 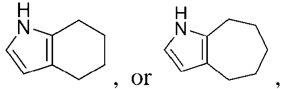 R⁵ is not 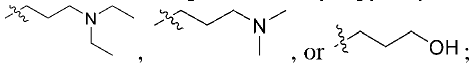
(3) -L²-R^{1a} is not 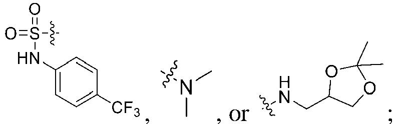
(4) ring A is not tetrahydropyrrolyl, tetrahydrofuryl, tetrahydrothienyl, 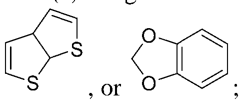 and
(5) when ring A is thienyl or pyrrolyl, -L²-R^{1a} is not

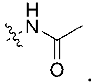

3. The compound according to claim 1 or 2, **characterized in that** the compound has a structure of formula (II):

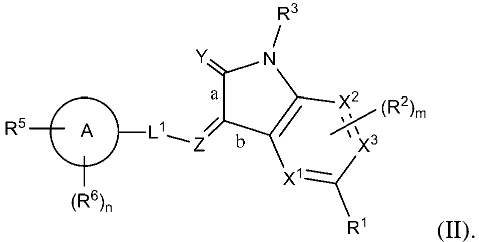

4. The compound according to any one of claims 1 to 3, **characterized in that**:
Y is O;
and/or
ring A is selected from C₃₋₁₀ monocyclic or fused bicyclic cyclohydrocarbyl, 5-to 10-membered monocyclic or fused bicyclic heterocyclyl, C₆₋₁₀ aryl, and 5- to 10-membered monocyclic or fused bicyclic heteroaryl;
preferably, ring A is selected from: C₅₋₆ monocyclic cyclohydrocarbyl, C₉₋₁₀ fused bicyclic cyclohydrocarbyl, 5- to 6-membered monocyclic heterocyclyl, 9- to 10-membered fused bicyclic heterocyclyl, phenyl, naphthyl, 5- to 6-membered monocyclic heteroaryl, and 9- to 10-membered fused bicyclic heteroaryl;
more preferably, ring A is selected from: 9- to 10-membered fused bicyclic heterocyclyl, 5- to 6-membered monocyclic heteroaryl, and 9- to 10-membered fused bicyclic heteroaryl;
more preferably, ring A is selected from: 5- to 6-membered monocyclic heteroaryl, C₅₋₆ monocyclic cyclohydrocarbyl fused 5- to 6-membered monocyclic heteroaryl, 5- to 6-membered monocyclic heterocyclyl fused 5- to 6-membered monocyclic heteroaryl, benzo 5- to 6-membered monocyclic heteroaryl, and 5- to 6-membered monocyclic heteroaryl fused 5- to 6-membered monocyclic heteroaryl, wherein preferably, ring A is connected to L¹ via the 5- to 6-membered monocyclic heteroaryl ring;
more preferably, ring A is selected from: 5-membered monocyclic heteroaryl, and selected from the following fused bicyclic groups BiCy: C₆ monocyclic cyclohydrocarbyl fused 5-membered monocyclic heteroaryl, 6-membered monocyclic heterocyclyl fused 5-membered monocyclic heteroaryl, benzo 5-membered monocyclic heteroaryl, and 6-membered monocyclic heteroaryl fused 5-membered monocyclic heteroaryl, wherein preferably, ring A is connected to L¹ via the 5-membered monocyclic heteroaryl ring; and/or wherein preferably, the 5-membered monocyclic heteroaryl is selected from furyl, thienyl, oxazolyl, isoxazolyl, thiazolyl, isothiazolyl, oxadiazolyl, thiadiazolyl, pyrrolyl, pyrazolyl, imidazolyl, triazolyl, and tetrazolyl;
more preferably, ring A is selected from: furyl, thienyl, oxazolyl, isoxazolyl, thiazolyl, isothiazolyl, pyrrolyl, pyrazolyl, imidazolyl, and triazolyl, and selected from the following fused bicyclic groups BiCy: C₆ monocyclic cyclohydrocarbyl fused 5-membered monocyclic heteroaryl, 6-membered monocyclic heterocyclyl fused 5-membered monocyclic heteroaryl, benzo 5-membered monocyclic heteroaryl, and 6-membered monocyclic heteroaryl fused 5-membered monocyclic heteroaryl, wherein the 5-membered monocyclic heteroaryl is selected from furyl, thienyl, oxazolyl, isoxazolyl, thiazolyl, isothiazolyl, pyrrolyl, pyrazolyl, imidazolyl, and triazolyl; wherein preferably, ring A is connected to L¹ via the furyl, thienyl, oxazolyl, isoxazolyl, thiazolyl, isothiazolyl, pyrrolyl, pyrazolyl, imidazolyl, or triazolyl;
and/or
wherein preferably, the bicyclic group BiCy has a structure of formula (a), (b), or (c): wherein: 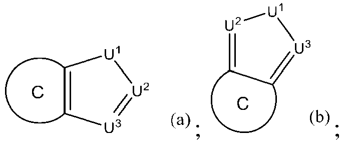
U¹ is O, S, or NH, and U² and U³ are each CH or N; and
at least one of U¹, U², and U³ is CH and connected to L¹ (in this case, H in the CH is replaced); and
ring C is C₆ monocyclic cyclohydrocarbyl, 6-membered monocyclic heterocyclyl, phenyl, or 6-membered monocyclic heteroaryl;
or
wherein: 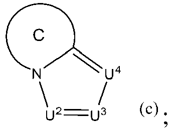
U², U³, and U⁴ are each CH or N; and at least one of U², U³, and U⁴ is CH and connected to L¹ (in this case, H in the CH is replaced); and
ring C is C₆ monocyclic cyclohydrocarbyl, 6-membered monocyclic heterocyclyl, or 6-membered monocyclic heteroaryl;
more preferably, ring A is selected from: 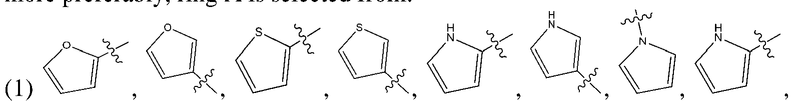 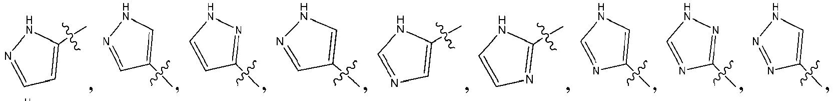 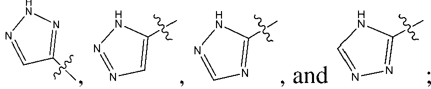 and
(2) the bicyclic group BiCy having a structure of one of formulas (a-1) - (a-16), formulas (b-1) - (b-12), and formulas (c-1) - (c-12):

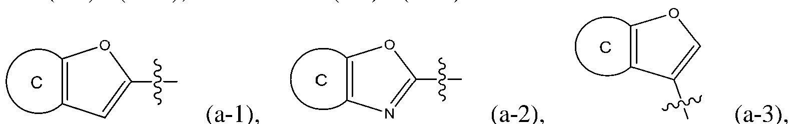

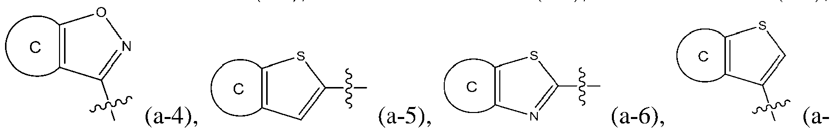

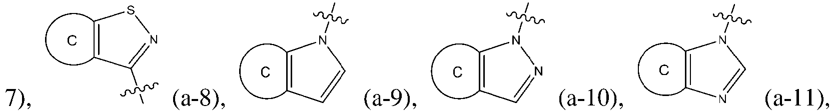

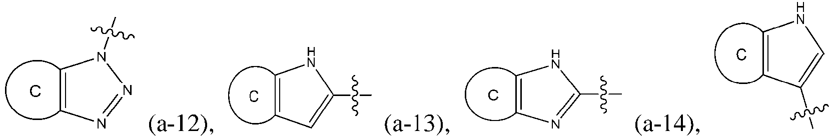

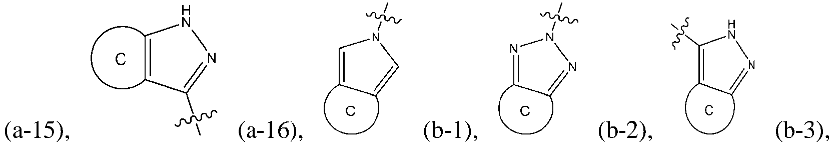

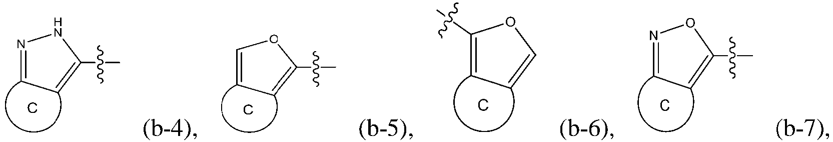

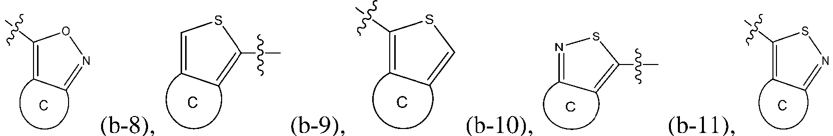

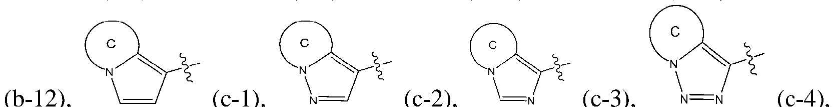

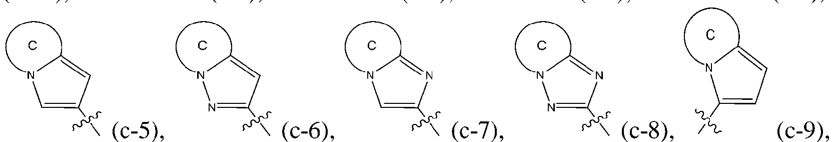

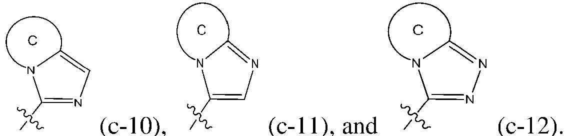

5. The compound according to any one of claims 1 to 4, **characterized in that**:
the C₆ monocyclic cyclohydrocarbyl is cyclohexyl;
and/or
the 6-membered monocyclic heterocyclyl is selected from tetrahydropyridinyl, piperidinyl, tetrahydropyrazinyl, piperazinyl, dihydropyranyl, tetrahydropyranyl, dihydrothiopyranyl, tetrahydrothiopyranyl, dihydrooxazinyl, morpholinyl, dihydrothiazinyl, and thiomorpholinyl, preferably tetrahydropyridinyl or piperidinyl;
and/or
the 6-membered monocyclic heteroaryl is selected from pyridinyl, pyrazinyl, pyridazinyl, pyrimidinyl, and triazinyl, preferably pyridinyl;
and/or
ring C is phenyl, pyridinyl, tetrahydropyridinyl, or piperidinyl;
and/or
the bicyclic group BiCy is selected from benzofuryl, benzothienyl, indolyl, indazolyl, benzimidazolyl, benzotriazolyl, pyrrolopyridinyl, pyrazolopyridinyl, imidazolopyridinyl, pyrrolotetrahydropyridinyl, pyrazolotetrahydropyridinyl, and imidazolotetrahydropyridinyl.

6. The compound according to any one of claims 1-5, **characterized in that**:
the moiety 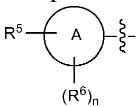 is selected from: 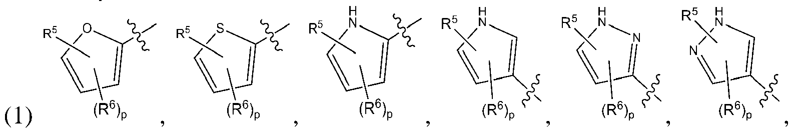 and 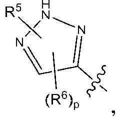 wherein p is 0, 1, or 2;
preferably 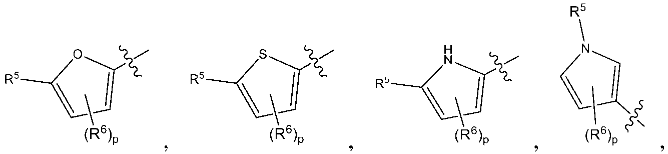 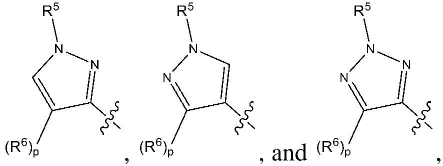 wherein p is 0 or 1;
(2) a structure of formula (a-1'), (a-5'), or (c-1'): 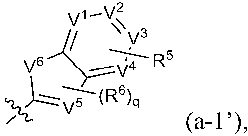 wherein V⁶ is NH, O, or S;
preferably 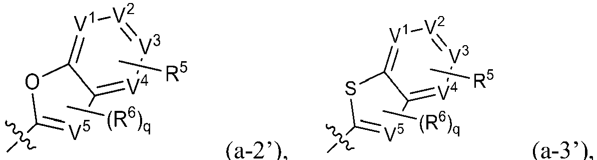 or 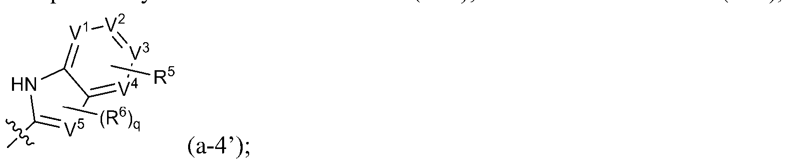
wherein in any one of formulas (a-1') - (a-4'), V¹, V², V³, V⁴, and V⁵ are each independently N or CH (preferably, V¹, V², and V³, and V², V³, and V⁴ are not simultaneously N); wherein preferably, V¹, V², V³, and V⁴ are each CH; or one of V¹, V², V³, and V⁴, preferably V⁴ is N, and the rest are all CH;
or 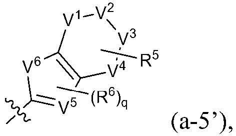 wherein V⁶ is NH, O, or S;
preferably 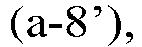 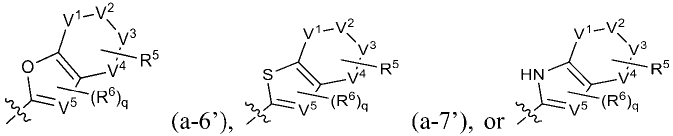
wherein in any one of formulas (a-5') - (a-8'), V¹, V², V³, and V⁴ are each independently NH or CH₂ (and preferably, V¹, V², and V³, or V², V³, and V⁴ are not simultaneously NH), and V⁵ is N or CH; wherein preferably, V¹, V², V³, and V⁴ are each CH₂, or one of V¹, V², V³, and V⁴, preferably V³ is NH, and the rest are all CH₂; and/or V⁵ is CH;
or 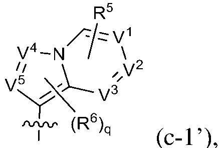 wherein V⁴ and V⁵ are each independently N or CH, preferably V⁴ and V⁵ are not simultaneously N;
preferably 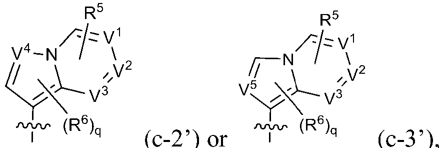
wherein in any one of formulas (c-1') - (c-3'), V¹, V², and V³ are each independently N or CH (preferably V¹, V², and V³ are not simultaneously N); wherein preferably, V¹, V², and V³ are each CH, or one of V¹, V², and V³ is N, and the rest are both CH;
preferably structures of formulas (a-9') - (a-14') and formulas (c-4') - (c-5'): 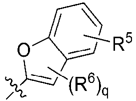 (preferably 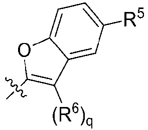 ) (a-9'), 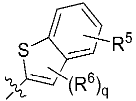 (preferably 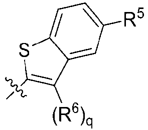 ) (a-10'), 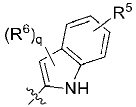 (preferably 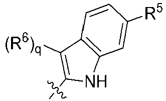 ) (a-11'), 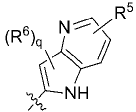 ( 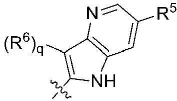 ) (a-12'), 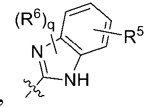 (preferably 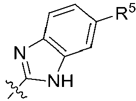 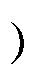 (a-13'), 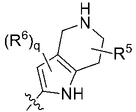 (preferably 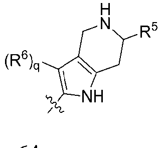 ) (a-14'), 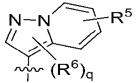 (preferably 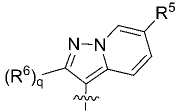 ) (c-4'), and 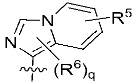 (preferably 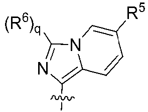 ) (c-5'),
wherein q is 0, 1, 2, 3, or 4, preferably 0 or 1.

7. The compound according to claim 6, **characterized in that** the compound has a structure of one of formulas (III) - (X): 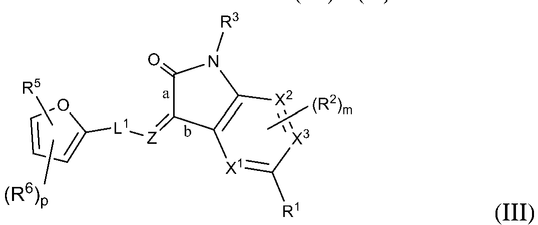 (preferably 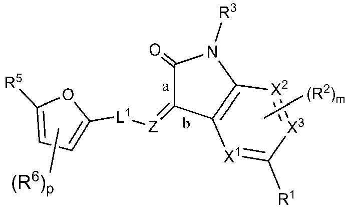 ), 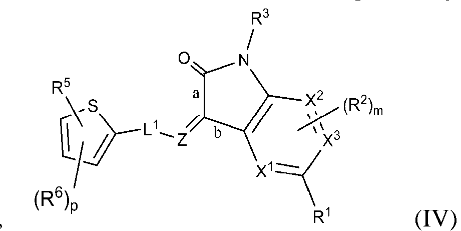 (preferably 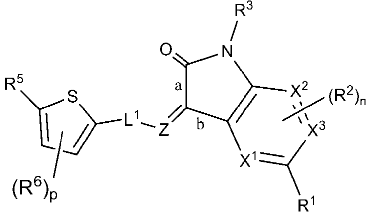 ), 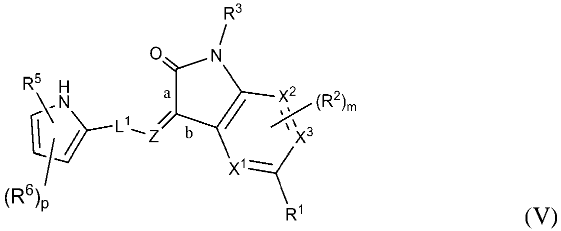 (preferably 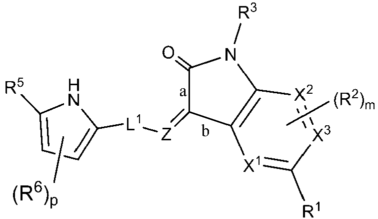 ), 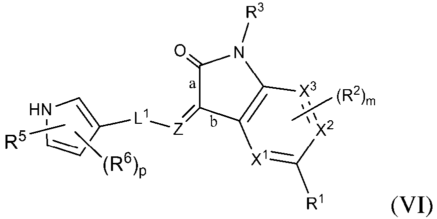 (preferably 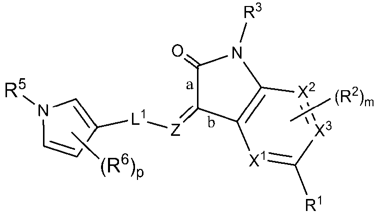 ), 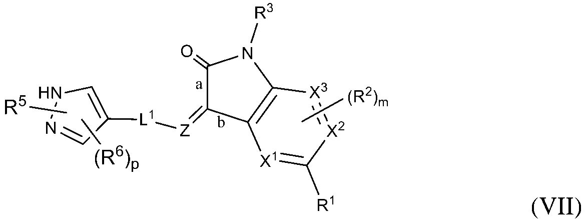 (preferably 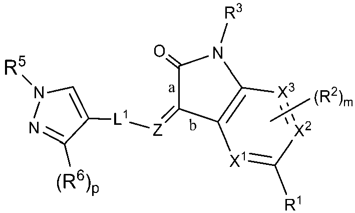 ), 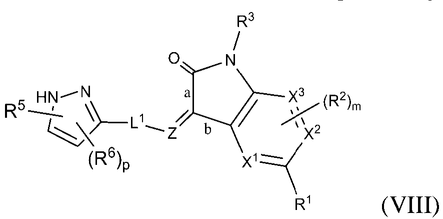 (preferably 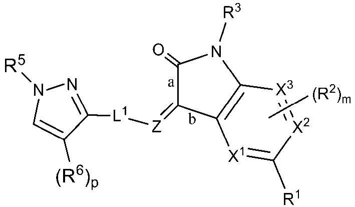 ), 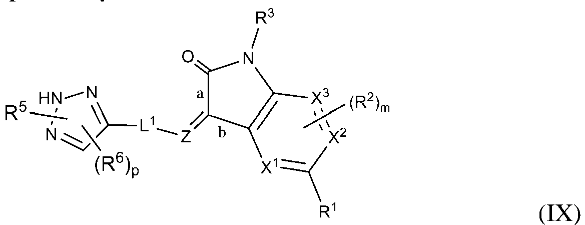 (preferably 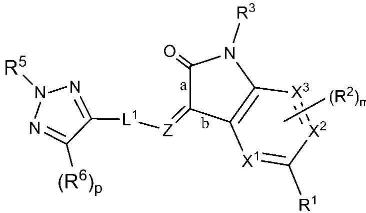 ), and 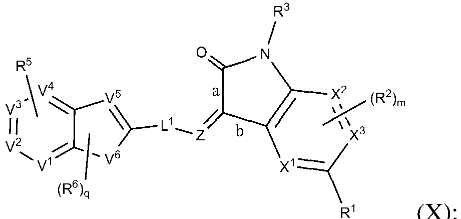
wherein V¹, V², V³, V⁴, V⁵, and V⁶ are each as defined in claim 6 with respect to the formulas (a-1') - (a-4');
preferably 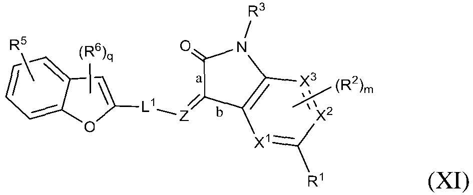 (preferably 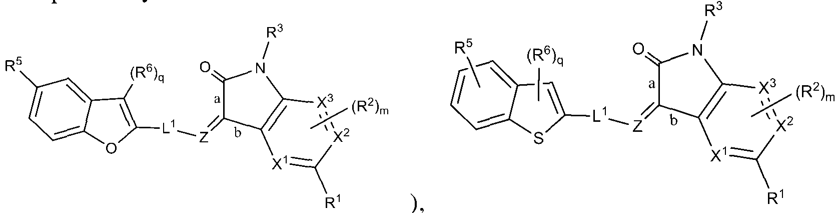 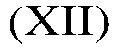 (preferably 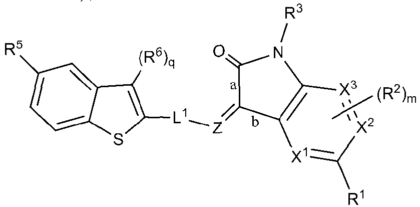 ), 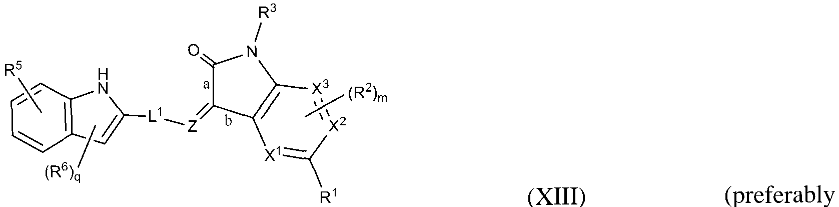 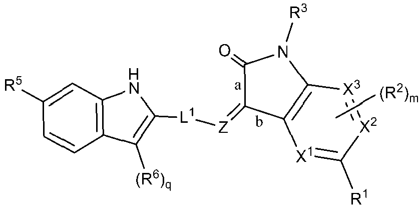 ), 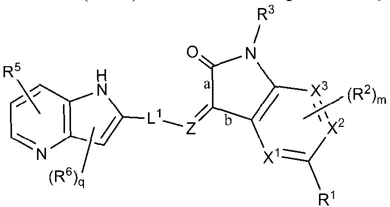 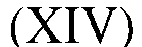 (preferably 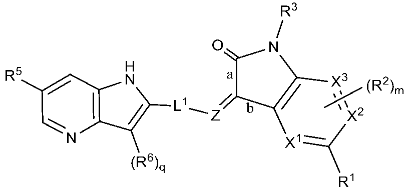 ), and 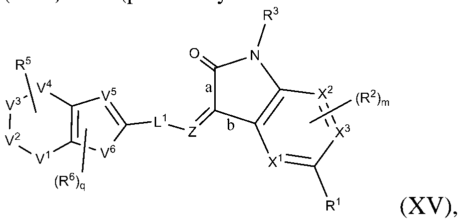
wherein V¹, V², V³, V⁴, V⁵, and V⁶ are each as defined in claim 6 with respect to the formulas (a-5') - (a-8');
preferably 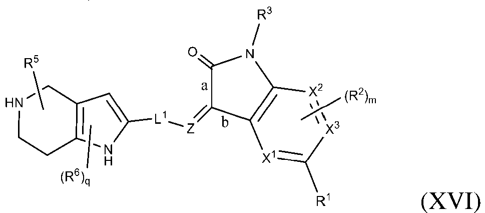 (preferably 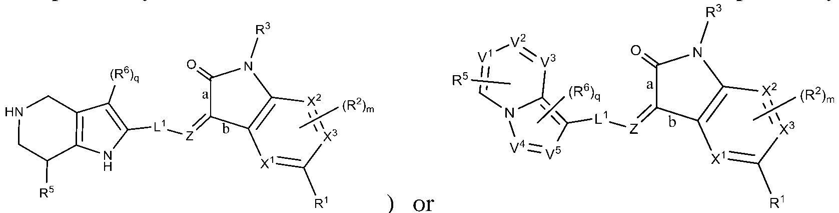 (XVII),
wherein V¹, V², V³, V⁴, and V⁵ are each as defined in claim 6 with respect to the formulas (c-1') - (c-3');
preferably 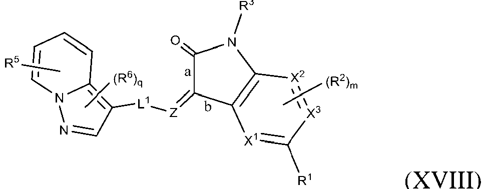 (preferably 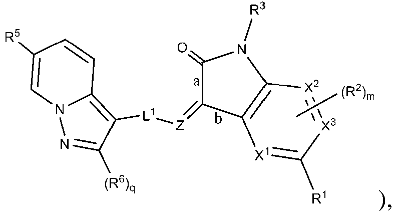
wherein p is 0, 1, or 2, preferably 0 or 1; and
q is 0, 1, 2, 3, or 4, preferably 0 or 1.

8. The compound according to any one of claims 1-7, **characterized in that**:
R^{1a} is the cyclic group Cy1, wherein the cyclic group Cy1 is selected from C₃₋₁₀ cyclohydrocarbyl, 3- to 10-membered heterocyclyl, C₆₋₁₀ aryl, and 5- to 10-membered heteroaryl, wherein the C₃₋₁₀ cyclohydrocarbyl, 3- to 10-membered heterocyclyl, C₆₋₁₀ aryl, and 5- to 10-membered heteroaryl are each optionally substituted with one or more substituents independently selected from the following groups: halogen, OH, SH, -NR^{1e}R^{1f}, CN, C₁₋₄ alkyl, C₁₋₄ haloalkyl, C₂₋₄ alkenyl, C₂₋₄ haloalkenyl, -O-C₁₋₄ alkyl, -O-C₁₋₄ haloalkyl, -S-C₁₋₄ alkyl, -C₁₋₄ alkylene-O-C₁₋₄ alkyl, -OC₁₋₄ alkylene-OC₁₋₄ alkyl, -C₁₋₄ alkylene-OH, -C₁₋₄ alkylene-SH, -C₁₋₄ alkylene-CN, and -C₁₋₄ alkylene-NR^{1e}R^{1f};
preferably, the cyclic group Cy1 is selected from C₃₋₆ cyclohydrocarbyl, 5- to 10-membered heterocycloalkyl, phenyl, and 5- to 10-membered heteroaryl, wherein the C₃₋₆ cyclohydrocarbyl, 5- to 10-membered heterocycloalkyl, phenyl, and 5- to 10-membered heteroaryl are each optionally substituted with one or more substituents independently selected from the following groups: halogen, OH,-NR^{1e}R^{1f}, CN, C₁₋₄ alkyl, C₁₋₄ haloalkyl, -O-C₁₋₄ alkyl, and -O-C₁₋₄ haloalkyl;
more preferably, the cyclic group Cy1 is selected from 5- to 6-membered heterocycloalkyl, phenyl, and 5- to 6-membered heteroaryl, wherein the 5- to 6-membered heterocycloalkyl, phenyl, and 5- to 6-membered heteroaryl are each optionally substituted with one or more substituents independently selected from the following groups: F, Cl, OH -NH₂, CN, methyl, and ethyl; and/or
more preferably, R^{1a} is selected from 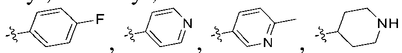 and

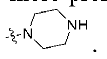

9. The compound according to any one of claims 1-8, **characterized in that** the compound has a structure of one of formulas (XIX) - (XXVII): 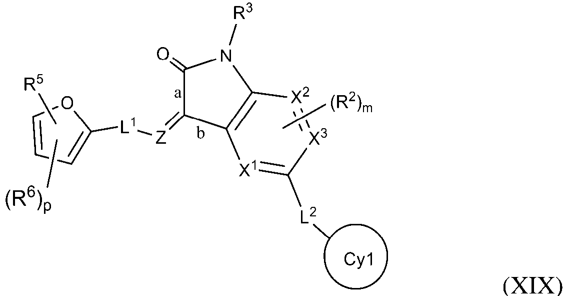 (preferably 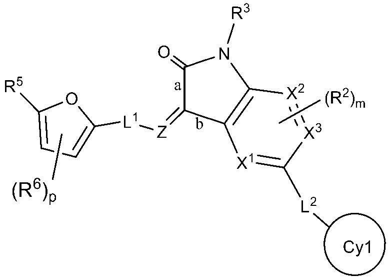 ), 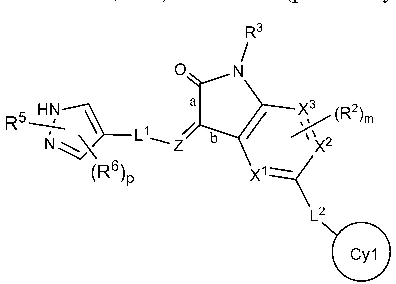 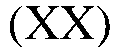 (preferably 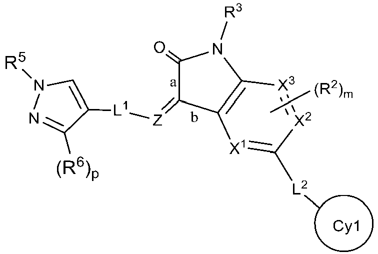 ), 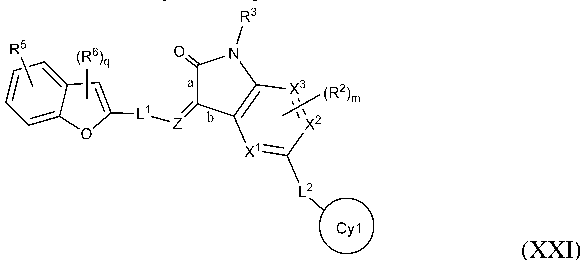 (preferably 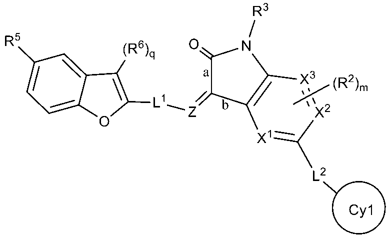 ). 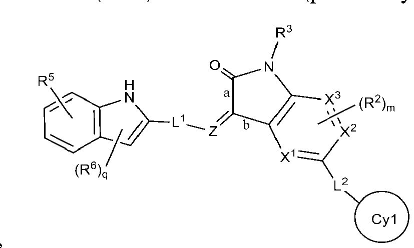 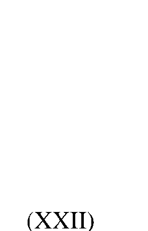 (preferably 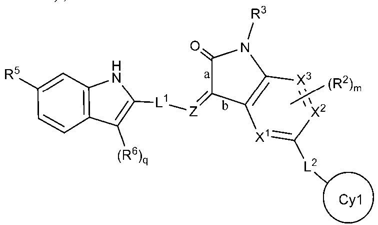 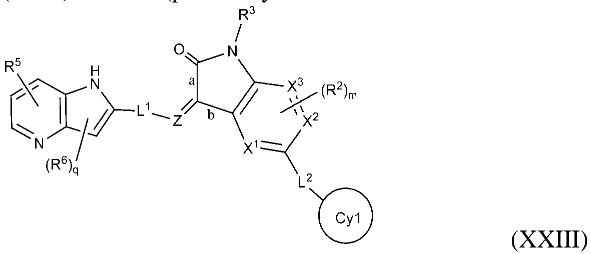 (preferably 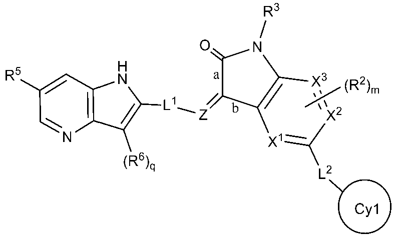 ), 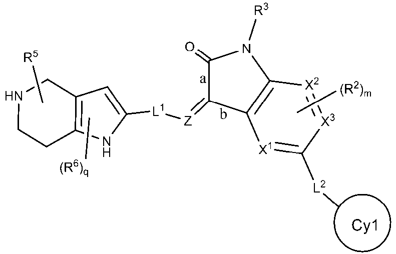 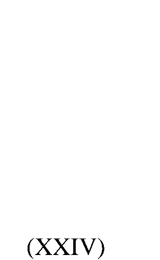 (preferably 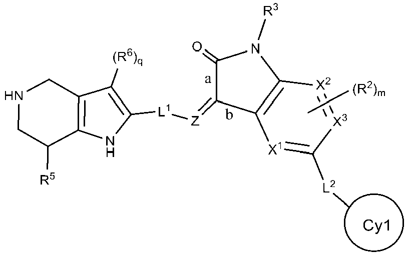 ), 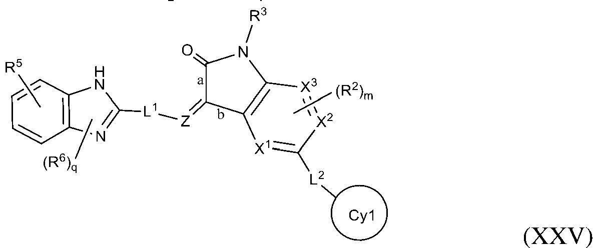 (preferably 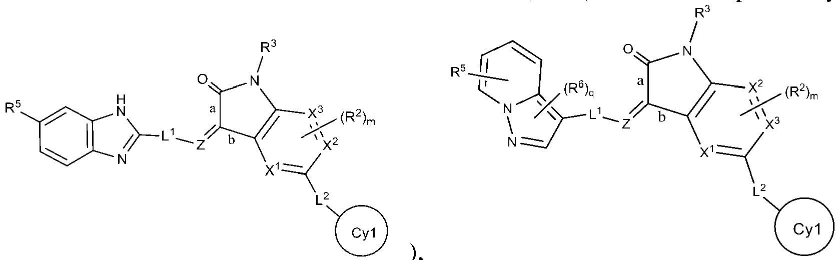 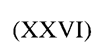 (preferably 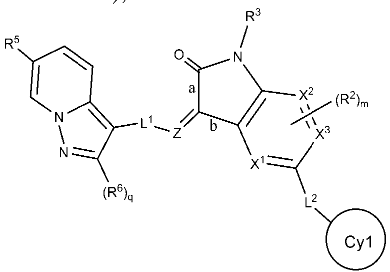 ), and 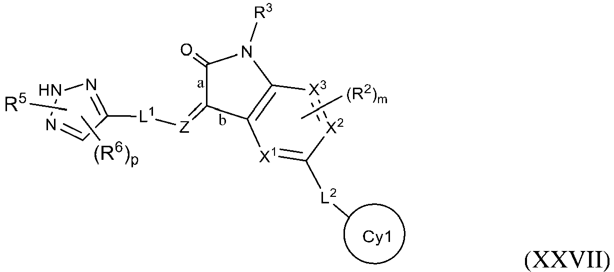 (preferably 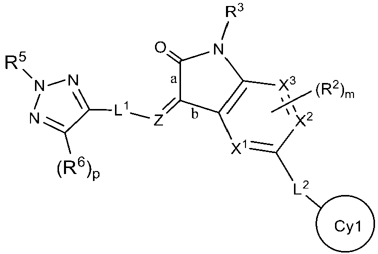 ),wherein:
p is 0, 1, or 2, preferably 0 or 1; and
q is 0, 1, 2, 3, or 4, preferably 0 or 1.

10. The compound according to any one of claims 1-7, **characterized in that**:
R^{1a} is selected from: C₁₋₄ alkyl, C₁₋₄ haloalkyl, C₂₋₄ alkenyl, C₂₋₄ haloalkenyl,-C₁₋₄ alkylene-O-C₁₋₄ alkyl, -OC₁₋₄ alkylene-OC₁₋₄ alkyl, -C₁₋₄ alkylene-OH, -C₁₋₄ alkylene-SH, -C₁₋₄ alkylene-CN, and -C₁₋₄ alkylene-NR^{1e}R^{1f};
preferably R^{1a} is selected from: C₁₋₄ alkyl and C₁₋₄ haloalkyl;
more preferably, R^{1a} is selected from methyl and ethyl.

11. The compound according to any one of claims 1-10, **characterized in that**:
Z is N, wherein preferably, the moiety 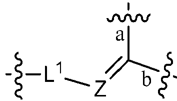
is represented by a structure of formula (iii) or by a structure of formula (iv); or
Z is CR⁴, wherein preferably, the moiety 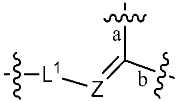
is represented by a structure of formula (i) or by a structure of formula (ii);
and/or
L¹ is selected from: a direct bond and NR⁷; preferably, L¹ is a direct bond, or L¹ is NR⁷.

12. The compound according to any one of claims 1-9 and 11, **characterized in that** the compound has a structure of one of formulas (1) - (37): 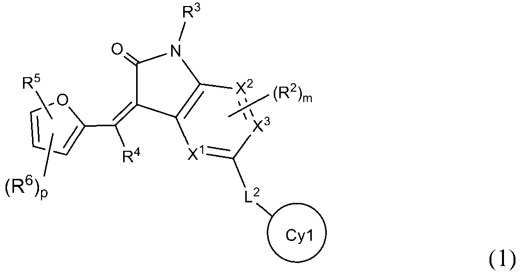 (preferably 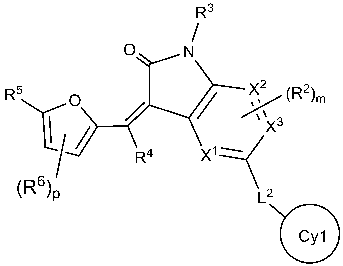 ), 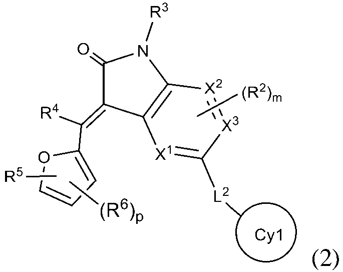 (preferably 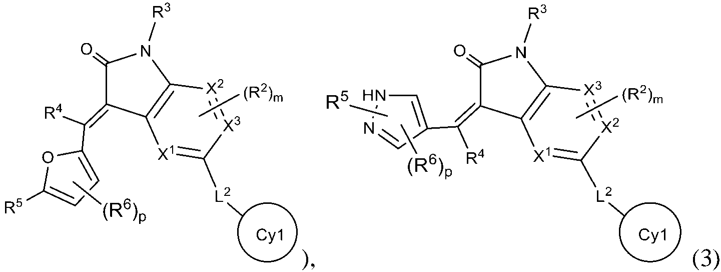 (preferably 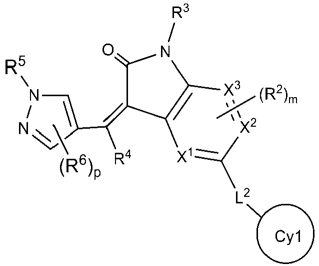 ), 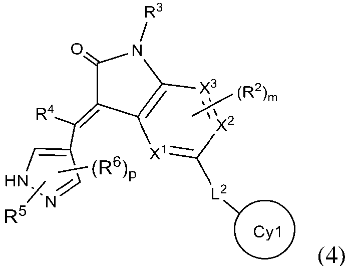 (preferably 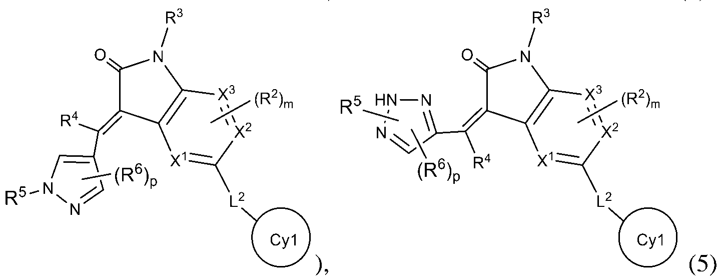 (preferably 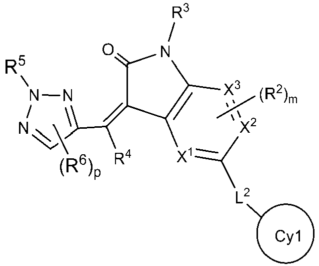 ), 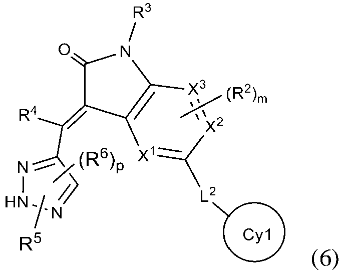 (preferably 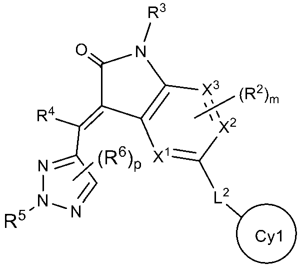 ), 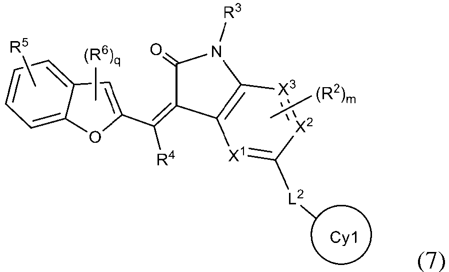 (preferably 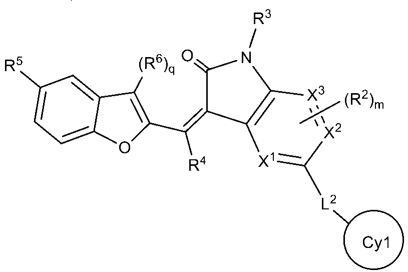 ), 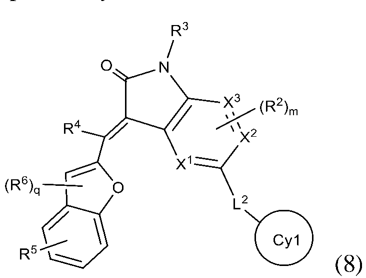 (preferably 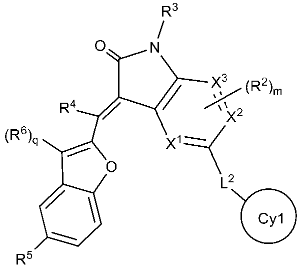 ), 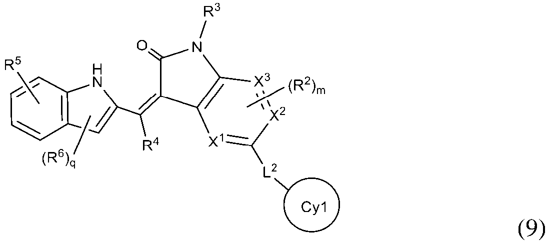 (preferably 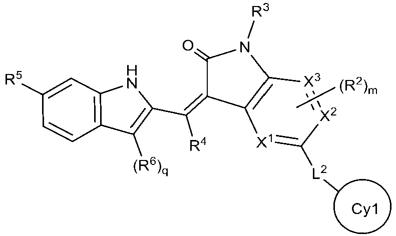 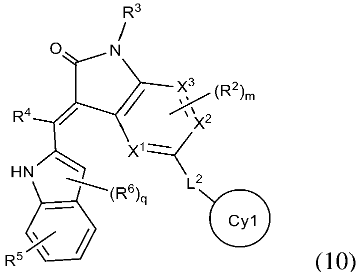 (preferably 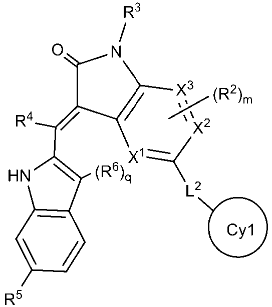 ), 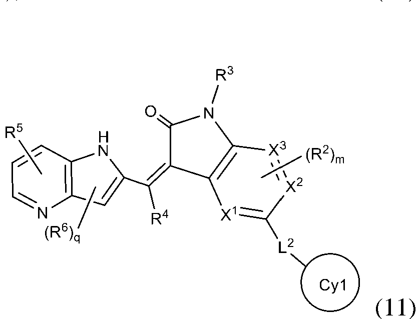 (preferably 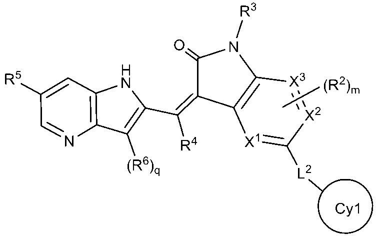 ), 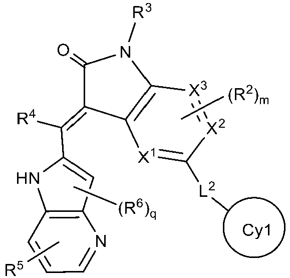 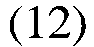 (preferably 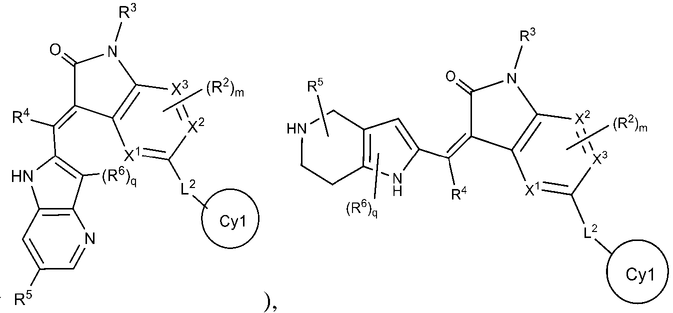 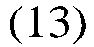 (preferably 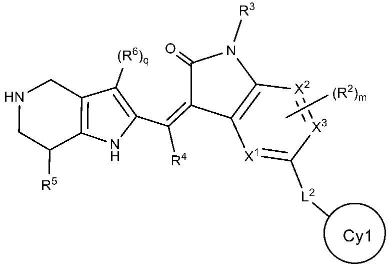 ), 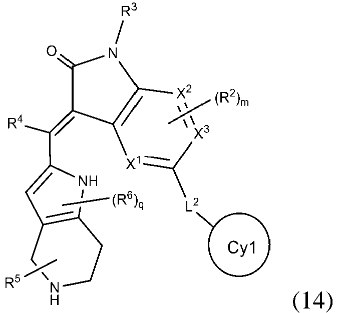 (preferably 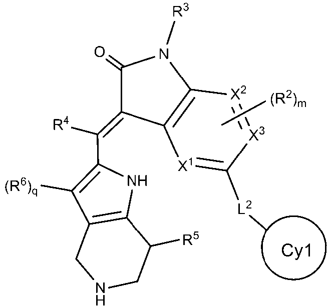 ), 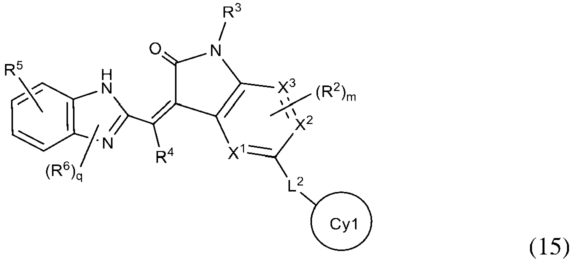 (preferably 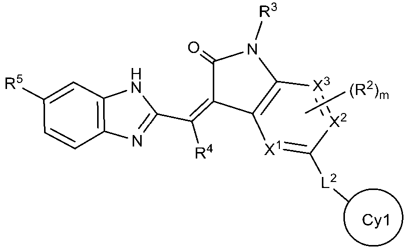 ), 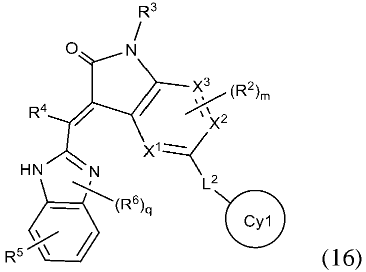 (preferably 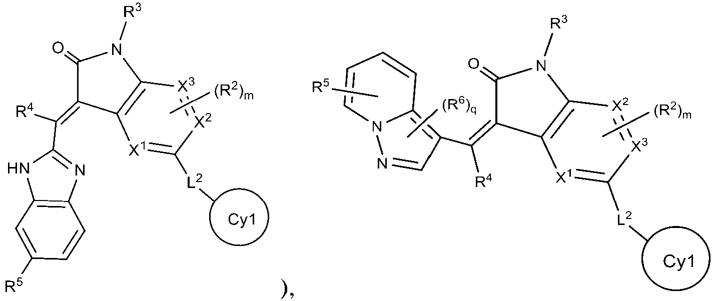 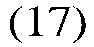 (preferably 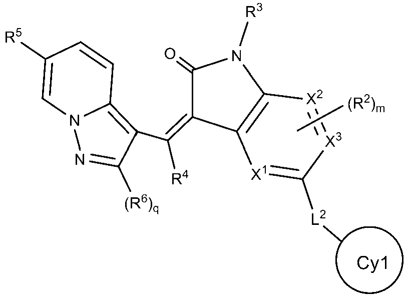 ), 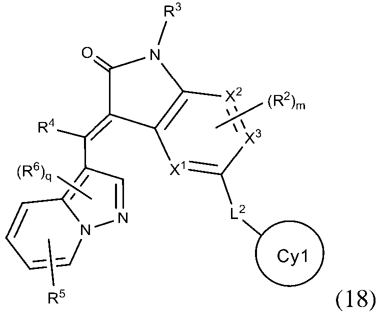 (preferably 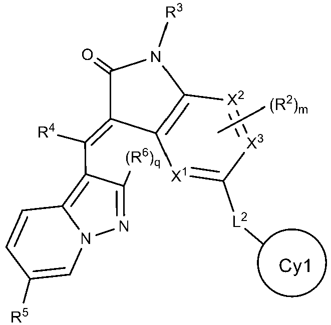 ), 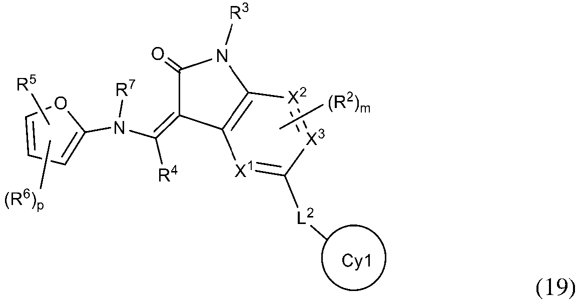 (preferably 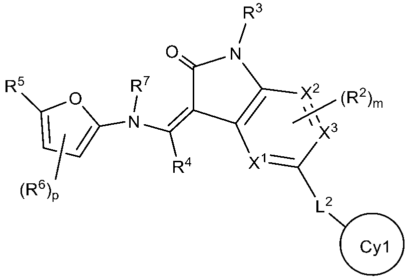 ), 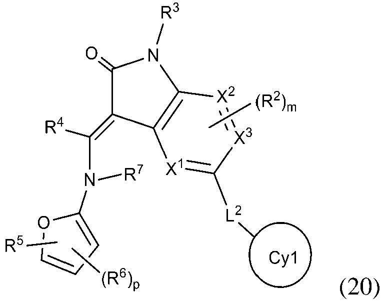 (preferably 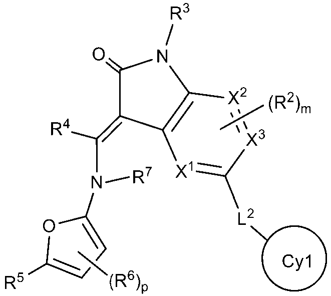 ), 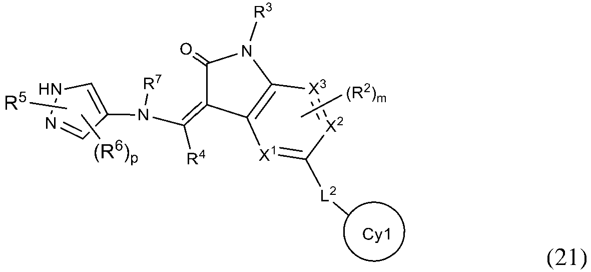 (preferably 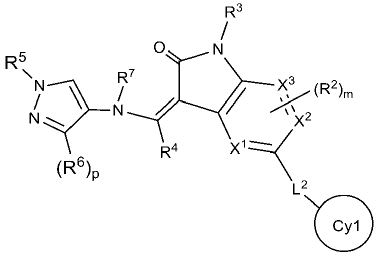 ), 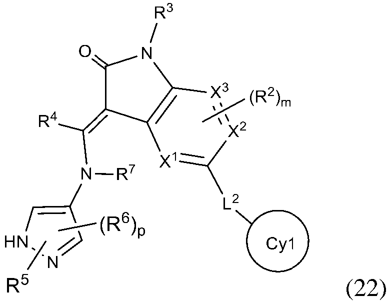 (preferably 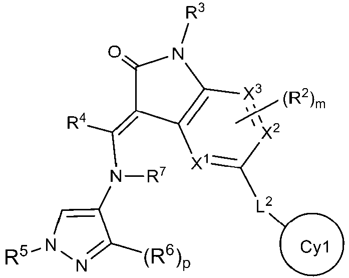 ), 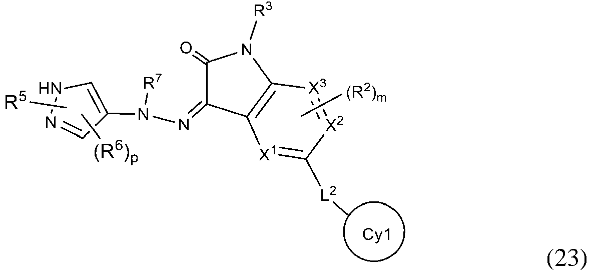 (preferably 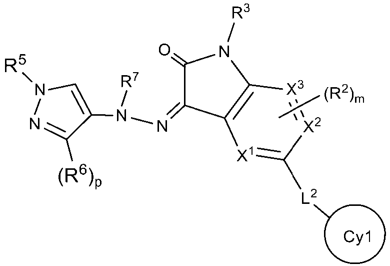 ), 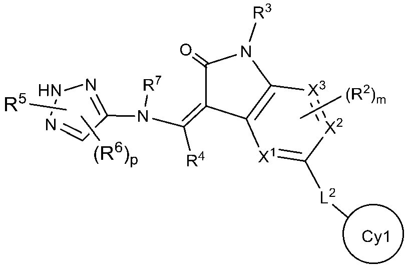 (24) (preferably 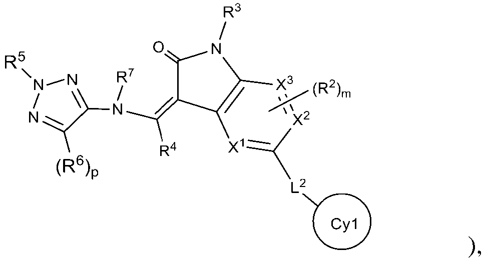 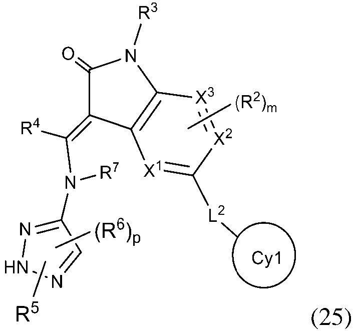 (preferably 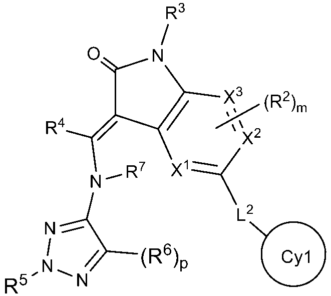 ), 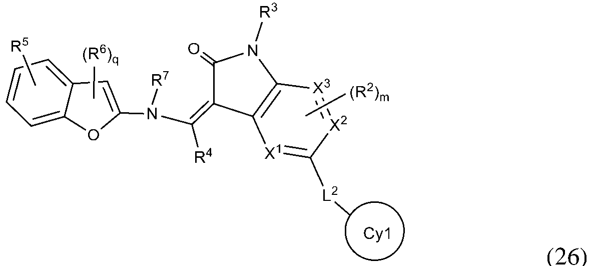 (preferably 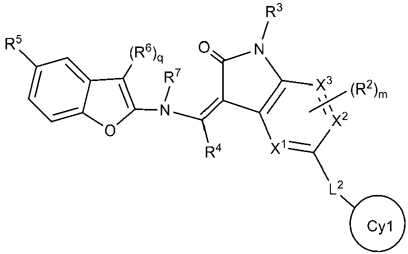 ), 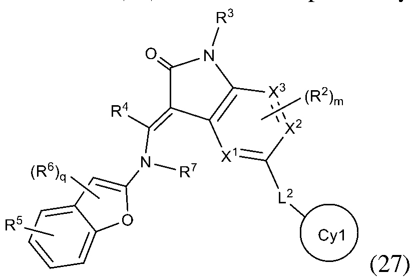 (preferably 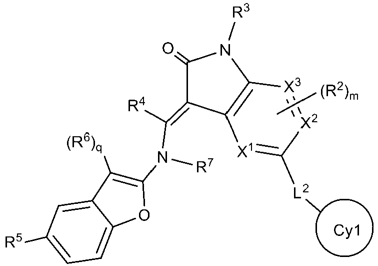 ), 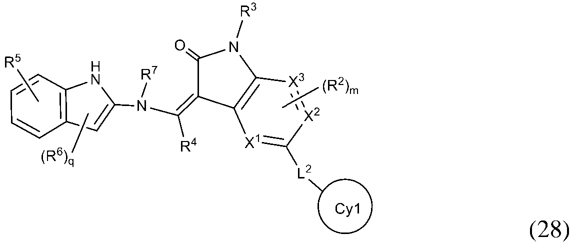 (preferably 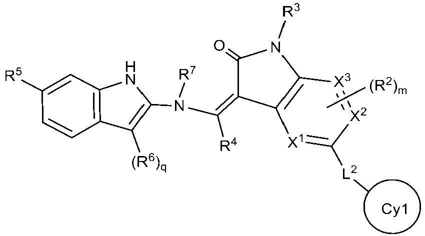 ), 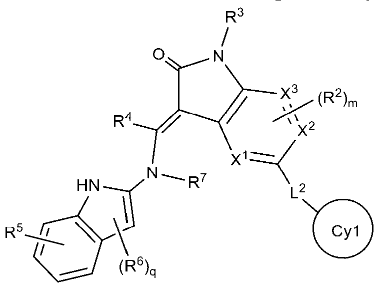 (29) (preferably 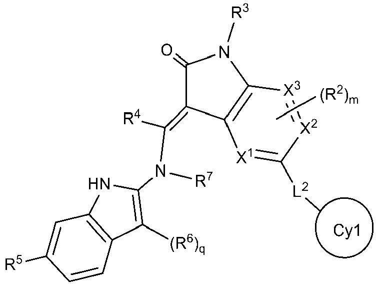 ), 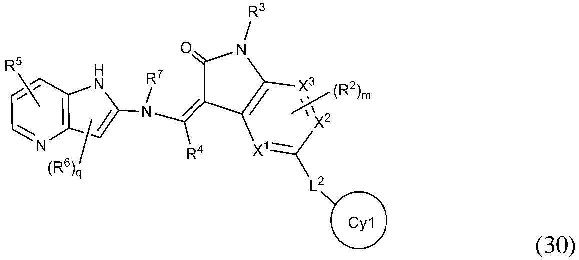 (preferably 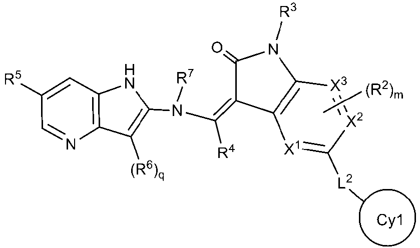 ), 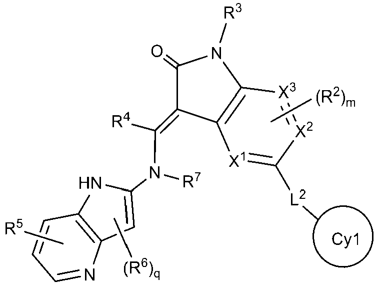 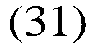 (preferably 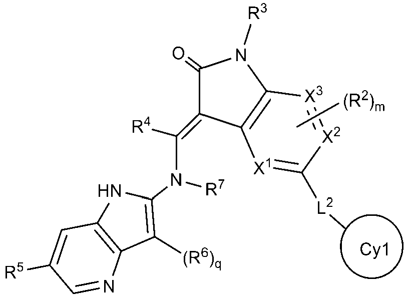 ), 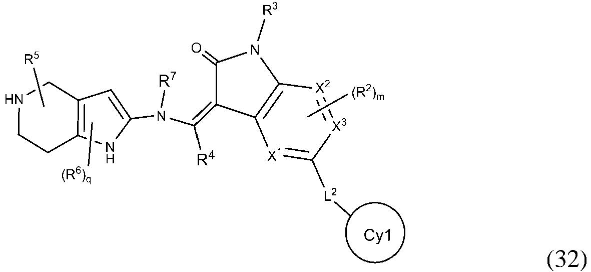 (preferably 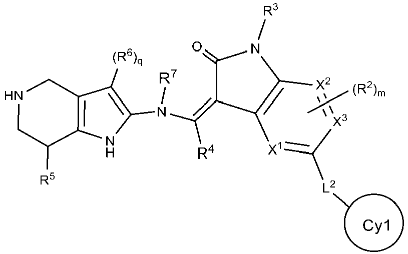 ), 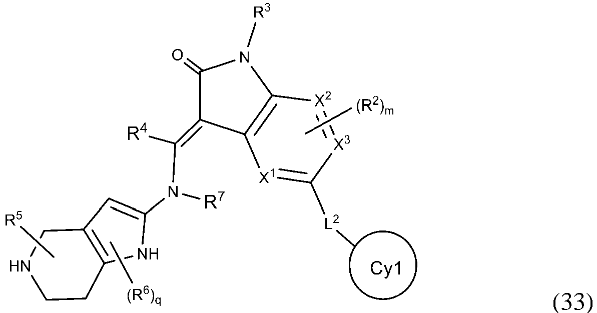 (preferably 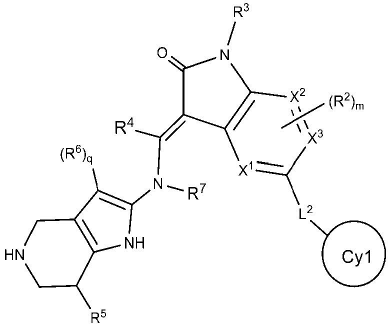 ), 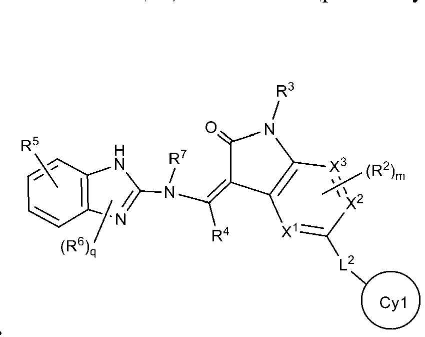 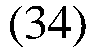 (preferably 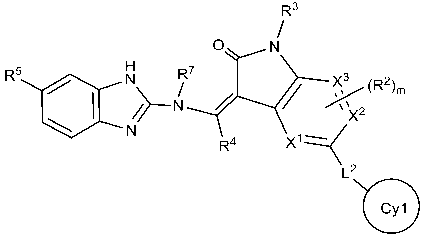 ), 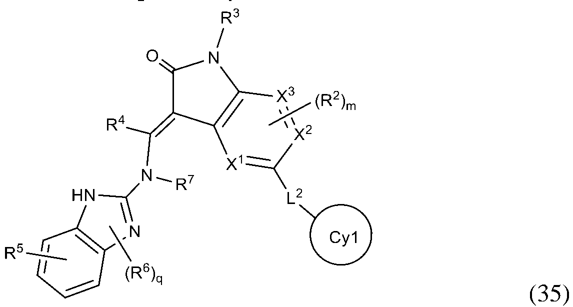 (preferably 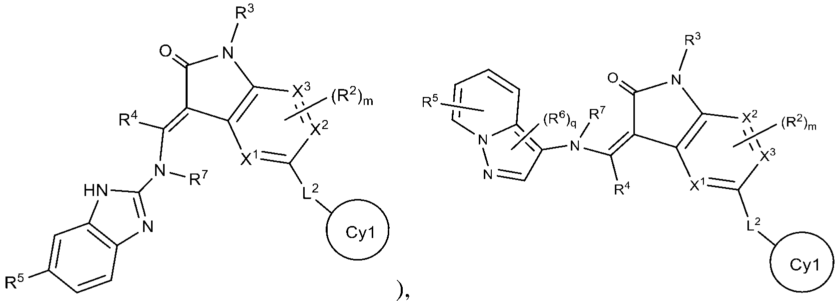 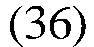 (preferably 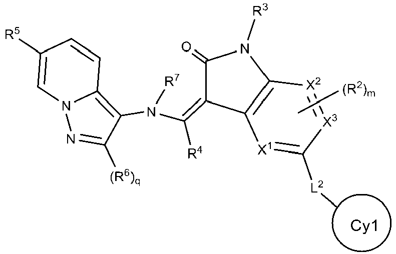 ), and 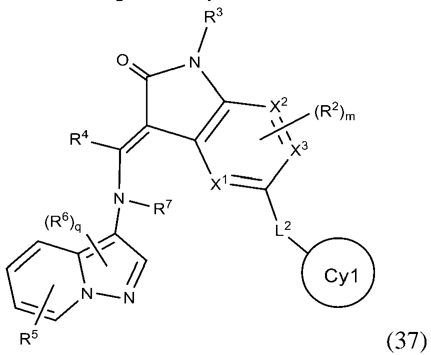 (preferably 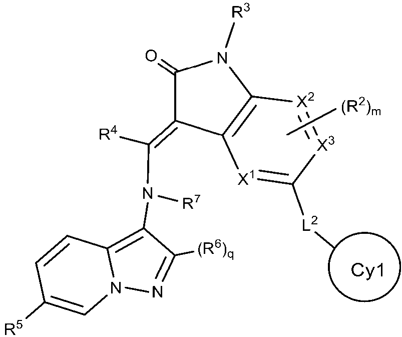 ); wherein:
p is 0, 1, or 2, preferably 0 or 1; and
q is 0, 1, 2, 3, or 4, preferably 0 or 1.

13. The compound according to any one of claims 1-12, **characterized in that**:
X¹, X², and X³ are each CH; or X¹ is N, and X² and X³ are each CH; or X² is N, and X¹ and X³ are each CH; or X³ is N, and X¹ and X² are each CH; or X¹ and X² are each N, and X³ is CH; or X¹ and X³ are each N, and X² is CH; or X² and X³ are each N, and X¹ is CH; or X¹, X², and X³ are each N;
and/or
R³ and R⁷ are each independently selected from: H, C₁₋₄ alkyl, C₁₋₄ haloalkyl, C₂₋₄ alkenyl, C₂₋₄ haloalkenyl, -C₁₋₄ alkylene-O-C₁₋₄ alkyl, -C₁₋₄ alkylene-OH, -C₁₋₄ alkylene-SH, -C₁₋₄ alkylene-CN, and -C₁₋₄ alkylene-NR^{3a}R^{3b}; preferably H and C₁₋₄ alkyl, more preferably H and methyl;
and/or
R⁴ is selected from: H, D, halogen, OH, SH, -NR^{4a}R^{4b}, CN, C₁₋₄ alkyl, C₁₋₄ haloalkyl, C₂₋₄ alkenyl, C₂₋₄ haloalkenyl, -C₁₋₄ alkylene-O-C₁₋₄ alkyl, -C₁₋₄ alkylene-OH, -C₁₋₄ alkylene-SH, -C₁₋₄ alkylene-CN, and -C₁₋₄ alkylene-NR^{4a}R^{4b}; preferably H, D, halogen, OH, -NR^{4a}R^{4b}, CN, C₁₋₄ alkyl, and C₁₋₄ haloalkyl; preferably H, D, F, Cl, OH, -NH₂, CN, methyl, ethyl, -CHF₂, and -CF₃; more preferably H, D, F, Cl, methyl, and ethyl;
and/or
L² is -NR^{1b}-*, -S(O)₂NR^{1b}-*, -C(O)-NR^{1b}-*, -CR^{1c}R^{1d}-NR^{1b}-*, or -NR^{1b}-C(O)-*, preferably -S(O)₂NR^{1b}-*, wherein the bond indicated by * is connected to R^{1a};
and/or
R^{1c} and R^{1d} are each independently selected from: H, halogen, OH, SH,-NR^{2a}R^{2b}, CN, C₁₋₄ alkyl, C₁₋₄ haloalkyl, C₂₋₄ alkenyl, C₂₋₄ haloalkenyl, -O-C₁₋₄ alkyl, -O-C₁₋₄ haloalkyl, -S-C₁₋₄ alkyl, -C₁₋₄ alkylene-O-C₁₋₄ alkyl, -OC₁₋₄ alkylene-OC₁₋₄ alkyl, -C₁₋₄ alkylene-OH, -C₁₋₄ alkylene-SH, -C₁₋₄ alkylene-CN, and -C₁₋₄ alkylene-NR^{2a}R^{2b}; preferably H, F, and Cl;
and/or
each R² is independently selected from: H, halogen, OH, SH, -NR^{2a}R^{2b}, CN, C₁₋₄ alkyl, C₁₋₄ haloalkyl, C₂₋₄ alkenyl, C₂₋₄ haloalkenyl, -O-C₁₋₄ alkyl, -O-C₁₋₄ haloalkyl, -S-C₁₋₄ alkyl, -C₁₋₄ alkylene-O-C₁₋₄ alkyl, -OC₁₋₄ alkylene-OC₁₋₄ alkyl,-C₁₋₄ alkylene-OH, -C₁₋₄ alkylene-SH, -C₁₋₄ alkylene-CN, and -C₁₋₄ alkylene-NR^{2a}R^{2b}; preferably H;
and/or
R⁵ is selected from: halogen, OH, SH, -NR^{5a}R^{5b}, CN, C₁₋₄ alkyl, C₁₋₄ haloalkyl, C₂₋₄ alkenyl, C₂₋₄ haloalkenyl, -O-C₁₋₄ alkyl, -O-C₁₋₄ haloalkyl, -S-C₁₋₄ alkyl, -C₁₋₄ alkylene-O-C₁₋₄ alkyl, -OC₁₋₄ alkylene-OC₁₋₄ alkyl, -C₁₋₄ alkylene-OH, -C₁₋₄ alkylene-SH, -C₁₋₄ alkylene-CN, -C₁₋₄ alkylene-NR^{5a}R^{5b}, cyclic group Cy2, and -C₁₋₄ alkylene-Cy2, wherein the group Cy2 is selected from C₃₋₁₀ cyclohydrocarbyl, 3-to 10-membered heterocyclyl, C₆₋₁₀ aryl, and 5- to 10-membered heteroaryl, wherein the C₃₋₁₀ cyclohydrocarbyl, 3- to 10-membered heterocyclyl, C₆₋₁₀ aryl, and 5- to 10-membered heteroaryl are each optionally substituted with one or more substituents independently selected from the following groups: halogen, OH, SH, -NR^{5a}R^{5b}, CN, C₁₋₄ alkyl, C₁₋₄ haloalkyl, C₂₋₄ alkenyl, C₂₋₄ haloalkenyl, -O-C₁₋₄ alkyl, -O-C₁₋₄ haloalkyl, -S-C₁₋₄ alkyl, -C₁₋₄ alkylene-O-C₁₋₄ alkyl, -OC₁₋₄ alkylene-OC₁₋₄ alkyl,-C₁₋₄ alkylene-OH, -C₁₋₄ alkylene-SH, -C₁₋₄ alkylene-CN, and -C₁₋₄ alkylene-NR^{5a}R^{5b};
preferably, R⁵ is selected from: C₁₋₄ alkyl, C₁₋₄ haloalkyl, C₂₋₄ alkenyl, C₂₋₄ haloalkenyl, and the cyclic group Cy2, wherein the group Cy2 is selected from C₃₋₆ cycloalkyl, 5- to 10-membered heterocycloalkyl, C₆₋₁₀ aryl, and 5- to 10-membered heteroaryl, wherein the C₃₋₆ cycloalkyl, 5- to 10-membered heterocycloalkyl, C₆₋₁₀ aryl, and 5- to 10-membered heteroaryl are each optionally substituted with one or more substituents independently selected from the following groups: halogen, OH, SH, -NR^{5a}R^{5b}, CN, C₁₋₄ alkyl, C₁₋₄ haloalkyl, C₂₋₄ alkenyl, C₂₋₄ haloalkenyl, -O-C₁₋₄ alkyl, -O-C₁₋₄ haloalkyl, -S-C₁₋₄ alkyl, -C₁₋₄ alkylene-O-C₁₋₄ alkyl, -OC₁₋₄ alkylene-OC₁₋₄ alkyl, -C₁₋₄ alkylene-OH, -C₁₋₄ alkylene-SH, -C₁₋₄ alkylene-CN, and -C₁₋₄ alkylene-NR^{5a}R^{5b};
more preferably, R⁵ is selected from: C₁₋₄ alkyl, C₁₋₄ haloalkyl, C₂₋₄ alkenyl, C₂₋₄ haloalkenyl, and the cyclic group Cy2, wherein the group Cy2 is selected from C₃₋₆ cycloalkyl, 5- to 10-membered heterocycloalkyl, C₆₋₁₀ aryl, and 5- to 10-membered heteroaryl, wherein the C₃₋₆ cycloalkyl, 5- to 10-membered heterocycloalkyl, C₆₋₁₀ aryl, and 5- to 10-membered heteroaryl are each optionally substituted with one or more substituents independently selected from the following groups: C₁₋₄ alkyl, C₁₋₄ haloalkyl, -C₁₋₄ alkylene-O-C₁₋₄ alkyl, -OC₁₋₄ alkylene-OC₁₋₄ alkyl, -C₁₋₄ alkylene-OH, -C₁₋₄ alkylene-SH, -C₁₋₄ alkylene-CN, and -C₁₋₄ alkylene-NR^{5a}R^{5b};
more preferably, R⁵ is selected from methyl, cyclopentyl, cyclohexyl, 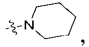 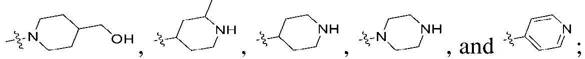 and/or
R⁶ is selected from: halogen, OH, SH, -NR^{6a}R^{6b}, CN, C₁₋₄ alkyl, C₁₋₄ haloalkyl, C₂₋₄ alkenyl, C₂₋₄ haloalkenyl, -O-C₁₋₄ alkyl, -O-C₁₋₄ haloalkyl, -S-C₁₋₄ alkyl, -C₁₋₄ alkylene-O-C₁₋₄ alkyl, -OC₁₋₄ alkylene-OC₁₋₄ alkyl, -C₁₋₄ alkylene-OH, -C₁₋₄ alkylene-SH, -C₁₋₄ alkylene-CN, and -C₁₋₄ alkylene-NR^{6a}R^{6b};
preferably, R⁶ is selected from: halogen, OH, SH, -NR^{6a}R^{6b}, CN, C₁₋₄ alkyl, C₁₋₄ haloalkyl, -C₁₋₄ alkylene-OH, -C₁₋₄ alkylene-SH, -C₁₋₄ alkylene-CN, and -C₁₋₄ alkylene-NR^{6a}R^{6b};
more preferably, R⁶ is selected from: F, Cl, OH, -NH₂, -NHCH₃, -N(CH₃)₂, CN, methyl, ethyl, -CHF₂, and -CF₃; more preferably -NH₂, -NHCH₃, -N(CH₃)₂, methyl, ethyl, -CHF₂, and -CF₃;
and/or
n is 0 or 1;
and/or
R^{1b}, R^{1e}, R^{1f}, R^{2a}, R^{2b}, R^{3a}, R^{3b}, R^{4a}, R^{4b}, R^{5a}, R^{5b}, R^{6a}, R^{6b} are, at each occurrence, independently selected from H and C₁₋₄ alkyl, preferably H, methyl, and ethyl.

14. The compound according to claim 1, **characterized in that** the compound is selected from:

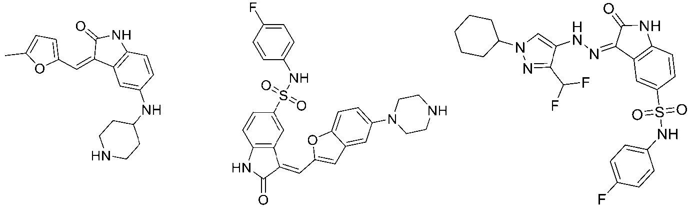

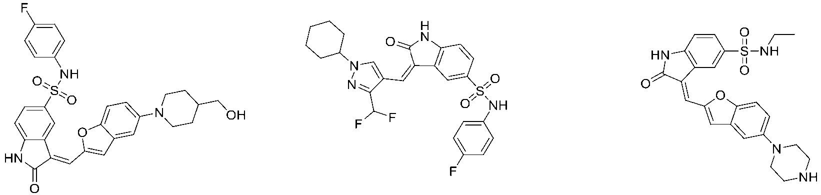

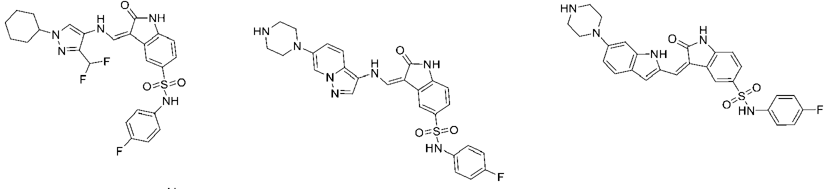

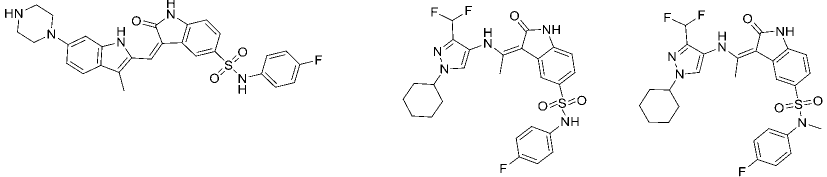

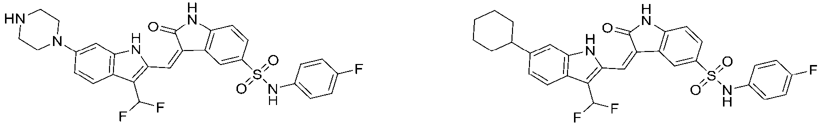

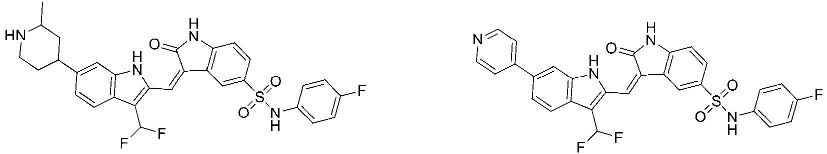

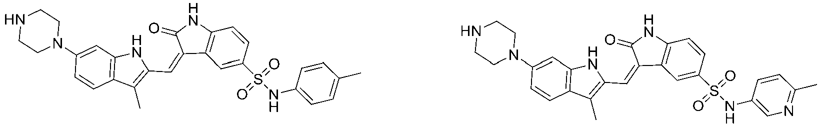

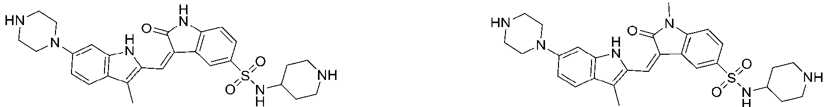

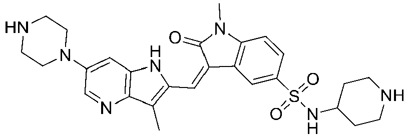

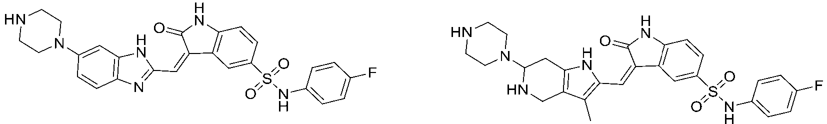

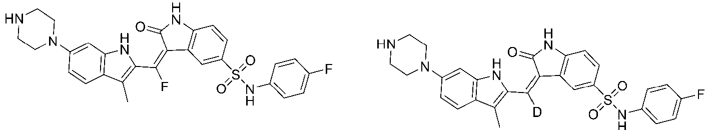

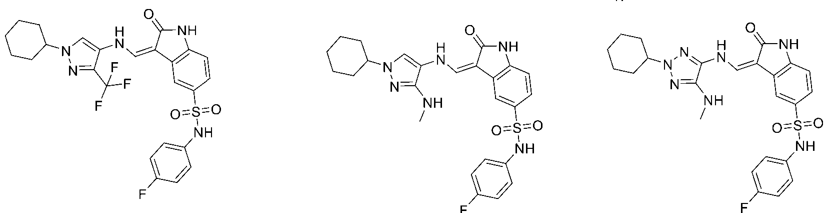

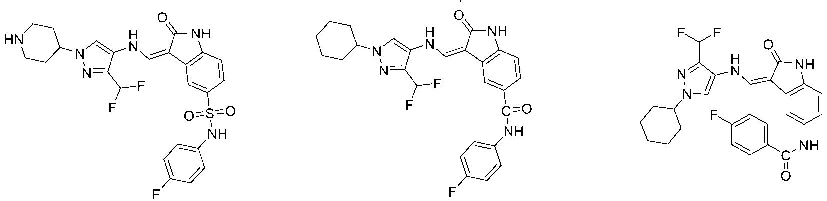

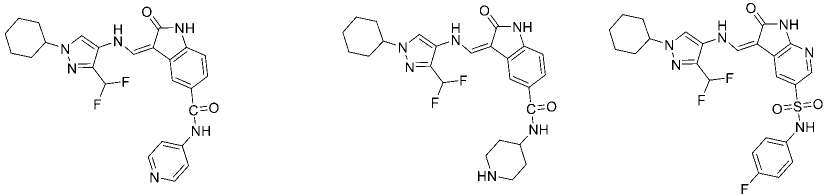

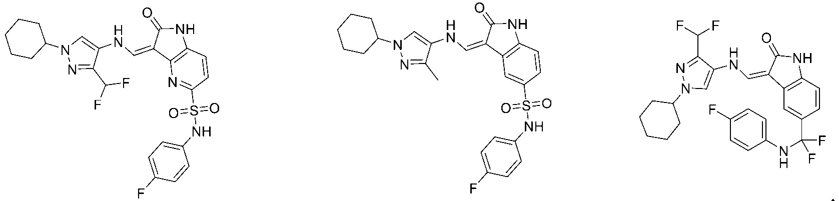

15. Use of the compound or the stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof according to any one of claims 1 to 14 for the preparation of a proteolysis targeting chimera (PROTAC).

16. A proteolysis targeting chimera (PROTAC), **characterized by** comprising a moiety with IRAK4 protein kinase inhibitory activity, wherein the moiety is derived from the compound or the stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof according to any one of claims 1 to 14.

17. A pharmaceutical composition, **characterized by** comprising the compound or the stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof according to any one of claims 1 to 14, or the proteolysis targeting chimera according to claim 16, and a pharmaceutically acceptable excipient, carrier or diluent.

18. Use of the compound or the stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof according to any one of claims 1 to 14, or the proteolysis targeting chimera according to claim 16, or the pharmaceutical composition according to claim 17 in the preparation of a medicament for treating a disease, disorder or condition associated with IRAK4 protein kinase.

19. A method for treating a disease, disorder or condition associated with IRAK4 protein kinase, **characterized by** comprising administering to an individual in need thereof a therapeutically effective amount of the compound or the stereoisomer, tautomer, diastereomer, racemate, cis/trans isomer, isotopically labeled compound (preferably deuterated compound), N-oxide, metabolite, ester, prodrug, crystal form, hydrate, solvate, or pharmaceutically acceptable salt thereof according to any one of claims 1 to 14, or the proteolysis targeting chimera according to claim 16, or the pharmaceutical composition according to claim 17.

20. The pharmaceutical composition according to claim 17, the use according to claim 18, or the method according to claim 19, **characterized in that** the disease, disorder or condition associated with IRAK4 protein kinase is selected from: autoimmune condition, inflammatory condition, cancer, graft rejection, thromboembolism, atherosclerosis, myocardial infarction, and metabolic syndrome;
preferably, the inflammatory condition is selected from; osteoarthritis, gout, gouty arthritis, chronic obstructive pulmonary disease, periodic fever, atopic dermatitis, allergic eczema, lymphadenectasis, sepsis, irritable bowel syndrome (IBD), ulcerative colitis, asthma, and allergy; and/or
preferably, the autoimmune condition is selected from; Crohn's disease, rheumatoid arthritis, systemic lupus erythematosus, lupus nephritis, cutaneous lupus, psoriasis, psoriatic arthritis, multiple sclerosis, neuropathic pain, ankylosing spondylitis, reactive arthritis, and systemic juvenile idiopathic arthritis; and/or
preferably, the graft rejection is selected from graft-versus-host disease and allograft rejection; and/or
preferably, the cancer is selected from; brain cancer, renal cancer, liver cancer, gastric cancer, vaginal cancer, ovarian cancer, gastric tumor, breast cancer, bladder cancer, colon cancer, prostate cancer, pancreatic cancer, lung cancer, cervical cancer, testicular cancer, skin cancer, bone cancer, thyroid cancer, sarcoma, glioblastoma, neuroblastoma, multiple myeloma, gastrointestinal cancer, neck and head tumor, adenoma, adenocarcinoma, keratoacanthoma, epidermoid carcinoma, large cell carcinoma, non-small cell lung cancer, Hodgkin's lymphoma and non-Hodgkin's lymphoma, mastocarcinoma, follicular carcinoma, papillary carcinoma, seminoma, melanoma, acute myeloid leukaemia, chronic myeloid leukemia, diffuse large B-cell lymphoma, activated B cell-like diffuse large B-cell lymphoma, chronic lymphocytic leukemia, chronic lymphocytic lymphoma, primary exudative lymphoma, Burkitt's lymphoma/leukemia, acute lymphoblastic leukemia, B-cell prolymphocytic leukemia, lymphoplasmacytic lymphoma, Waldenstrom's macroglobulinemia, splenic marginal zone lymphoma, intravascular large B-cell lymphoma, plasmacytoma, and multiple myeloma.
